# EUROPÄISCHE PATENTSCHRIFT

(11) **EP 0 586 343 B1**
(45) Veröffentlichungstag und Bekanntmachung des Hinweises auf die Patenterteilung: **06.08.1997**
(21) Anmeldenummer: 93810597.0
(22) Anmeldetag: 24.08.1993
(51) Int. Cl.: G03C 7/392, G03C 7/396, C07C 43/205, C07C 43/23, C07C 69/734, C07F 7/18

(54) **Farbphotographisches Aufzeichnungsmaterial**
Colour photographic recording material
Matériau d'enregistrement photographique couleur

(30) Priorität: 01.09.1992 CH 2731/92
(43) Veröffentlichungstag der Anmeldung: 09.03.1994
(73) Patentinhaber: Ciba Specialty Chemicals Holding Inc., 4057 Basel (CH)
(72) Erfinder: Leppard, David G., Dr., CH-1723 Marly (CH); Laver, Hugh Stephen, Dr., CH-1700 Fribourg (CH)

(56) Entgegenhaltungen:
- EP-A- 0 327 455
- EP-A- 0 357 442
- EP-A- 0 384 393
- DATABASE WPIL Section Ch, Week 8713, Derwent Publications Ltd., London, GB; Class C13, AN-87-091059

## Beschreibung

Die vorliegende Erfindung betrifft ein neues farbphotographisches Aufzeichnungsmaterial, enthaltend einen Magentakuppler und als Lichtschutzmittel einen Hydroxyalkyl-hydrochinonether.

Stabilisierte farbphotographische Materialien sind bekannt, wie z. B. beschrieben in der EP-A-0 357 442 und EP-A-0 384 393.

Bisher in photographischen Materialien als Stabilisatoren verwendete alkylierte Hydrochinonether oder -diether zeigten insbesondere bei 1H-Pyrazolo-[5,1-c][1,2,4]triazol-Magentakupplern (siehe auch Struktur C-5) eine unzureichende Aktivität. Die Lichtstabilität der Magentafarbstoffe war nicht ausreichend. Auch mussten bisher die Farbkuppler in hochsiedenden Ölen vorgelöst werden, bevor sie in der Gelatine dispergiert werden konnten. Einige bekannte Stabilisatoren hätten sich zwar prinzipiell als Kuppleröle geeignet, jedoch besitzen sie Nebenwirkungen, z.B. sensitometrische Effekte, Wellenlängenverschiebung der Farbstoffabsorption, Klima- oder Atlasvergilbung, und konnten daher nicht voll befriedigen.

Es wurde nun eine Gruppe von Hydroxyalkyl-hydrochinonethern gefunden, die sich überraschenderweise weitgehend frei von solchen Nachteilen erweisen. Sie eignen sich darüberhinaus auch als Kuppleröl und ermöglichen damit die vereinfachte Einarbeitung der Kuppler. Insbesondere ist diese Gruppe von Hydochinonen geeignet, die Stabilität der Magentafarbstoffe in farbphotographischen Materialien zu erhöhen.

Die erfindungsgemässen Stabilisatoren können für alle Arten photosensitiven Materials verwendet werden. Beispielsweise können sie für Farbpapier, Farbumkehrpapier, Direkt-Positiv-Farbmaterial, Farbnegativfilm, Farbpositivfilm, Farbumkehrfilm und weitere eingesetzt werden. Unter anderem werden sie bevorzugt für photosensitives Farbmaterial, welches ein Umkehrsubstrat enthält oder welches Positive bildet, verwendet

Üblicherweise enthalten die farbphotographischen Aufzeichnungsmaterialien auf einem Träger eine blauempfindliche und/oder eine grünempfindliche und/oder eine rotempfindliche Silberhalogenidemulsionsschicht sowie gegebenenfalls eine Protektionsschicht, wobei sich der Stabilisator für den Magentafarbstoff in der grünempfindlichen Schicht befindet.

Gegenstand vorliegender Anmeldung ist somit ein farbphotographisches Aufzeichnungsmaterial, welches einen Magentakuppler enthält, dadurch gekennzeichnet, dass es als Stabilisator mindestens eine Verbindung der Formel 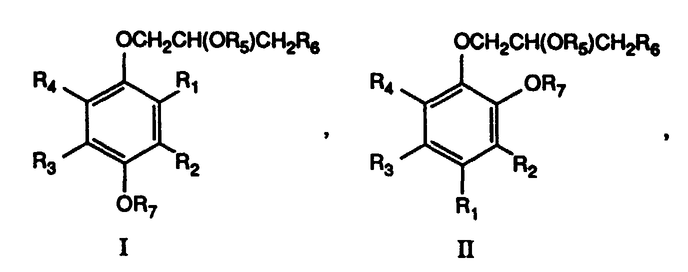 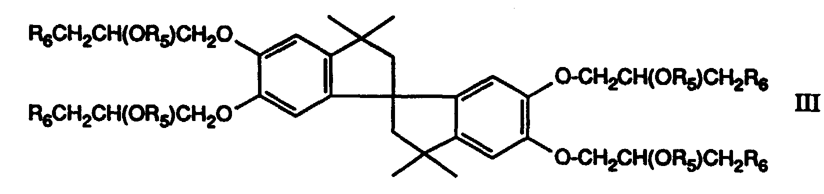 oder 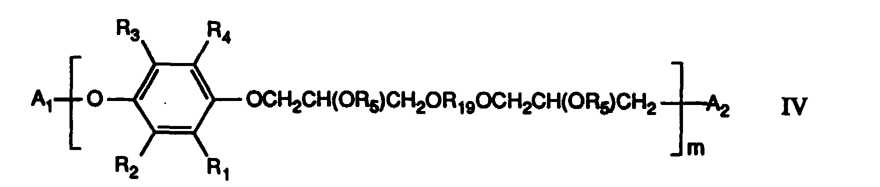 enthält, wobei
R₁ und R₃ unabhängig voneinander Wasserstoff, C₁-C₁₂-Alkyl, C₅-C₇-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 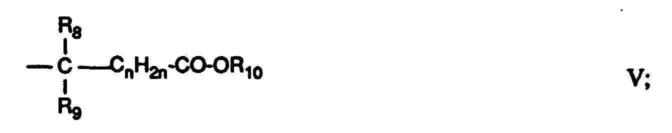
   worin R₈ und R₉ unabhängig voneinander C₁-C₈-Alkyl;
   n = 1-10
   R₁₀ Wasserstoff, C₁-C₂₄-Alkyl, das unsubstituiert oder durch ein oder mehrene -O- Atome unterbrochen und gegebenenfalls durch ein -OH substituiert ist, C₂-C₁₈-Alkenyl, C₅-C₁₂-Cycloalkyl, das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
   worin R₁₁ C₁-C₁₈-Alkyl, C₂-C₁₈-Alkenyl oder Phenyl;
   R₁₂ C₁-C₄-Alkyl;
   R₁₃, R₁₄ und R₁₅ unabhängig voneinander C₁-C₆-Alkyl oder Phenyl ist;
die Reste R₆ unabhängig voneinander -OR₁₆ oder C₁-C₁₅-Alkyl;
   worin R₁₆ Wasserstoff, C₁-C₁₈-Alkyl, C₂-C₁₈-Alkenyl, Phenyl-C₁-C₄-alkyl, durch ein oder mehrere O-Atome unterbrochenes C₃-C₂₄-Alkyl oder C₂-C₁₄-Hydroxyalkyl, Phenyl, Tolyl, C₅-C₆-Cycloalkyl, das gegebenenfalls mit 1 bis 3 C₁-C₈-Alkyl substituiert ist, oder -CO-R₁₁ ist;
   worin R₁₁ C₁-C₁₈-Alkyl, C₂-C₁₈-Alkenyl oder Phenyl ist;
   R₇ eine Gruppe der Formel VI 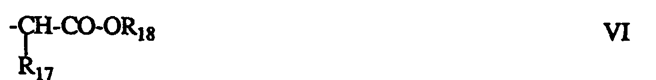 oder eine Gruppe der Formel VII

      -CH₂CH(OR₅)CH₂R₆ VII;
   worin R₅ und R₆ wie für Formel I angegeben;
   R₁₇ Wasserstoff oder C₁-C₁₈-Alkyl; und
   R₁₈ C₁-C₁₂-Alkyl, das unsubstituiert oder durch ein oder mehrere -O- Atome unterbrochen ist, C₂-C₁₈-Alkenyl, Benzyl oder Phenyl, das gegebenenfalls mit 1-3 C₁-C₄-Alkyl substituiert ist, ist;
R₁₉ C₂-C₁₀-Alkylen, Phenylen oder eine Gruppe -Phenylen-R₂₀-Phenylen-;
   worin R₂₀ -O-, -S-, -SO₂-, -CH₂- oder -C(CH₃)₂- ist;
m = 1-100;
A₁ Wasserstoff, -CH₂CH(OR₅)CH₂OR₁₉OCH(OR₅)CH₂OR₅ oder 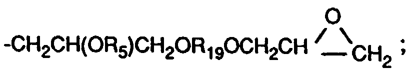 und
A₂ -OH oder 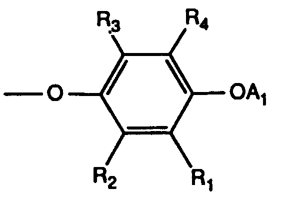 bedeutet;
oder in Formel I R₃ und R₇ zusammen mit den Atomen an die sie gebunden sind einen C₅-C₆ Ring, der gegebenenfalls mit 1 bis 3 C₁-C₈-Alkyl substituiert ist, bilden.

Bedeuten Substituenten in den erfindungsgemässen Verbindungen Alkyl mit 1 bis 24 Kohlenstoffatomen, so kommen hierfür Reste wie Methyl, Ethyl, Propyl, Butyl, Pentyl, Hexyl, Heptyl, Octyl, Nonyl, Decyl, Undecyl, Dodecyl, Tetradecyl, Hexadecyl und Octadecyl sowie entsprechende verzweigte Isomeren in Frage.

C₅-C₁₂-Cycloalkylreste bedeuten beispielsweise Cyclopentyl, Cyclohexyl, Cyclooctyl oder Cyclododecyl.

Alkylreste mit 3 bis 24 Kohlenstoffatomen, die durch Sauerstoff unterbrochen sind, sind beispielsweise

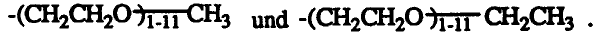

Alkenylreste mit 2 bis 18 Kohlenstoffatomen können ein- oder, ab 4 Kohlenstoffatomen, mehrfach ungesättigt sein.

Alkylenreste mit 2 bis 10 Kohlenstoffatomen können von entsprechenden Alkylresten abgeleitet werden.

C₁-C₈-Alkoxyreste bedeuten beispielsweise Methoxy, Ethoxy, Propoxy, Butoxy oder Hexoxy sowie entsprechende verzweigte Isomere.

Bevorzugt sind Stabilisatoren der Formeln 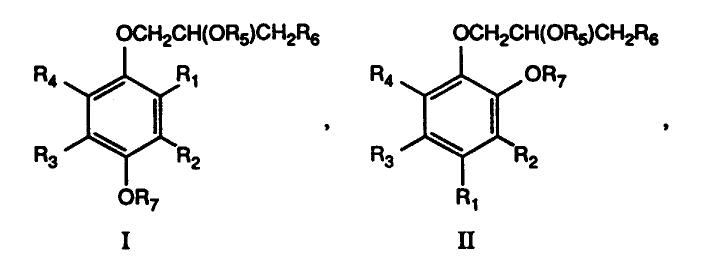 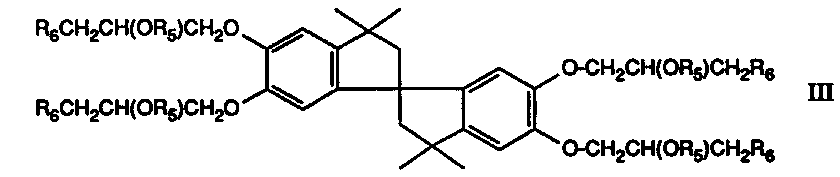 oder 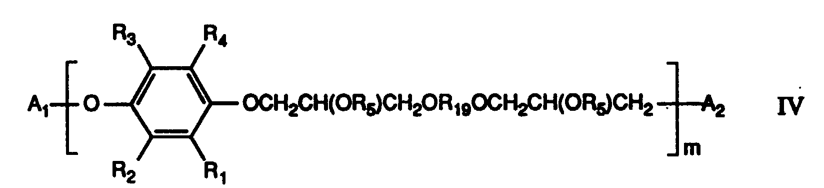
wobei R₁ und R₃ unabhängig voneinander Wasserstoff, C₁-C₈-Alkyl, Cyclohexyl, -C(CH₃)₂C₆H₅, C₁-C₄-Alkoxy oder eine Gruppe der Formel V 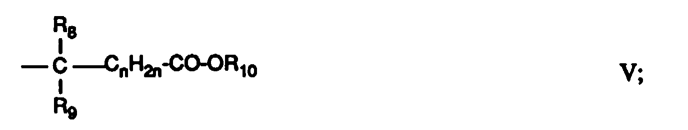
   worin R₈ und R₉ Methyl;
   n = 3 und
   R₁₀ Wasserstoff, C₁-C₁₂-Alkyl, das unsubstituiert ist oder durch ein oder mehrere -O- Atome unterbrochen und gegebenenfalls durch ein -OH substituiert ist, C₂-C₁₈-Alkenyl oder Benzyl ist;
R₂ Wasserstoff;
R₄ Wasserstoff oder C₁-C₈-Alkyl;
R₅ Wasserstoff, -CO-R₁₁, -CO-OR₁₂ oder -Si(R₁₃)(R₁₄)(R₁₅);
   worin R₁₁ C₁-C₁₈-Alkyl, C₂-C₁₈-Alkenyl oder Phenyl;
   R₁₂ C₁-C₄-Alkyl;
   R₁₃, R₁₄ und R₁₅ unabhängig voneinander C₁-C₆-Alkyl oder Phenyl ist; die Reste R₆ unabhängig voneinander -OR₁₆;
   worin R₁₆ Wasserstoff, C₁-C₁₈-Alkyl, C₂-C₁₈-Alkenyl, Phenyl-C₁-C₄-alkyl, durch ein oder mehrere O-Atome unterbrochenes C₃-C₂₄-Alkyl oder C₂C₁₄-Hydroxyalkyl, Phenyl, Tolyl, C₅-C₆-Cycloalkyl, das gegebenenfalls mit 1 bis 3 C₁-C₈-Alkyl substituiert ist, oder -CO-R₁₁ ist;
      worin R₁₁ C₁-C₁₈-Alkyl, C₂-C₁₈-Alkenyl oder Phenyl ist;
   R₇ eine Gruppe der Formel VI 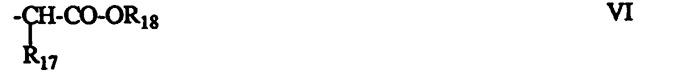 oder eine Gruppe der Formel VII

      -CH₂CH(OR₅)CH₂R₆ VII;

      worin R₅ und R₆ wie für Formel I angegeben;
   R₁₇ Wasserstoff oder C₁-C₁₂-Alkyl und
   R₁₈ C₁-C₈-Alkyl ist;
R₁₉ C₂-C₈-Alkylen, Phenylen oder eine Gruppe -Phenylen-R₂₀-Phenylen-;
   worin R₂₀ -SO₂-, -CH₂- oder -C(CH₃)₂- ist;
m = 1-50;
A₁ Wasserstoff, -CH₂CH(OR₅)CH₂OR₁₉OCH(OR₅)CH₂OR₅ oder 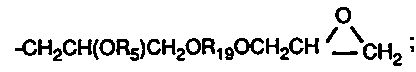 und
A₂ - OH oder 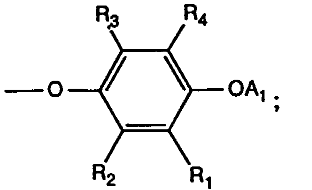 bedeuten.

Besonders bevorzugt sind Stabilisatoren der Formeln 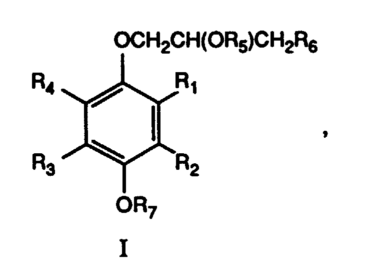 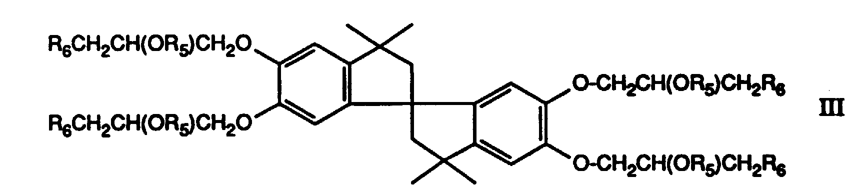 oder 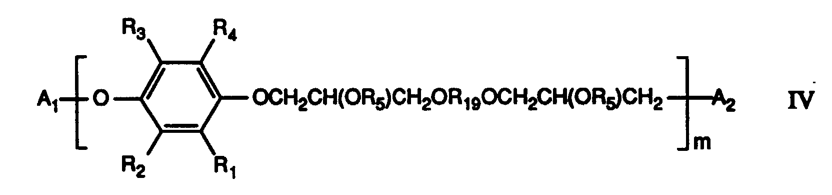
wobei R₁ und R₃ unabhängig voneinander Wasserstoff, C₁-C₈-Alkyl, C₁-C₄-Alkoxy oder eine Gruppe der Formel V 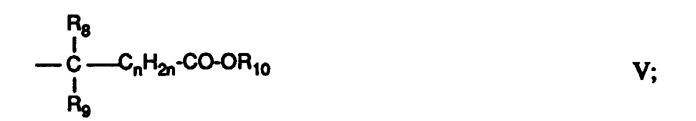
   worin R₈ und R₉ Methyl;
   n = 3; und
   R₁₀ C₁-C₄-Alkyl ist;
R₂ Wasserstoff;
R₄ Wasserstoff oder C₁-C₈-Alkyl;
R₅ Wasserstoff,-CO-R₁₁, -COOR₁₂ oder -Si(R₁₃)(R₁₄)(R₁₅);
worin R₁₁ C₁-C₄-Alkyl oder C₂-C₃-Alkenyl;
   R₁₂ C₁-C₄-Alkyl;
   R₁₃, R₁₄, R₁₅ unabhängig voneinander C₁-C₆-Alkyl ist;
die Reste R₆ unabhängig voneinander -OR₁₆;
worin R₁₆ C₁-C₁₈-Alkyl, Allyl, Benzyl, Phenyl, durch ein oder mehrere O-Atome unterbrochenes C₃-C₁₂-Alkyl, Cyclohexyl oder -CO-R₁₁ ist;
   worin R₁₁ C₁-C₄-Alkyl oder C₂-C₃-Alkenyl ist;
R₇ eine Gruppe der Formel VII

   -CH₂CH(OR₅)CH₂R₆ VII;

   worin R₅ und R₆ wie für Formel I angegeben ist;
R₁₉ C₂-C₈-Alkylen oder eine Gruppe -Phenylen-R₂₀-Phenylen-
worin R₂₀ -C(CH₃)₂- ist;
m = 1-25;
A₁ Wasserstoff, -CH₂CH(OR₅)CH₂OR₁₉OCH(OR₅)CH₂OR₅ oder 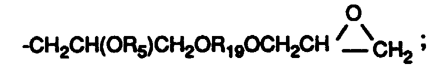 und
A₂ -OH oder 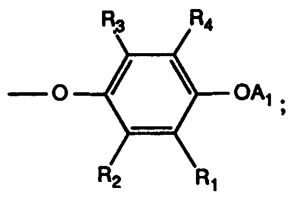 bedeutet.

Von besonderer Bedeutung sind Stabilisatoren der Formel 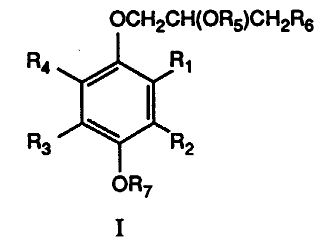 wobei
R₁, R₃ und R₄ unabhängig voneinander Wasserstoff oder C₁-C₈-Alkyl;
R₂ Wasserstoff;
R₅ Wasserstoff oder -CO-CH₃;
die Reste R₆ unabhängig voneinander -OR₁₆;
   worin R₁₆ C₁-C₁₂-Alkyl, Allyl oder durch 1-3 O-Atome unterbrochenes C₃-C₇-Alkyl ist; und
R₇ eine Gruppe der Formel VII

   -CH₂CH(OR₅)CH₂R₆ VII;

   worin R₅ und R₆ wie für Formel I angegeben ist;
bedeutet.

Ganz besonders bevorzugt sind Stabilisatoren der Formel 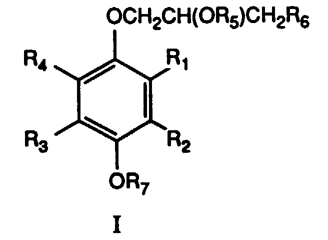 wobei
R₁, R₃ und R₄ unabhängig voneinander Wasserstoff oder C₁-C₈-Alkyl;
R₂ Wasserstoff;
R₅ Wasserstoff;
die Reste R₆ unabhängig voneinander -OR₁₆;
   worin R₁₆ C₁-C₁₂-Alkyl ist; und
R₇ eine Gruppe der Formel VII

   -CH₂CH(OR₅)CH₂R₆ VII;

   worin R₅ und R₆ wie für Formel I angegeben ist; bedeutet.

Vorzugsweise enthält das erfindungsgemässe Material zwischen den Silberhalogenidemulsionsschichten Gelatinezwischenschichten.

Bevorzugt sind solche photographischen Materialien, welche als Silberhalogenid in der blauempfindlichen und/oder grünempfindlichen und/oder rotempfindlichen Schicht Silberchloridbromid, welches mindestens zu 90 mol % aus Silberchlorid besteht, enthalten.

Desweiteren sind photographische Materialien bevorzugt, welche die Silberhalogenidemulsionsschichten in der Reihenfolge blauempfindliche, grünempfindliche und rotempfindliche Silberhalogenidemulsionsschicht enthalten.

Typische und bevorzugte erfindungsgemässe Stabilisatoren sind die folgenden:

### Beispiele

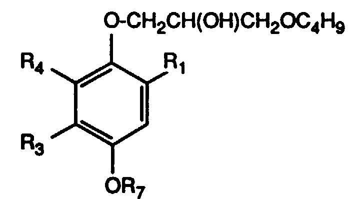

| Nr. | R₁ | R₃ | R₄ | R₇ |
|---|---|---|---|---|
| 1. | C(CH₃)₃ | C(CH₃)₃ | H | CH₂CH(OH)CH₂OC₄H₉ |
| 3. | OC₄H₉ | OC₄H₉ | H | CH₂CH(OH)CH₂OC₄H₉ |
| 5. | C(CH₃)₂(CH₂)₃CO-OCH₃ | C(CH₃)₂(CH₂)₃CO-OCH₃ | H | CH₂CH(OH)CH₂OC₄H₉ |
| 7. | C(CH₃)₂CH₂C(CH₃)₃ | H | H | CH₂CH(OH)CH₂OC₄H₉ |
| 9. | C(CH₃)₂CH₂C(CH₃)₃ | C(CH₃)₂CH₂C(CH₃)₃ | H | CH₂CH(OH)CH₂OC₄H₉ |
| 11. | C(CH₃)₃ | H | H | CH₂(C₁₂H₂₅)CO-OC₂H₅ |
| 12. | H | H | H | CH₂CH(OH)CH₂OC₄H₉ |
| 13. | CH₃ | CH₃ | CH₃ | CH₂CH(OH)CH₂OC₄H₉ |
| 14. | CH₃ | CH₃ | H | CH₂CH(OH)CH₂OC₄H₉ |
| 16. | CH₃ | H | H | CH₂CH(OH)CH₂OC₄H₉ |
| 19. | H | CH₃ | CH₃ | CH₂CH(OH)CH₂OC₄H₉ |
| 20. | C(CH₃)₃ | H | H | CH₂CH(OH)CH₂OC₄H₉ |

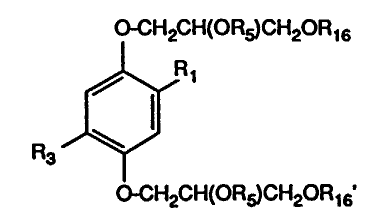

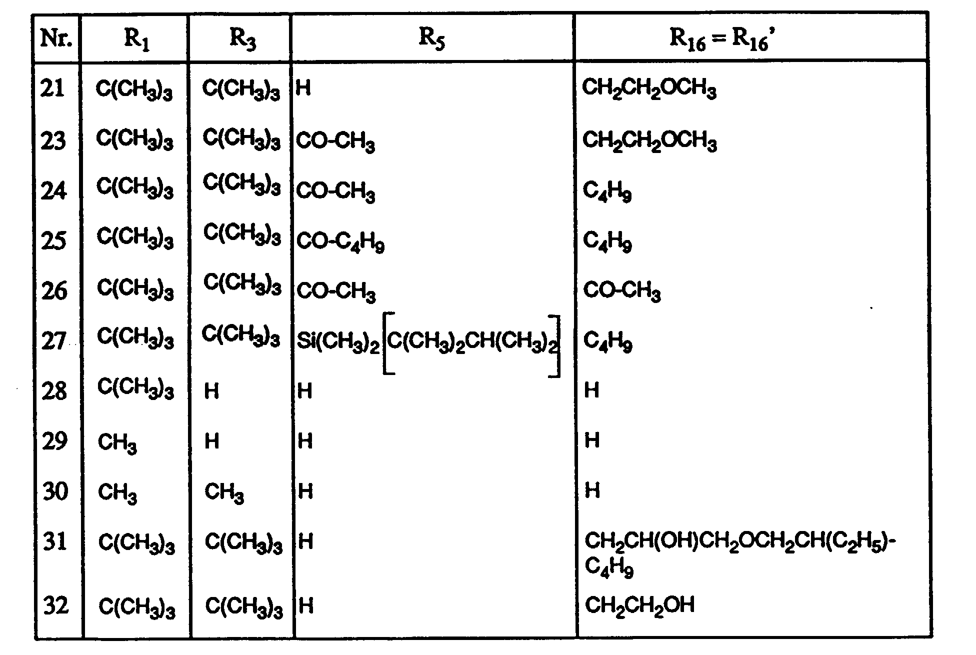

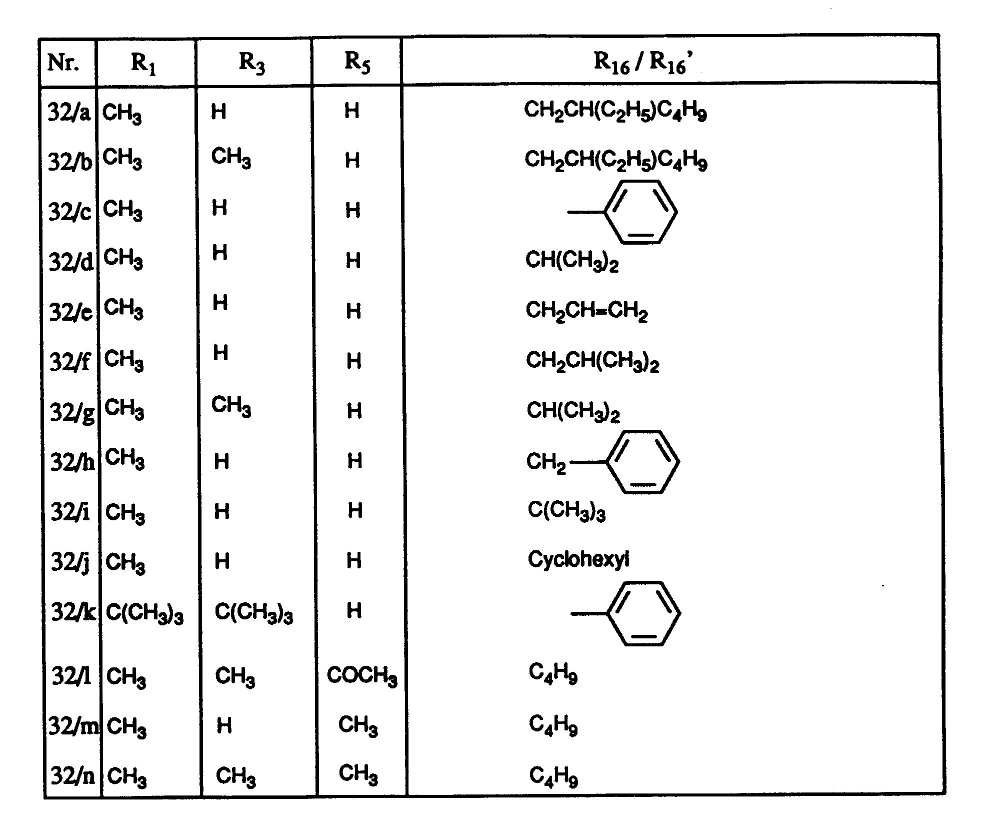

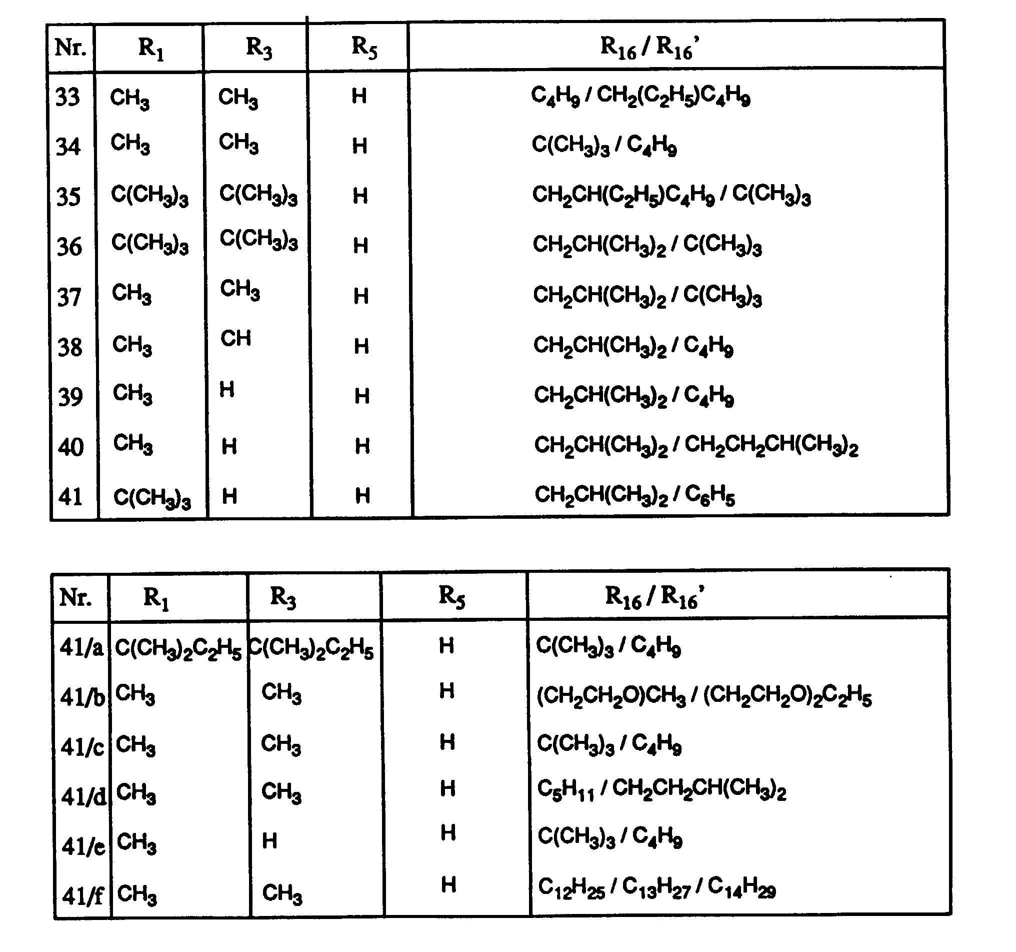

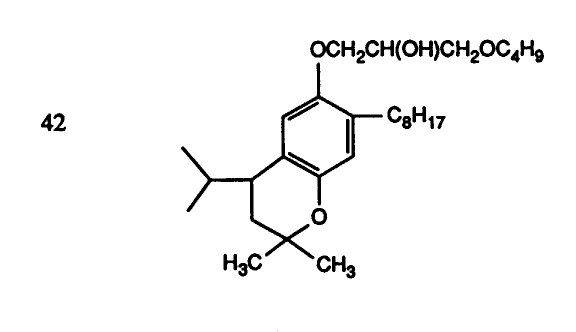

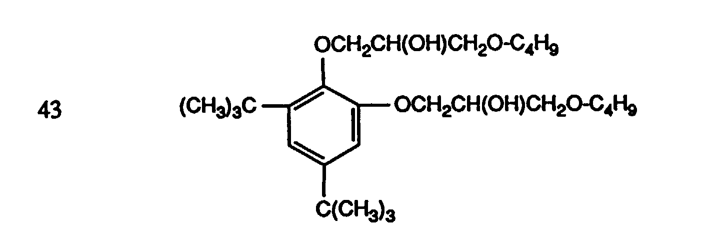

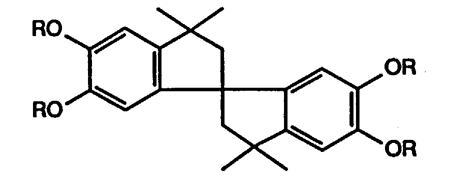

| Nr. | R |
|---|---|
| 44 | -CH₂CH(OH)CH₂OC₄H₉ |
| 45 | -CH₂CH(OH)CH₂OCH₂CH(C₂-H₅)C₄H₉ |
| 46 | -CH₂CH(OH)CH₂OC₁₃H₂₇(C₁₅H₃₁) |

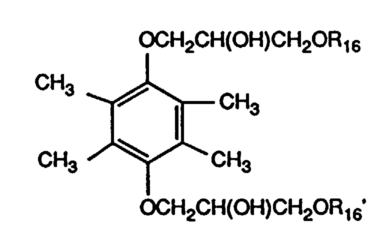

48 R₁₆ = R₁₆'= C₄H₉

49 R₁₆ = R₁₆'= CH₂CH(C₂H₅)C₄H₉

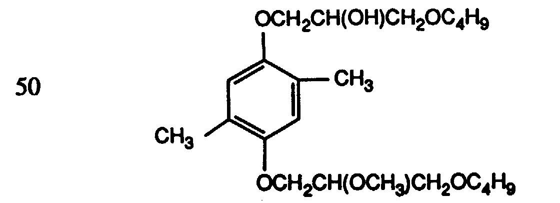

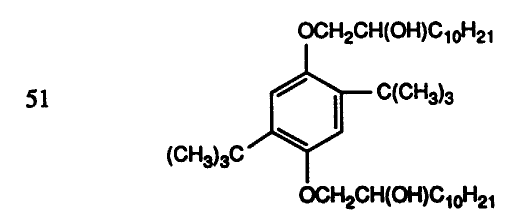

Im allgemeinen werden die erfindungsgemässen Stabilisatoren in der 0,1 bis 4-fachen, vorzugsweise 0,2 bis 2-fachen, Gewichtsmenge, bezogen auf den mit ihnen zusammen verwendeten Farbkuppler, verwendet.

Die in dem farbphotographischen Aufzeichnungsmaterial vorhandenen Magentakuppler können z.B. einfache 1-Aryl-5-pyrazolone sein oder mit 5-gliedrigen Heteroringen kondensierte Pyrazolderivate wie z.B. Imidazopyrazole, Pyrazolopyrazole, Pyrazolotriazole oder Pyrazolotetrazole.

Eine Gruppe von Magentakupplern sind 5-Pyrazolone der Formel C, 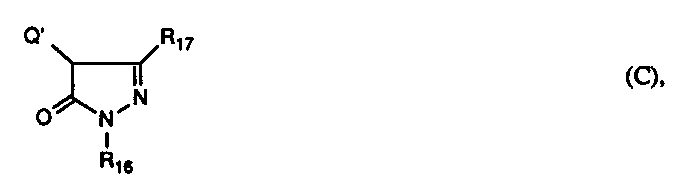 wie sie in der Britischen Patentschrift 2,003,473 beschrieben sind. Darin ist R₁₆ Wasserstoff, Alkyl, Aryl, Alkenyl oder eine heterocyclische Gruppe. R₁₇ ist Wasserstoff, Alkyl Aryl, eine heterocyclische Gruppe, eine Estergruppe, Alkoxygruppe, Alkylthiogruppe, Carboxylgruppe, Arylaminogruppe, Acylaminogruppe, (Thio)-harnstoffgruppe, (Thio)-carbamoylgruppe, Guanidinogruppe oder Sulfonamidogruppe.

Bevorzugt ist R₁₇ eine Gruppe 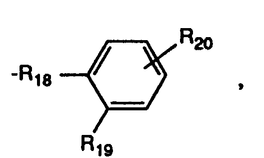 worin R₁₈ Imino, Acylamino oder Ureido ist, R₁₉ Wasserstoff, Halogen, Alkyl oder Alkoxy ist, R₂₀ Wasserstoff, Alkyl, Acylamino, Carbamoyl, Sulfamoyl, Sulfonamido, Alkoxycarbonyl, Acyloxy oder eine Urethangruppe ist.

Wenn Q' Wasserstoff ist, so ist der Magentakuppler tetraäquivalent in bezug auf das Silberhalogenid.

Bevorzugt für diesen Typ von Magentakupplern sind Verbindungen der Formel 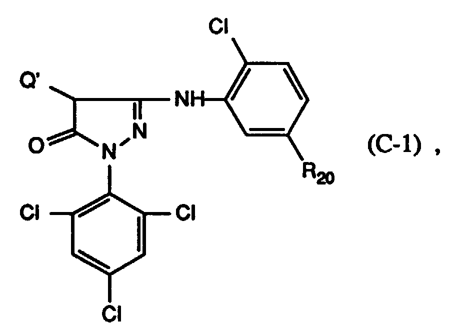 worin R₂₀ die oben genannten Bedeutungen hat, und Q', wie oben beschrieben, eine Abgangsgruppe ist. Diese Verbindungen liegen bevorzugt im erfindungsgemässen Material vor.

Weitere Beispiele solcher tetraäquivalenter Magentakuppler sind zu finden in den US-A 2,983,608, 3,061,432, 3,062,653, 3,127,269, 3,152,896, 3,311,476, 3,419,391, 3,519,429, 3,558,319, 3,582,322, 3,615,506, 3,684,514, 3,834,908, 3,888,680, 3,891,445, 3,907,571, 3,928,044, 3,930,861, 3,930,866 und 3,933,500 und in JP-A-89/309,058.

Wenn Q' in Formel C nicht Wasserstoff ist sondern eine Gruppe, die bei der Reaktion mit dem oxidierten Entwickler eliminiert wird, so handelt es sich um einen diäquivalenten Magentakuppler. Q kann in diesem Fall z.B. Halogen oder eine über O, S oder N an den Pyrazolring gebundenen Gruppe sein. Solche diäquivalenten Kuppler ergeben eine höhere Farbdichte und sind reaktiver gegenüber dem oxidierten Entwickler als die entsprechenden tetraäquivalenten Magentakuppler.

Beispiele für diäquivalente Magentakuppler sind beschrieben in den US-A 3,006,579, 3,419,391, 3,311,476, 3,432,521, 3,214,437, 4,032,346, 3,701,783, 4,351,897, 3,227,554, in den EP-A-133,503, DE-A-2,944,601, JP-A-78/34044, 74/53435, 74/53436, 75/53372 und 75/122935.

Typische und bevorzugte Magentakuppler entsprechen der Formeln

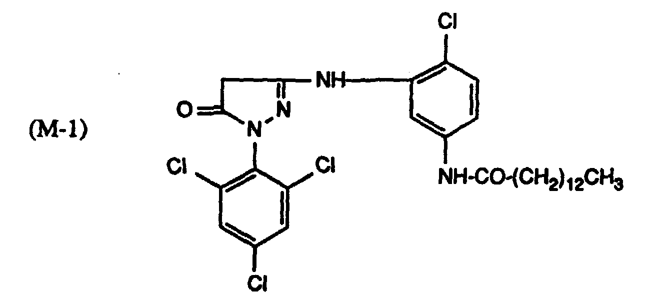

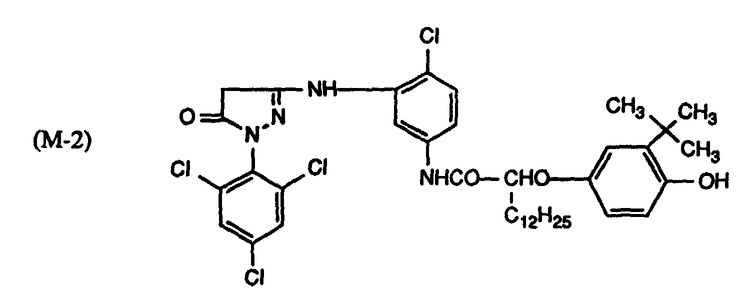

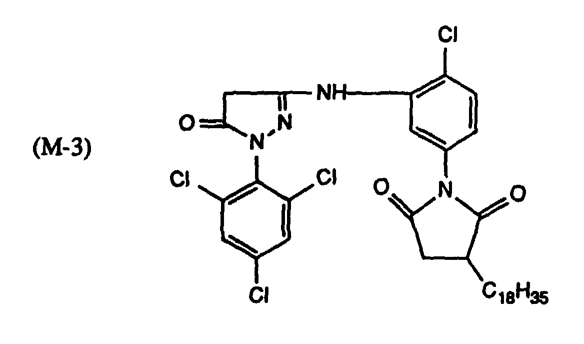

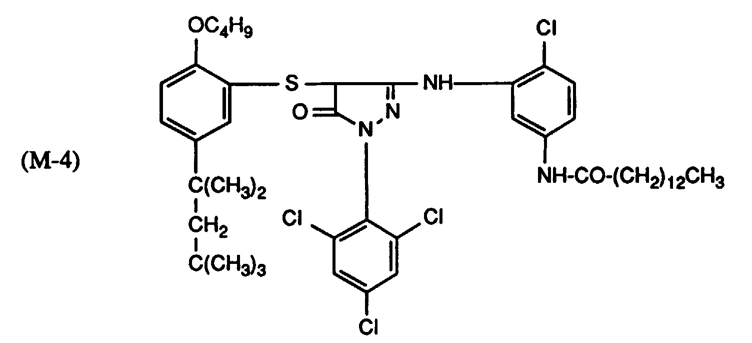

Ueber ein zweiwertiges Q' können 2 Pyrazolonringe verknüpft werden und man erhält dann sogenannte Bis-Kuppler. Solche sind z.B. beschrieben in den US-A-2,632,702, US-A-2,618,864, GB-A-968,461, GB-A-786,859, JP-A-76/37646, 59/4086, 69/16110, 69/26589, 74/37854 und 74/29638. Bevorzugt ist Y eine O-Alkoxyarylthio-Gruppe.

Wie vorstehend erwähnt, können als Magentakuppler auch mit 5-gliedrigen Heterocyclen kondensierte Pyrazole - sogenannte Pyrazoloazole - verwendet werden. Deren Vorteile gegenüber einfachen Pyrazolen ist, dass sie Farben von grösserer Formalin-Beständigkeit und reineren Absorptionsspektren aufweisen.

Magentakuppler vom Pyrazoloazoltyp, welche ebenfalls bevorzugt sind, können durch die Formeln 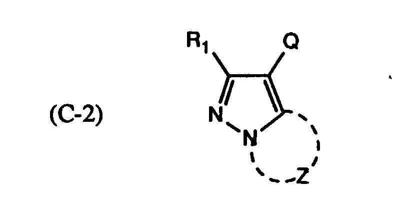 dargestellt werden, worin R₁ Wasserstoff oder ein Substituent ist, Z die zur Vervollständigung eines 5-gliedrigen Ringes mit 2 oder 3 Stickstoffatomen notwendigen nichtmetallischen Atome darstellt, wobei dieser Ring substituiert sein kann, und Q Wasserstoff oder eine Abgangsgruppe ist.

Bevorzugt hiervon sind Magentakuppler der Formeln

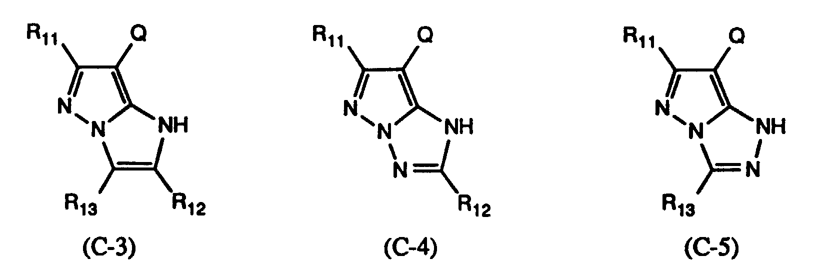

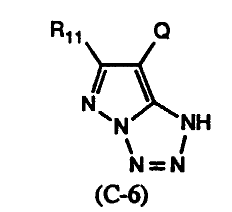

R₁₁, R₁₂ und R₁₃ bedeuten unabhängig voneinander beispielsweise Wasserstoff, Halogen, eine Gruppe der Formel -CR₃, worin die Reste R unabhängig voneinander Wasserstoff oder Alkyl sind, Aryl, Heterocyclyl, Cyano, Hydroxy, Nitro, Carboxyl, Amino, Alkoxy, Aryloxy, Acylamino, Alkylamino, Anilino, Ureido, Sulfamoylamino, Alkylthio, Arylthio, Alkoxycarbonylamino, Sulfonamido, Carbamoyl, Sulfamoyl, Sulfonyl, Alkoxycarbonyl, Heterocyclyl-oxy, Azo, Acyloxy, Carbamoyloxy, Silyloxy, Aryloxycarbonylamino, Imido, heterocyclische Ring-thio, Sulfinyl, Phosphonyl, Aryloxycarbonyl, Acyl oder Azolyl, und vorzugsweise Wasserstoff, Halogen (z.B. Chlor, Brom), eine Gruppe der Formel -CR₃, worin die Reste R unabhängig voneinander Wasserstoff oder Alkyl sind, Aralkyl, Alkenyl, Alkinyl, Cycloalkyl oder Cycloalkenyl und besonders bevorzugt Methyl, Ethyl, Propyl, Isopropyl, t-Butyl, Tridecyl, 2-Methansulfonylethyl, 3-(3-Pentadecylphenoxy)propyl, 3-(4-(2-(4-(4-Hydroxyphenylsulfonyl)phenoxy)dodecanamido)phenyl)propyl, 2-Ethoxytridecyl, Trifluoromethyl, Cyclopentyl, 3-(2,4-Di-t-Amylphenoxy)propyl); Aryl (z.B. Phenyl, 4-t-Butylphenyl, 2,4-Di-t-amylphenyl, 4-Tetradecaneamidophenyl); Heterocyclyl (z.B. 2-Furyl, 2-Thienyl, 2-Pyrimidinyl, 2-Benzothiazolyl); Cyano; Hydroxy, Nitro; Carboxy; Amino; Alkoxy (z.B. Methoxy, Ethoxy, 2-Methoxyethoxy; 2-Dodecylethoxy, 2-Methansulfonylethoxy); Aryloxy (z.B. Phenoxy, 2-Methylphenoxy, 4-t-Butylphenoxy, 3-Nitrophenoxy, 3-t-Butyloxycarbamoylphenoxy, 3-Methoxycarbamoyl); Acylamino (z.B. Acetoamido, Benzamido, Tetradecanamido, 2-(2,4-Di-t-amylphenoxy)butanamido, 4-(3-t-Butyl-4-hydroxyphenoxy)butanamido, 2-(4-(4-Hydroxyphenylsulfonyl)phenoxy)decanamido); Methylbutylamino); Anilino (z.B. Phenylamino, 2-Chloranilino, 2-Chloro-5-tetradecanaminoanilino, 2-Chloro-5-dodecyloxycarbonylanilino, N-Acetylanilino, 2-Chloro-5-(alpha-(3-t-butyl-4-hydroxyphenoxy)dodecanamidoanilino); Ureido (z.B. Phenylureido, Methylureido, N,N-Dibutylureido); Sulfamoylamino (z.B. N,N-Dipropylsulfamoylamino, N-Methyl-N-decylsulfamoylamino); Alkylthio (z.B. Methylthio, Octylthio, Tetradecylthio, 2-Phenoxyethylthio, 3-Phenoxypropylthio, 3-(4-t-Butylphenoxy)propylthio); Arylthio (z.B. Phenylthio, 2-Butoxy-5-t-octylphenylthio, 3-Pentadecylphenylthio, 2-Carboxyphenylthio, 4-Tetradecanamidophenylthio); Alkoxycarbonylamino (z.B. Methoxycarbonylamino, Tetradecyloxycarbonylamino); Sulfonamido (z.B. Methansulfonamido, Hexadecansulfonamido, Benzolsulfonamido, p-Toluolsulfonamido, Octadecansulfonamido, 2-Methyloxy-5-t-butylbenzolsulfonamido); Carbamoyl (z.B. N-Ethylcarbamoyl, N,N-Dibutylcarbamoyl, N-(2-Dodecyloxyethyl)carbamoyl, N-Methyl-N-dodecylcarbamoyl, N-(3-(2,4-Di-t-Amylphenoxy)propyl)carbamoyl); Sulfamoyl (z.B. N-Ethylsulfamoyl, N,N-Dipropylsulfamoyl, N-2(-Dodecyloxyethyl)sulfamoyl, N-Ethyl-N-dodecylsulfamoyl, N,N-Diethylsulfamoyl); Sulfonyl (z.B. Methansulfonyl, Octansulfonyl, Benzolsulfonyl, Toluolsulfonyl); Alkoxycarbonyl (z.B. Methoxycarbonyl, Butoxycarbonyl, Dodecyloxycarbonyl, Octadecyloxycarbonyl); heterocyclische Ringoxy (z.B. 1-Phenyltetrazol-5-oxy, 2-Tetrahydropyranyloxy); Azo (z.B. Phenylazo, 4-Methoxyphenylazo, 4-Pivaloylaminophenylazo, 2-Hydroxy-4-propanoylphenylazo); Acyloxy (z.B. Acetoxy); Carbamoyloxy (z.B. N-Methylcarbamoyloxy, N-Phenylcarbamoyloxy); Silyloxy (z.B. Trimethylsilyloxy, Dibutylmethylsilyloxy); Aryloxycarbonylamino (z.B. Phenoxycarbonylamino); Imido (z.B. N-Succinimido, N-Phthalimido, 3-Octadecenylsuccinimido); heterocyclische Ring-thio (z.B. 2-Benzothiazolylthio, 2,4-Diphenyloxy-1,3,5-triazol-6-thio, 2-Pyridylthio); Sulfinyl (z.B. Dodecansulfinyl, 3-Pentadecylphenylsulfinyl, 3-Phenoxypropylsulfinyl); Phosphonyl (z.B. Phenoxyphosphonyl, Octyloxyphosphonyl, Phenylphosphonyl); Aryloxycarbonyl (z.B. Phenoxycarbonyl); Acyl (z.B. Acetyl, 3-Phenylpropanoyl, Benzoyl, 4-Dodecyloxybenzoyl); Azolyl (z.B. Imidazolyl, Pyrazolyl, 3-Chloro-pyrazol-1-yl).

Diese Substituenten sind gegebenenfalls weiter substituiert, beispielsweise durch Halogen oder durch einen über ein C-, O-, N- oder S-Atom gebundenen organischen Rest.

Die bevorzugten Gruppen R₁₁ sind Alkyl, Aryl, Alkoxy, Aryloxy, Alkylthio, Ureido, Urethan und Acylaminogruppen.

R₁₂ kann die Bedeutung von R₁₁ besitzen und ist vorzugsweise Wasserstoff, Alkyl, Aryl, ein heterocyclischer Ring, Alkoxycarbonyl, Carbamoyl, Sulfamoyl, Sulfinyl, Acyl oder Cyano.

R₁₃ kann die Bedeutung von R₁₁ haben und ist vorzugsweise Wasserstoff, Alkyl, Aryl, Heterocyclic, Alkoxy, Aryloxy, Alkylthio, Arylthio, Alkoxycarbonyl, Carbamoyl oder Acyl, vorzugsweise Alkyl, Aryl, Heterocyclic, Alkylthio oder Arylthio.

Q ist Wasserstoff oder eine Abgangsgruppe wie Halogen, Alkoxy, Aryloxy, Acyloxy, Alkyl- oder Arylsulfonyloxy, Acylamino, Alkyl- oder Aryl-sulfonamido, Alkoxycarbonyloxy, Aryloxycarbonyloxy, Alkyl-, Aryl- oder Heterocyclyl-S-Carbamoylamino, ein 5- oder 6-gliedriger stickstoffhaltiger heterocyclischer Rest, Imido und Arylazo. Diese Gruppen sind gegebenenfalls wie für R₁₁ gezeigt weiter substituiert.

Vorzugsweise ist Q Halogen (z.B. Fluor, Chlor, Brom); Alkoxy (z.B. Ethoxy, Dodecyloxy, Methoxyethylcarbamoylmethoxy, Carboxypropyloxy, Methylsulfonylethoxy, Ethoxycarbonylmethoxy); Aryloxy (z.B. 4-Methylphenoxy, 4-Chlorphenoxy, 4-Methoxyphenoxy, 4-Carboxyphenoxy, 3-Ethoxycarboxyphenoxy, 3-Acetylaminophenoxy, 2-Carboxyphenoxy); Acyloxy (z.B. Acetoxy, Tetradecanoyloxy, Benzoyloxy); Alkyl-oder Aryl-sulfonyloxy (z.B. Methansulfonyloxy, Toluolsulfonyloxy); Acylamino (z.B. Dichloracetylamino, Heptafluorobutyrylamino); Alkyl- oder Arylsulfonamido (z.B. Methanesulfonamido, Trifluoromethansulfonamido, p-Toluolsulfonylamido); Alkoxycarbonyloxy (z.B. Ethoxycarbonyloxy, Benzyloxycarbonyloxy); Aryloxycarbonyloxy (z.B. Phenoxycarbonyloxy); Alkyl-, Aryl- oder Heterocyclyl-S- (z.B. Dodecylthio, 1-Carboxydodecylthio, Phenylthio, 2-Butoxy-5-t-octylphenylthio, Tetrazolylthio); Carbamoylamino (z.B. N-Methylcarbamoylamino, N-Phenylcarbamoylamino); 5- oder 6-gliedriger stickstoffhaltiger Ring (z.B. Imidazolyl, Pyrazolyl, Triazolyl, Tetrazolyl, 1,2-Dihydro-2-oxo-1-pyridyl); Imido (z.B. Succinimido, Hydantoinyl); Arylazo (z.B. Phenylazo, 4-Methoxyphenylazo).

Q kann auch entsprechende Bisverbindungen bilden durch Kondensation von 4 äquivalenten Kuppler mit einem Aldehyd oder Keton. Des weiteren kann Q photographisch wirksame Gruppen enthalten wie Entwicklungsinhibitoren oder Entwicklungsbeschleuniger. Vorzugsweise ist Q Halogen, Alkoxy, Aryloxy, Alkyl-, Aryl-thio, oder eine 5- oder 6-gliedrige stickstoffhaltige heterocyclische Gruppe, die an den Ort der Kupplung über ein Stickstoffatom gebunden ist.

Pyrazolo-tetrazole sind beschrieben in der JP-A-85/33552; Pyrazolo-pyrazole in der JP-A-85/43,695; Pyrazolo-imidazole in den JP-A-85/35732, JP-A-86/18949 und US-A-4,500,630; Pyrazolo-triazole in den JP-A-85/186,567, JP-A-86/47957, JP-A-85/215,687, JP-A-85/197,688, JP-A-85/172,982, EP-A-119,860, EP-A-173,256, EP-A-178,789, EP-A-178,788 und in Research Disclosure 84/24,624.

Weitere Pyrazoloazol-Magentakuppler sind beschrieben in: JP-A-86/28,947, JP-A-85/140,241, JP-A-85/262,160, JP-A-85/213,937, JP-A-87/278,552, JP-A-87/279,340, JP-A-88/100,457, EP-A-177,765, EP-A-176,804, EP-A-170,164, EP-A-164,130, EP-A-178,794, DE-A-3,516,996, DE-A-3,508,766 und Research Disclosure 81/20919, 84/24531 und 85/25758.

Geeignete Beispiele solcher Kuppler sind:

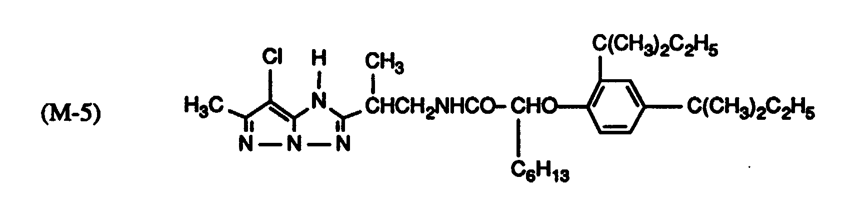

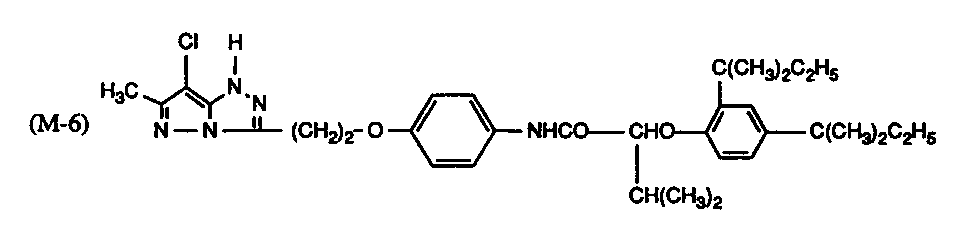

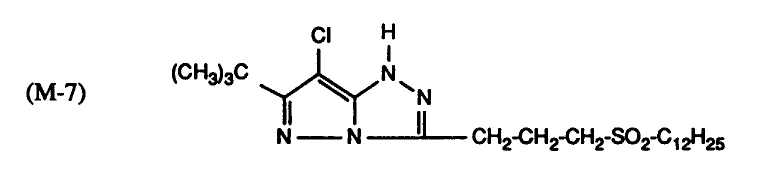

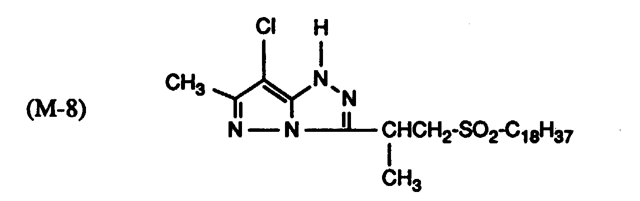

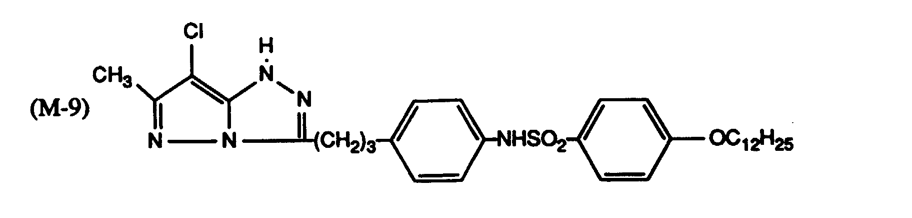

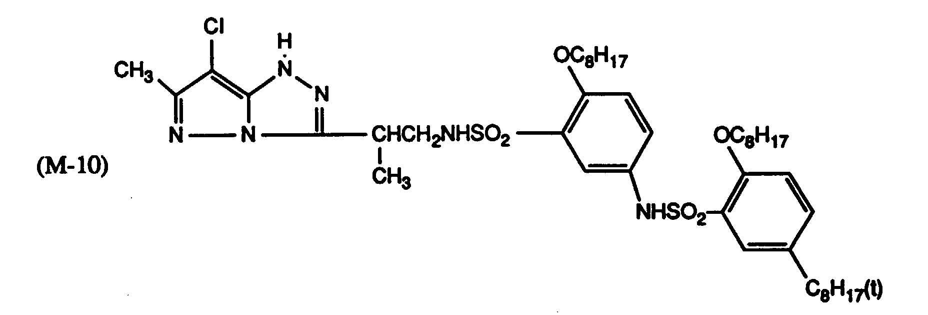

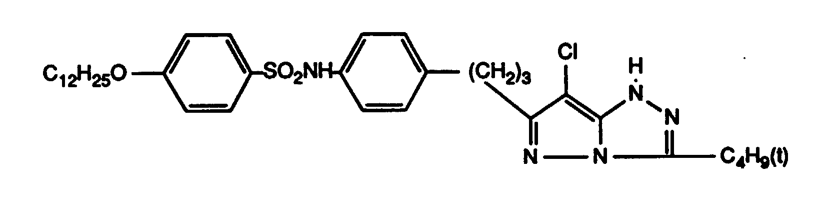

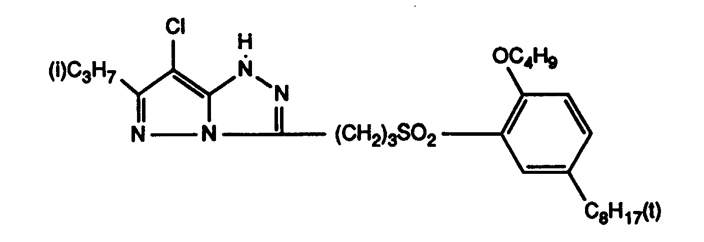

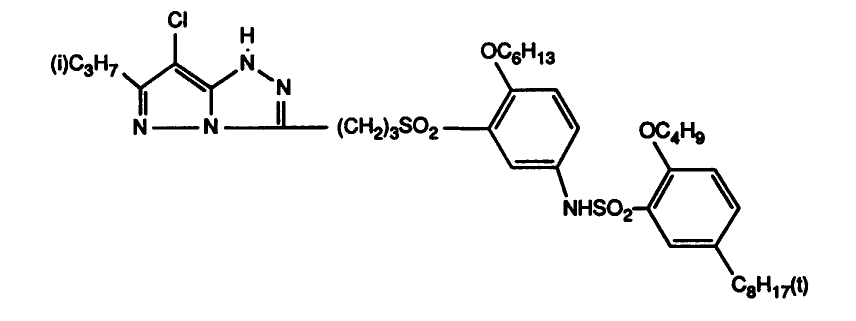

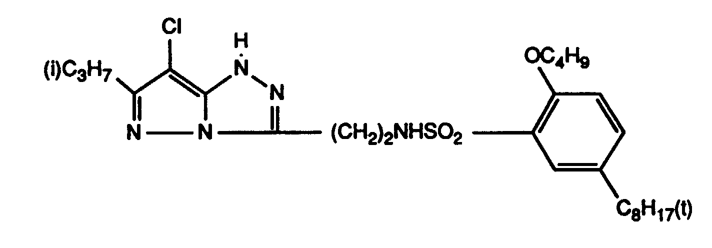

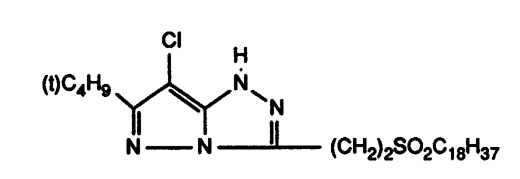

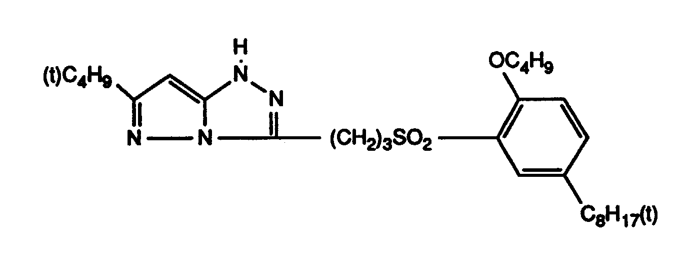

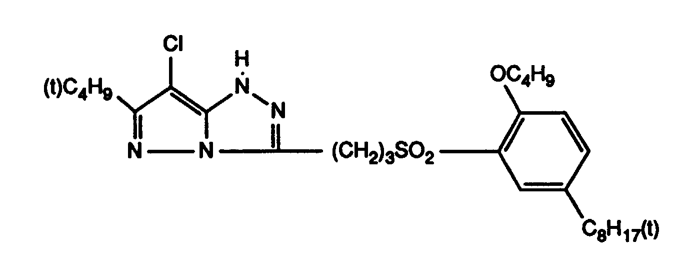

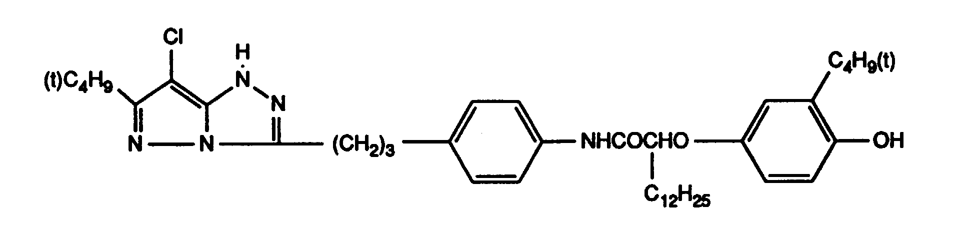

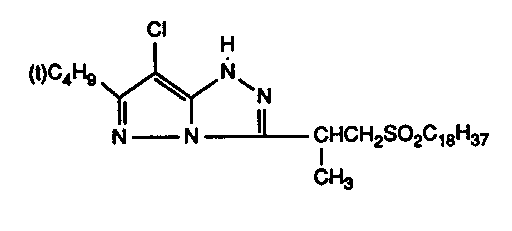

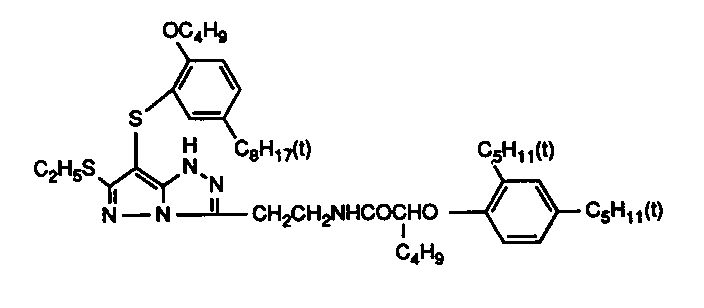

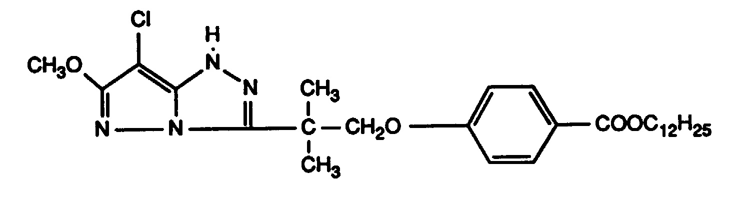

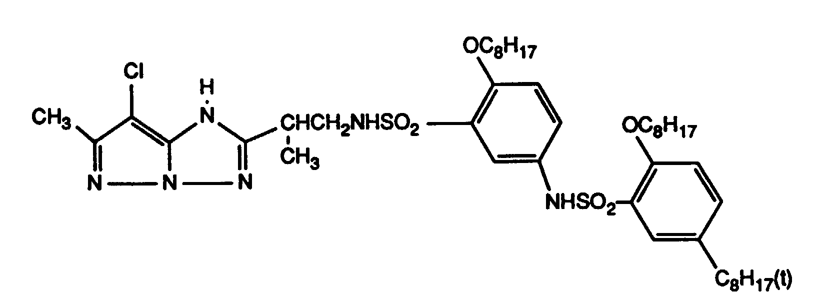

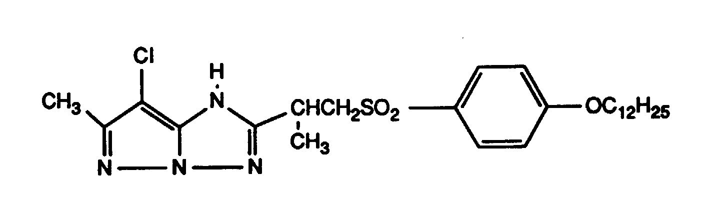

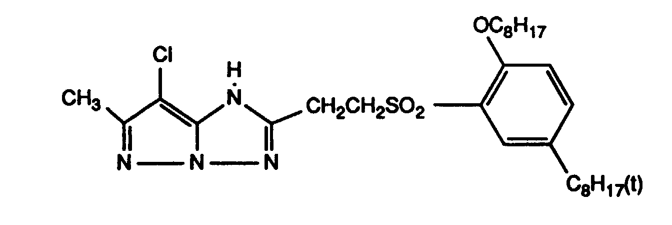

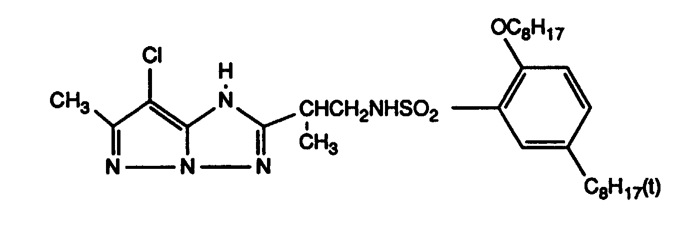

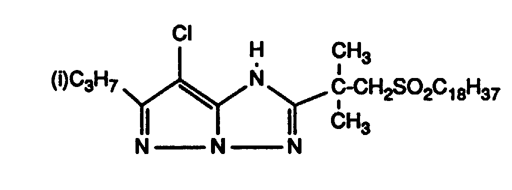

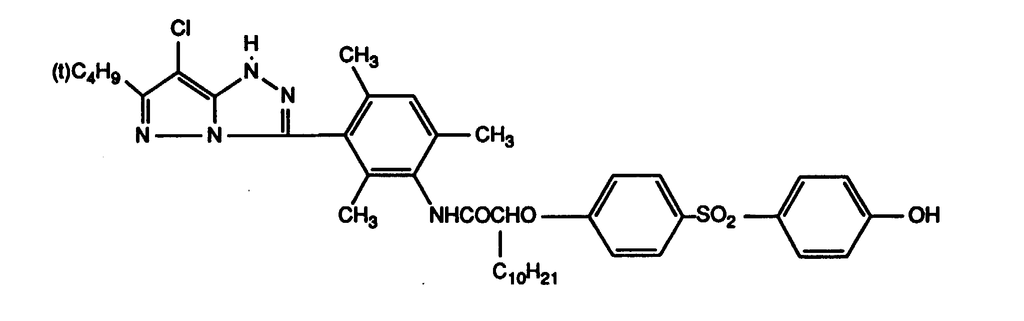

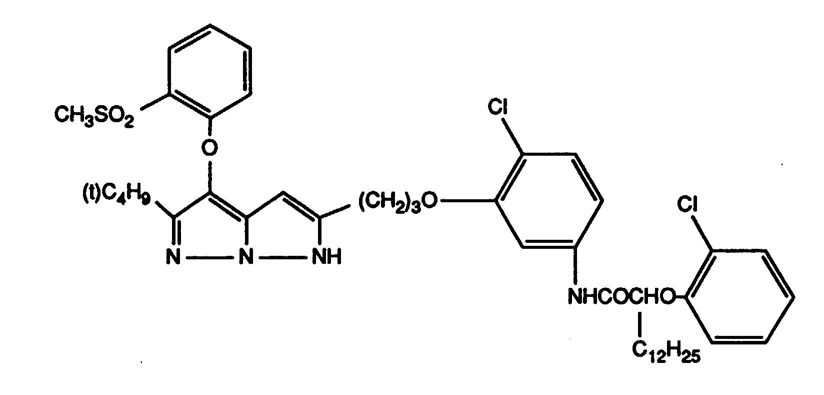

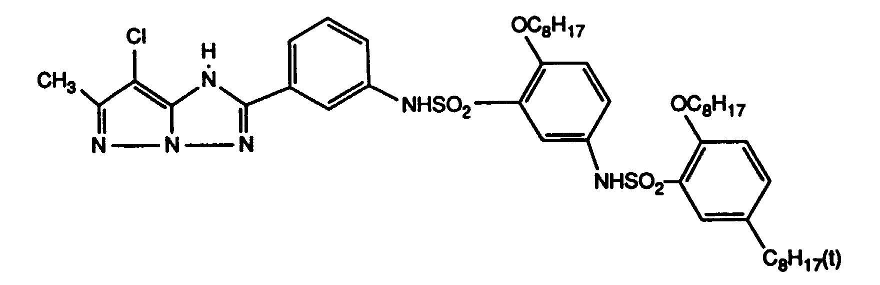

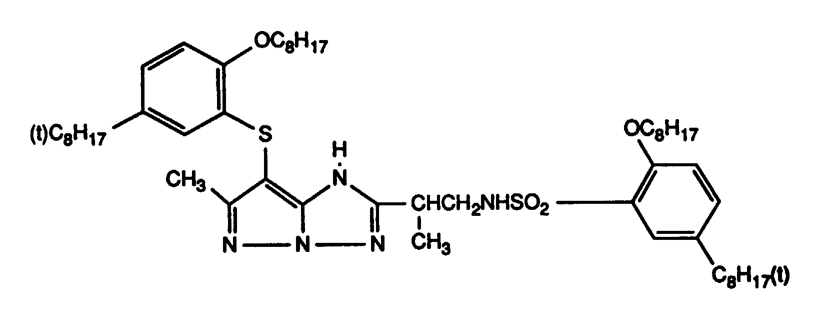

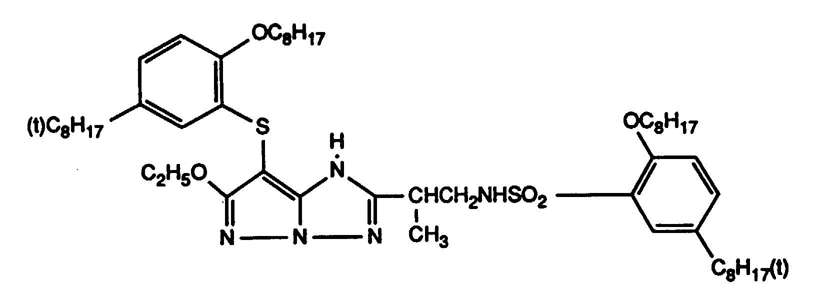

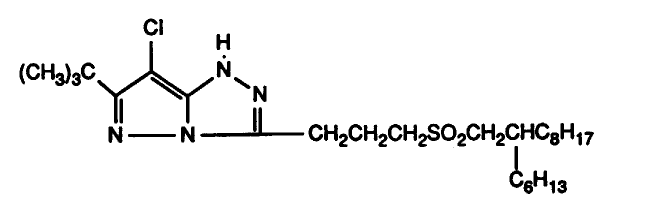

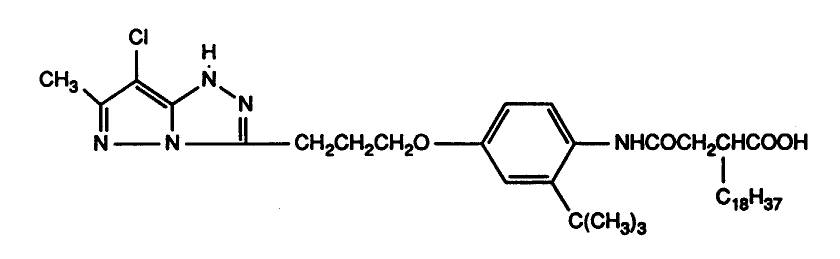

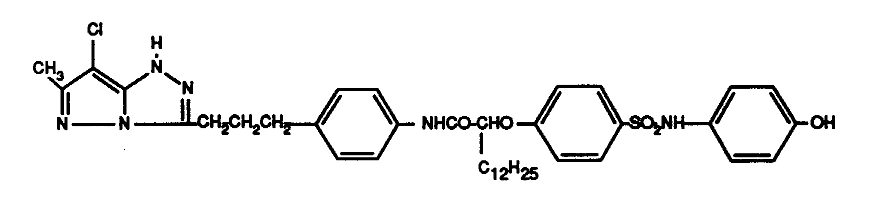

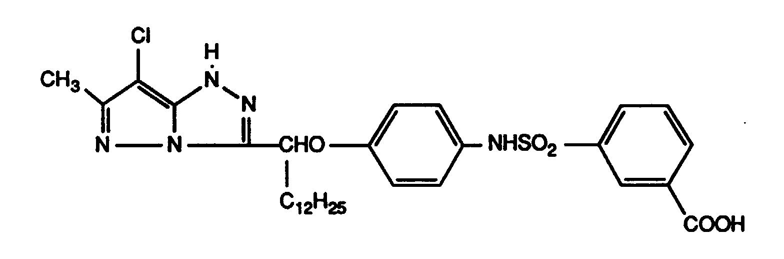

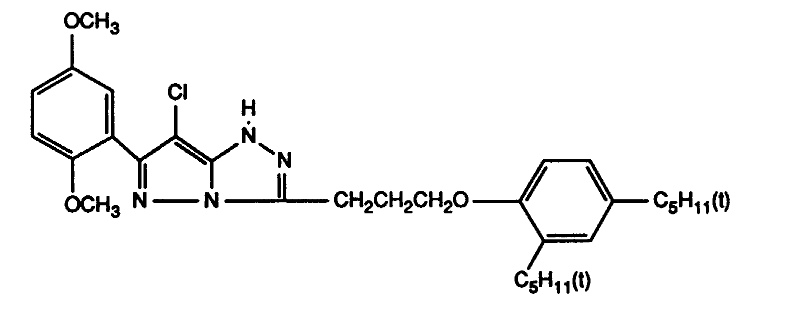

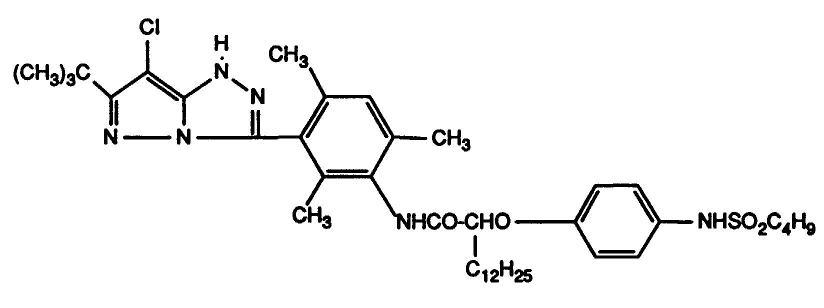

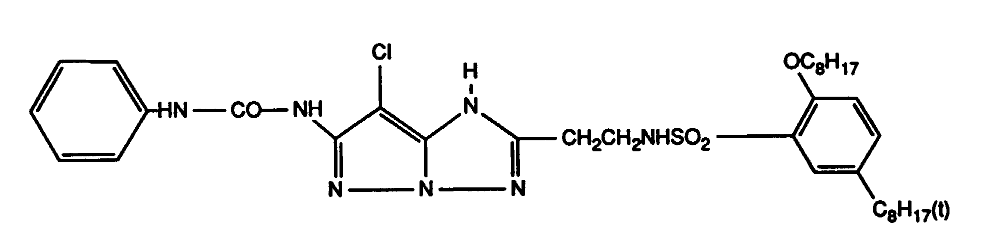

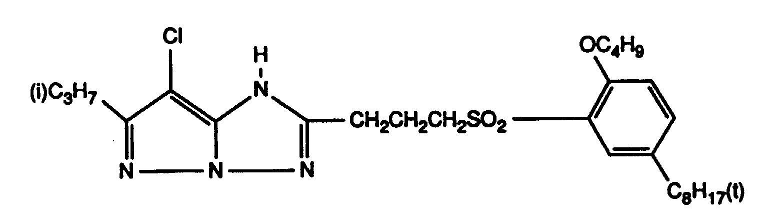

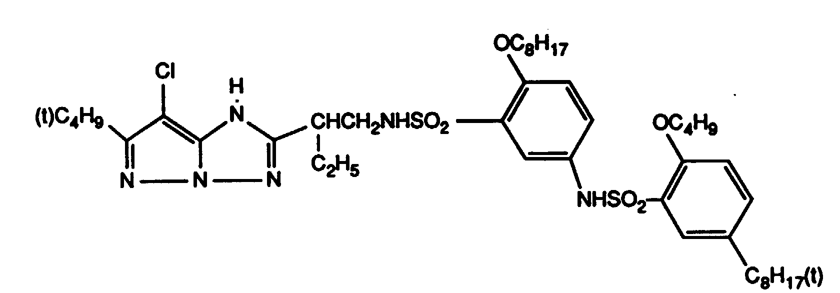

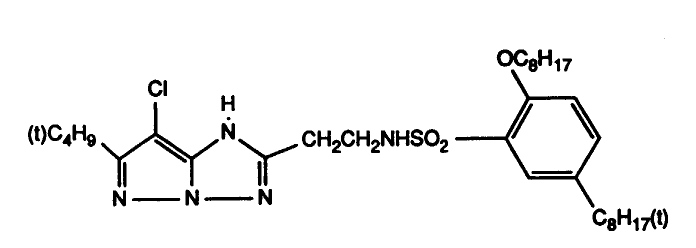

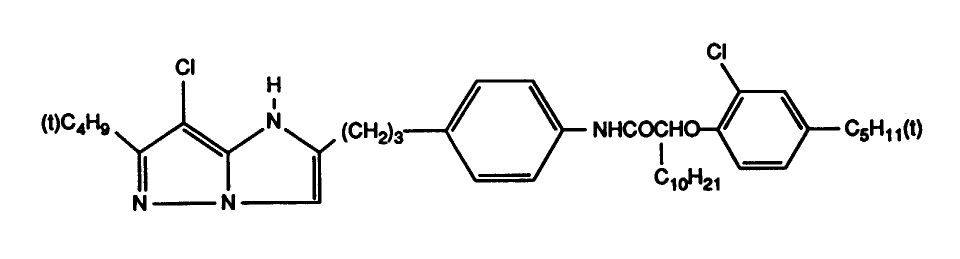

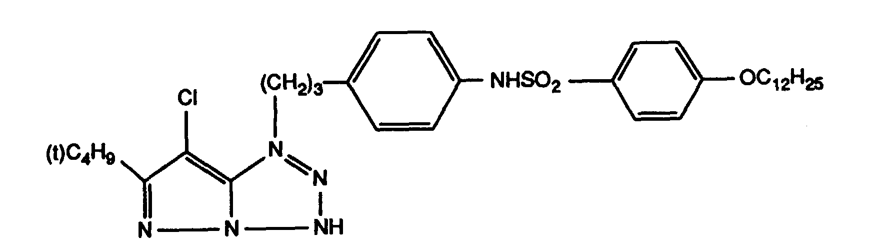

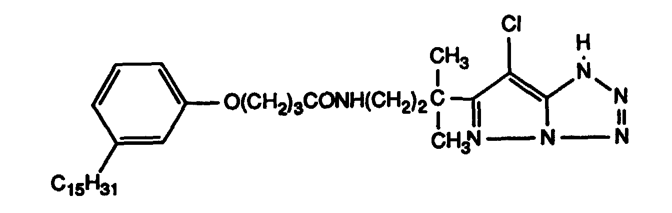

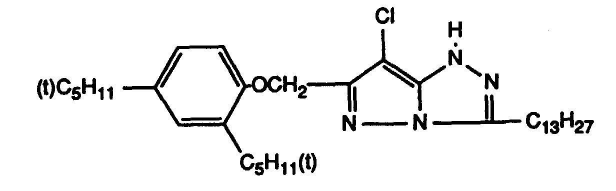

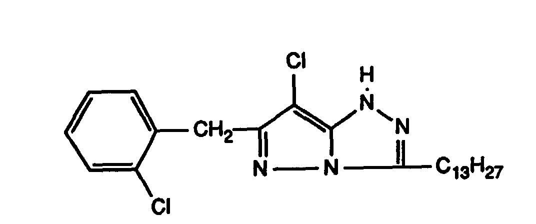

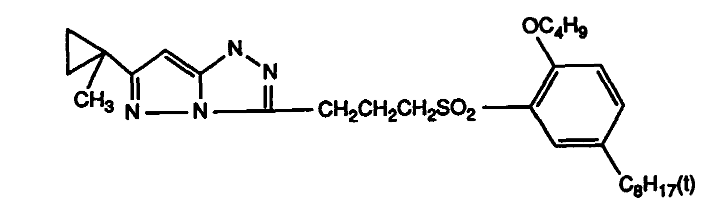

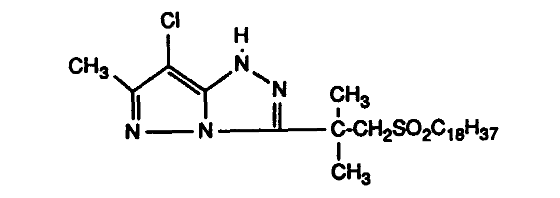

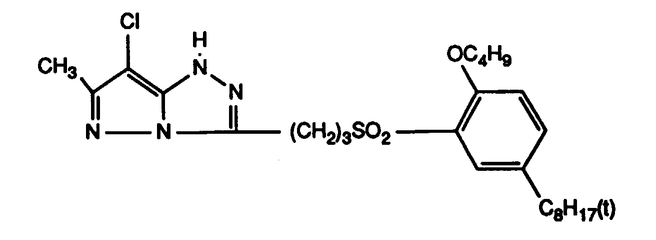

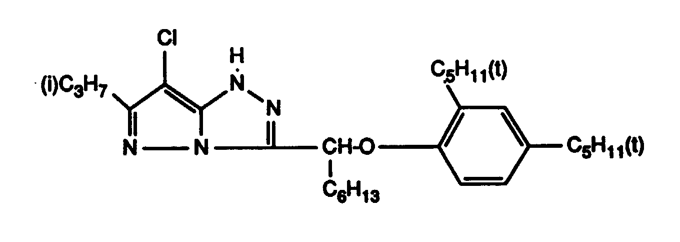

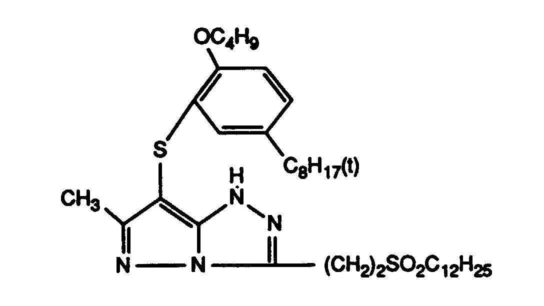

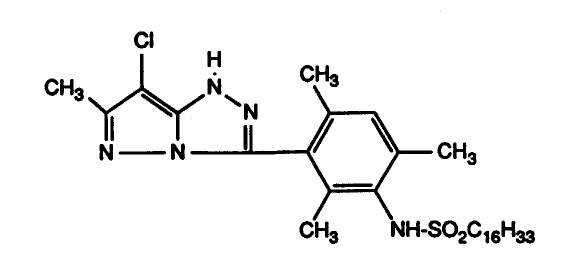

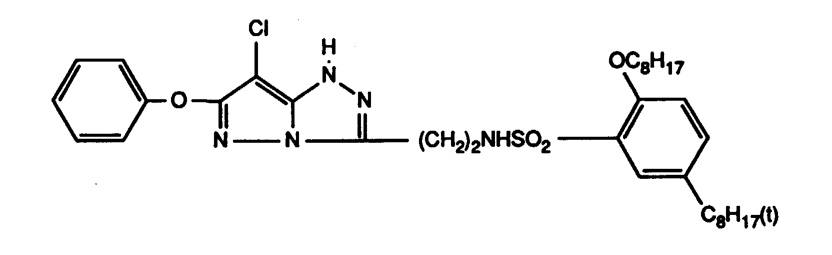

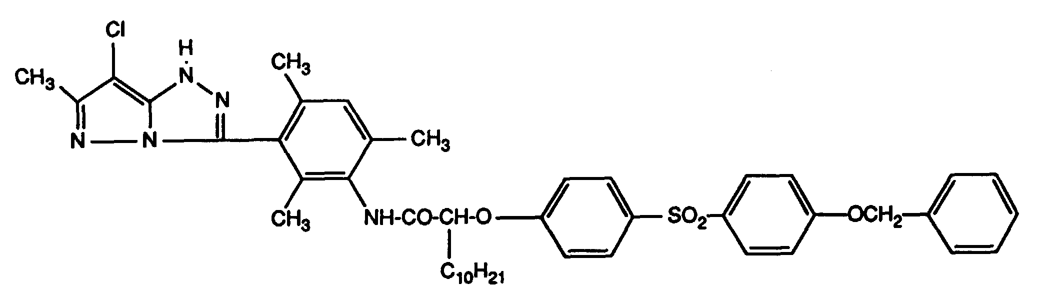

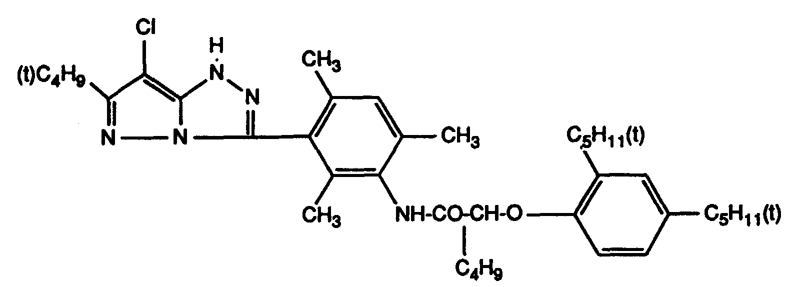

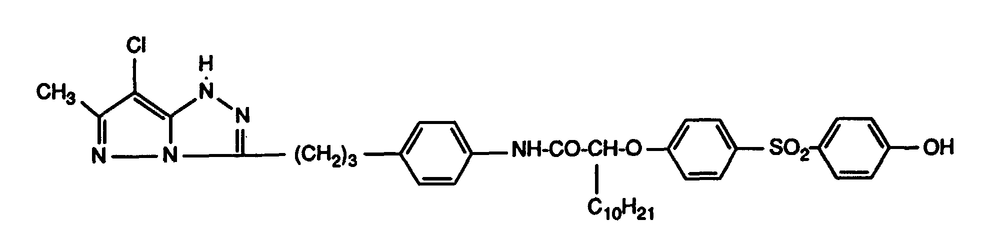

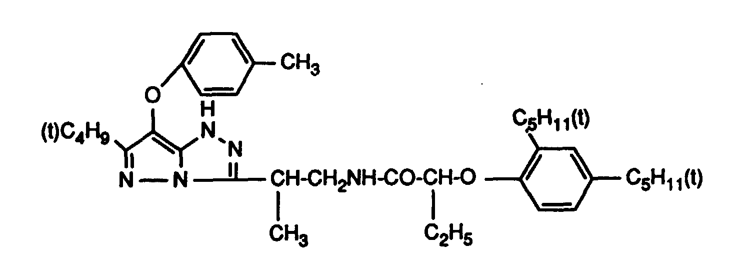

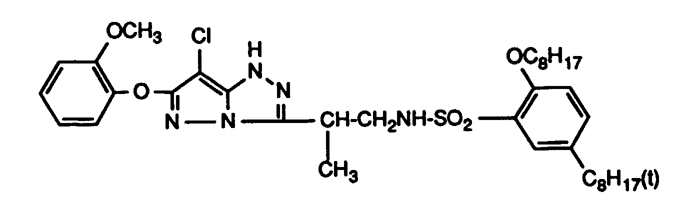

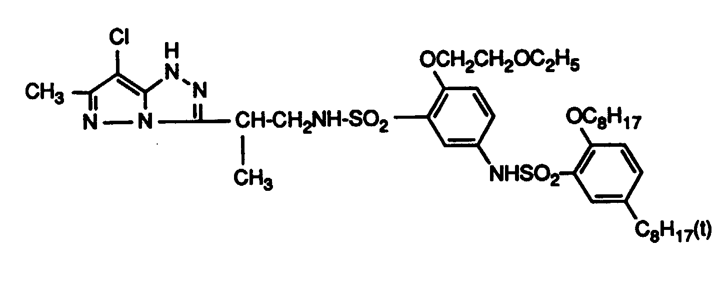

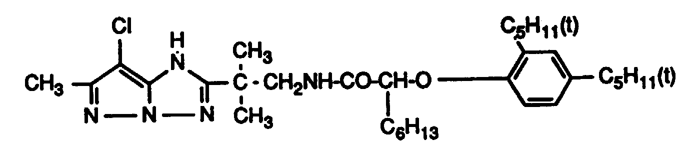

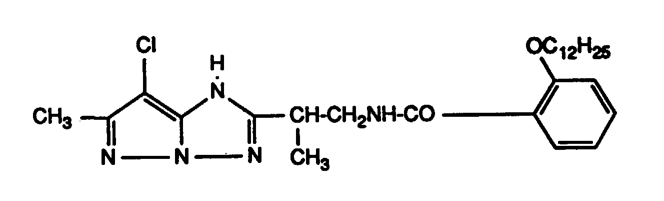

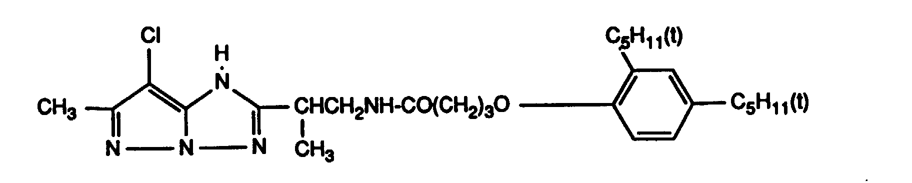

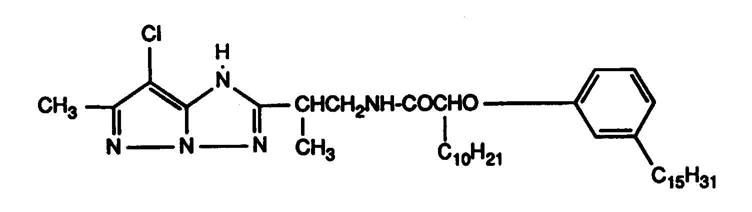

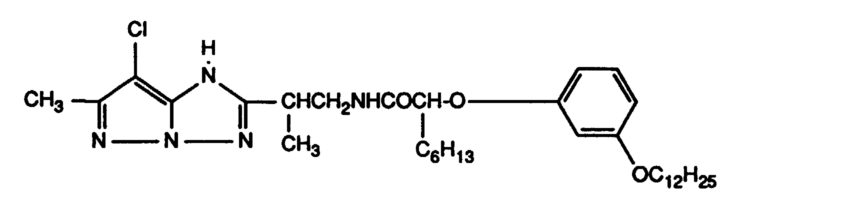

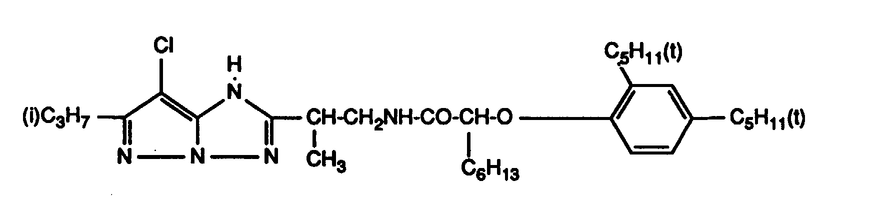

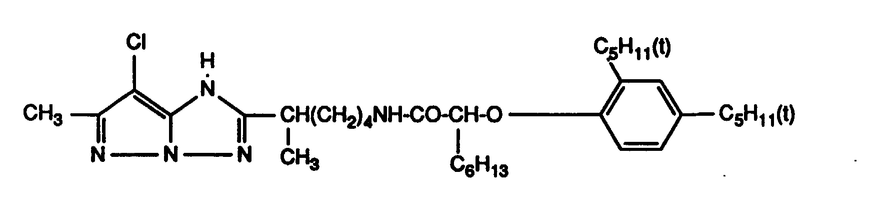

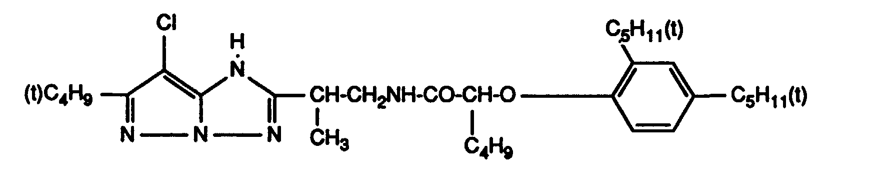

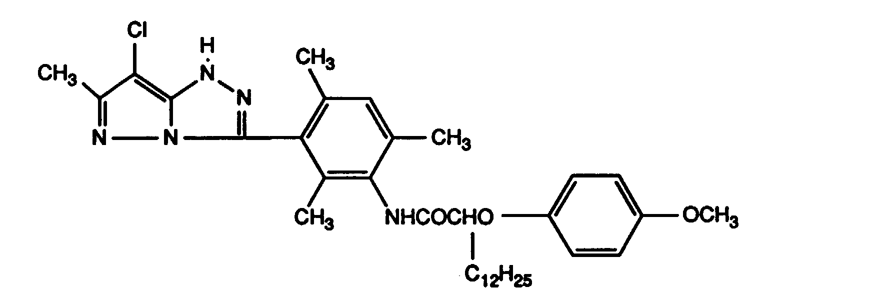

Im erfindungsgemässen Material verwendbare Gelbkuppler sind vorzugsweise Verbindungen der Formel A 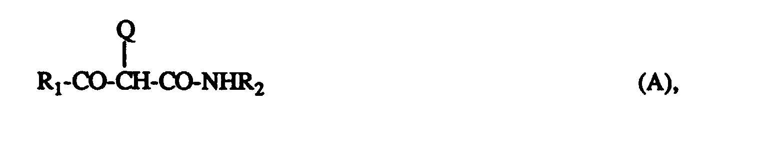 worin R₁ Alkyl oder Aryl ist, R₂ Aryl ist und Q Wasserstoff oder eine Gruppe ist, die durch Reaktion mit dem oxidierten Entwickler abgespaltet werden kann.

Eine Gruppe von Gelbkupplern sind solche Verbindungen der Formel A, in denen R₁ t-Butyl ist und R₂ eine Gruppe der Formel 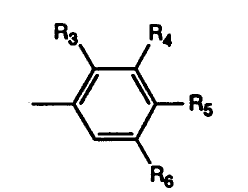 ist, worin R₃ Wasserstoff, Halogen, Alkyl oder Alkoxy bedeutet und R₄, R₅ und R₆ Wasserstoff, Halogen, Alkyl, Alkenyl, Alkoxy, Aryl, Carboxy, Alkoxycarbonyl, eine Carbamoylgruppe, eine Sulfon- oder Sulfamoylgruppe, eine Alkylsulfonamidogruppe, Acylaminogruppe, Ureidogruppe oder Aminogruppe bedeuten.

Vorzugsweise sind R₃ Chlor, R₄ und R₅ Wasserstoff und R₆ eine Acylaminogruppe. Hierzu gehören auch die Verbindungen der Formel 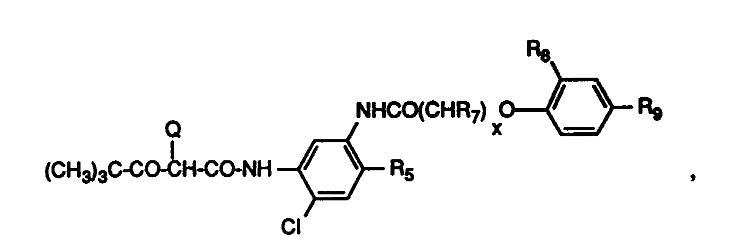 worin x O-4 ist, R₇ Wasserstoff oder Alkyl bedeutet und R₈ und R₉ Alkyl sind.

Eine andere Gruppe von Gelbkupplern entspricht der Formel B 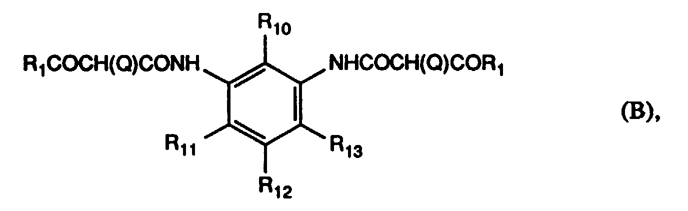 worin R₁₀ Wasserstoff, Halogen oder Alkoxy ist,
R₁₁, R₁₂ und R₁₃ Wasserstoff, Halogen, Alkyl, Alkenyl, Alkoxy, Aryl, Carboxyl, Alkoxycarbonyl, eine Carbamoylgruppe, eine Sulfongruppe, Sulfamoylgruppe, Sulfonamidogruppe, Acylaminogruppe, Ureidogruppe oder Aminogruppe bedeuten und R₁ und Q die oben angegebene Bedeutung haben.

Dazu gehören Verbindungen der Formel B, in denen R₁ t-Butyl ist, R₁₀ Chlor ist, R₁₁ und R₁₃ Wasserstoff sind und R₁₂ Alkoxycarbonyl ist.

In den Verbindungen der Formel A und B kann die Abgangsgruppe Q Wasserstoff sein oder sie ist eine heterocyclische Gruppe 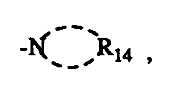 worin R₁₄ eine organische zweiwertige Gruppe ist, die den Ring zu einem 4-7-gliedrigen Ring ergänzt, oder Q ist eine Gruppe -OR₁₅, worin R₁₅ Alkyl, Aryl, Acyl oder ein heterocyclischer Rest ist.

Typische Beispiele für gebräuchliche Gelbkuppler sind die Verbindungen der folgenden Formeln:

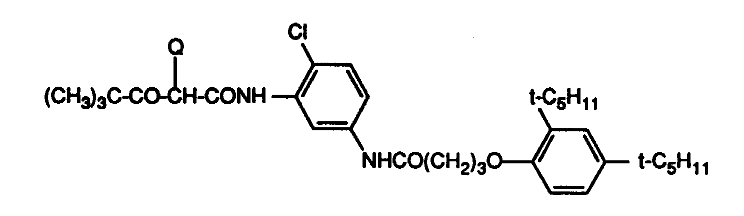

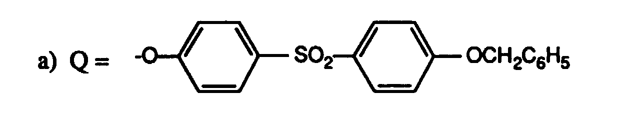

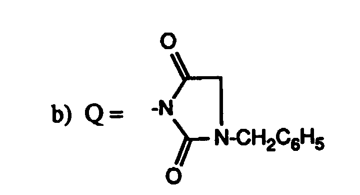

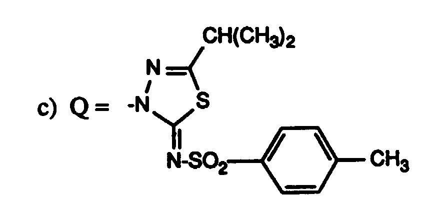

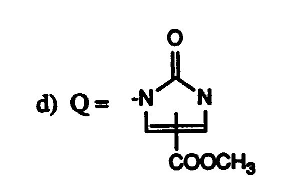

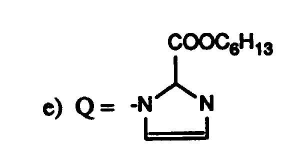

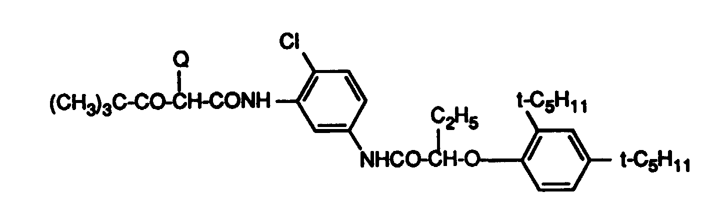

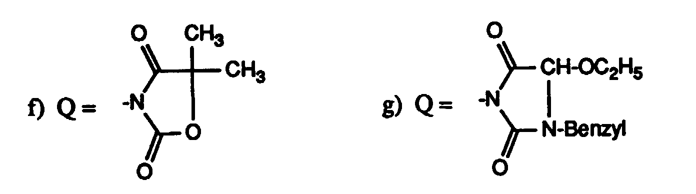

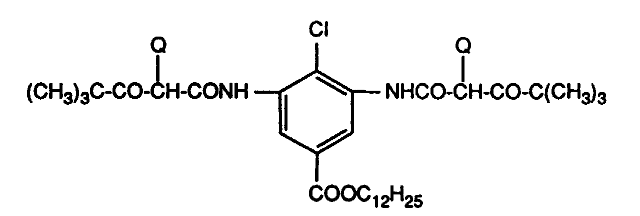

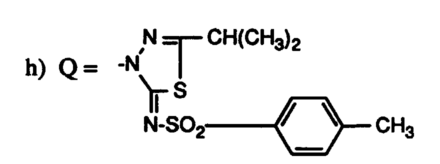

Weitere Beispiele für Gelbkuppler sind zu finden in den US-A 2,407,210, 2,778,658, 2,875,057, 2,908,513, 2,908,573, 3,227,155, 3,227,550, 3,253,924, 3,265,506, 3,277,155, 3,408,194, 3,341,331, 3,369,895, 3,384,657, 3,415,652, 3,447,928, 3,551,155, 3,582,322, 3,725,072, 3,891,445, 3,933,501, 4,115,121, 4,401,752 und 4,022,620, in den DE-A 1,547,868, 2,057,941, 2,162,899, 2,163,813, 2,213,461, 2,219,917, 2,261,361, 2,261,362, 2,263,875, 2,329,587, 2,414,006 und 2,422,812, in den GB-A 1,425,020 und 1,077,874 und in JP-A-88/123,047 und in EP-A-447,969.

Typische und bevorzugte Gelbkuppler entsprechen den Formeln:

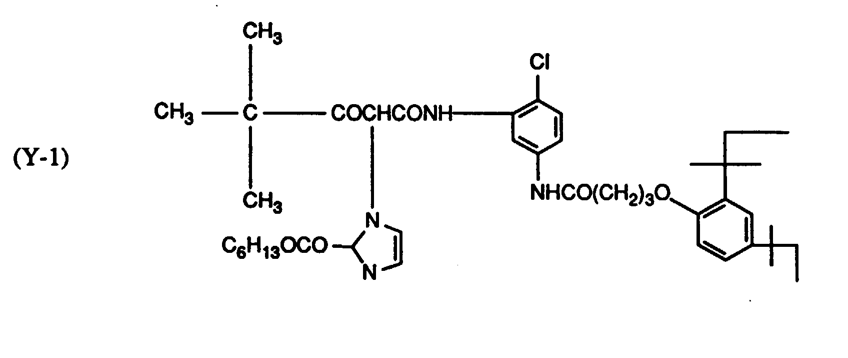

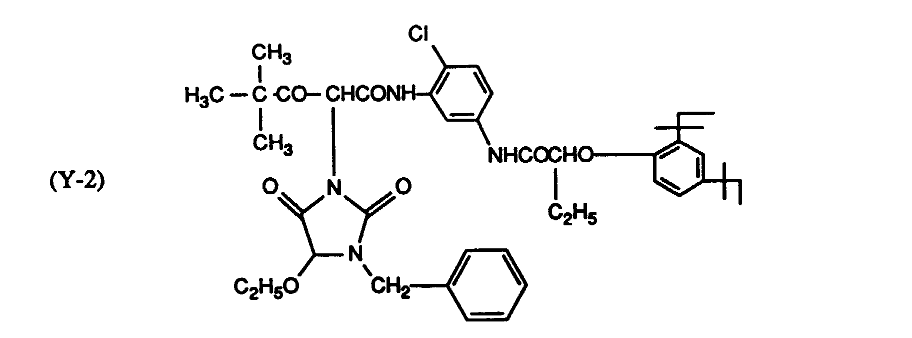

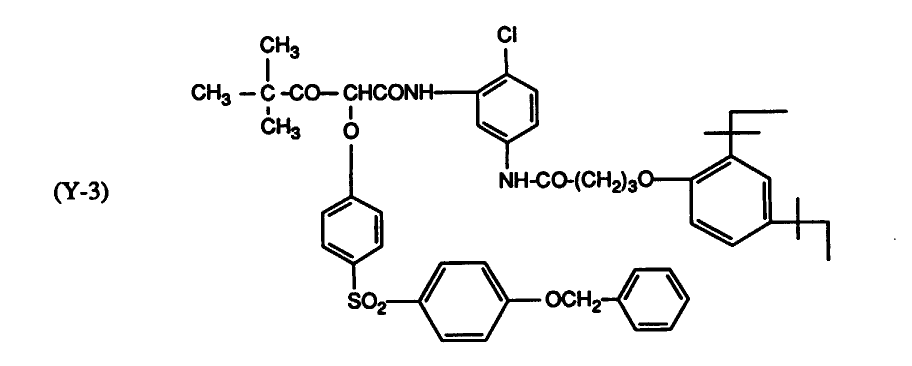

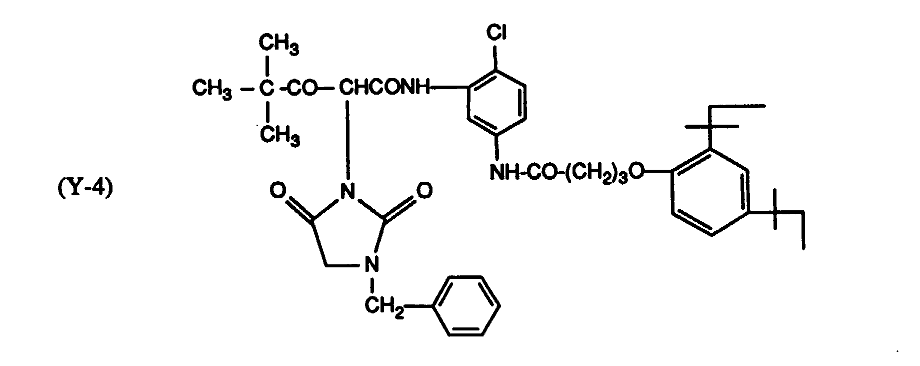

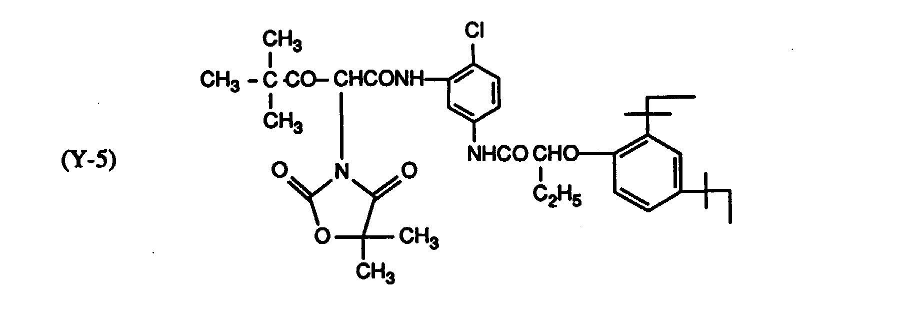

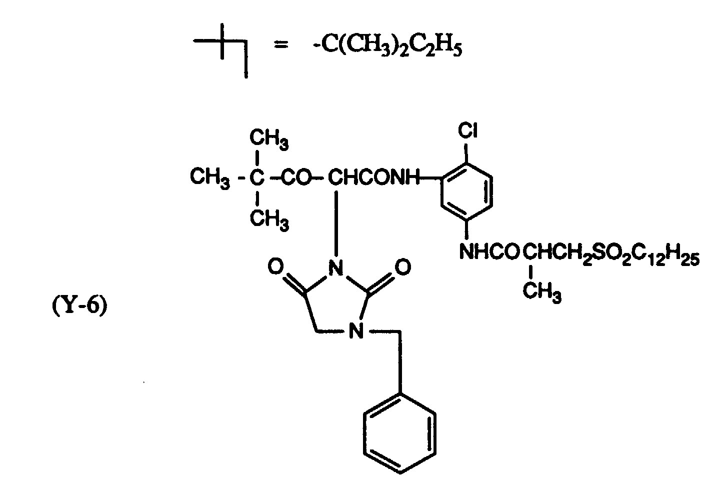

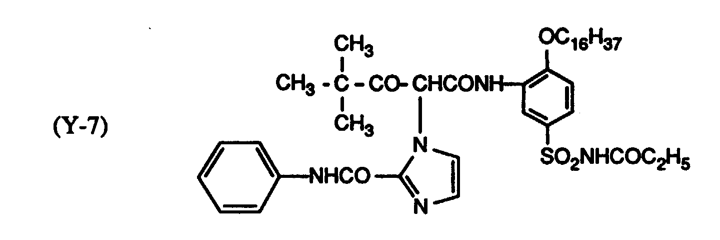

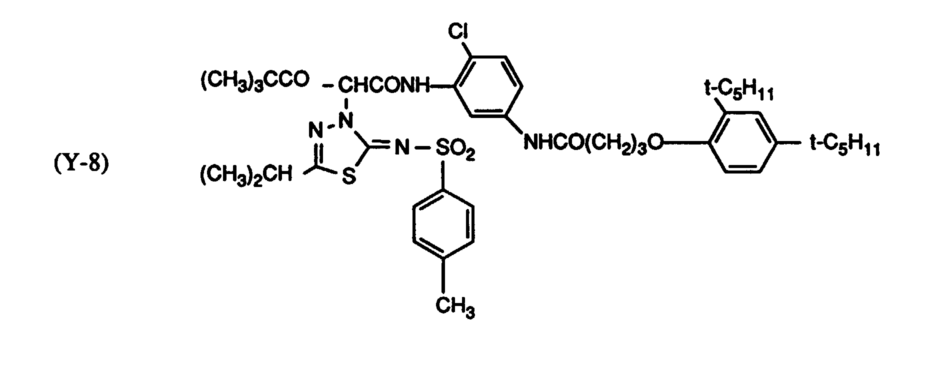

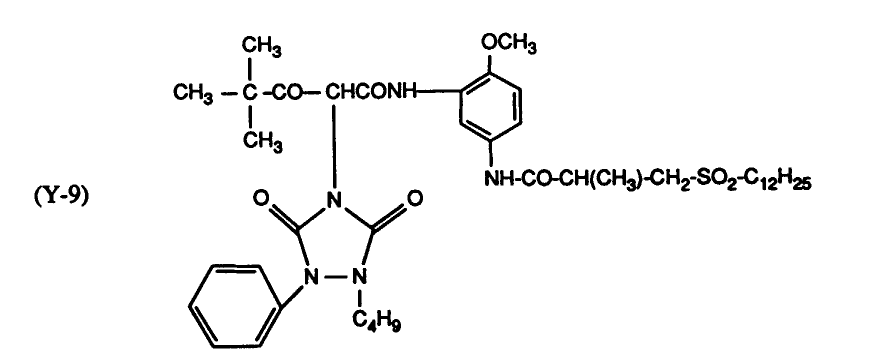

Cyankuppler können z.B. Derivate von Phenol, von 1-Naphthol oder von Pyrazolochinazolon sein. Bevorzugt sind Strukturen der Formel E, 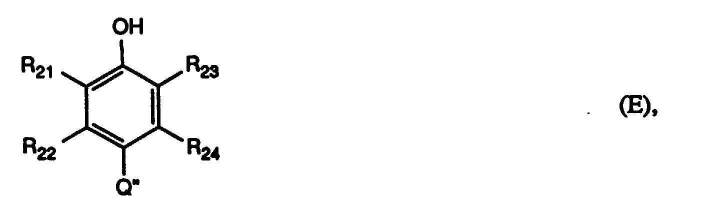 worin R₂₁, R₂₂, R₂₃ und R₂₄ Wasserstoff, Halogen, Alkyl, Carbamoyl, Amino, Sulfonamido, Phosphoramido oder Ureido sind. R₂₁ ist vorzugsweise H oder Cl, R₂₂ ist vorzugsweise eine Alkyl- oder Aminogruppe. R₂₃ ist vorzugsweise eine Aminogruppe und R₂₄ ist vorzugsweise Wasserstoff. Q'' ist Wasserstoff oder eine Abgangsgruppe, die bei der Reaktion mit dem oxidierten Entwickler abgespalten wird. Eine ausführliche Aufzählung von Cyankupplern ist im US-A-4,456,681 zu finden.

Weitere Beispiele von Cyankupplern sind in folgenden US-A- zu finden:
2,369,929, 2,423,730, 2,434,272, 2,474,293, 2,521,293, 2,521,908, 2,698,794, 2,706,684, 2,772,162, 2,801,171, 2,895,826, 2,908,573, 3,034,892, 3,046,129, 3,227,550, 3,253,294, 3,311,476, 3,386,301, 3,419,390, 3,458,315, 3,476,560, 3,476,563, 3,516,831, 3,560,212, 3,582,322, 3,583,971, 3,591,383, 3,619,196, 3,632,347, 3,652,286, 3,737,326, 3,758,308, 3,839,044, 3,880,661, 4,004,929, 4,124,396, 4,333,999, 4,463,086, 4,456,681, 4,873,183 und 4,923,791 und in den EP-A-354,549 und EP-A-398,664.

In der rotempfindlichen Silberhalogenidemulsionsschicht des erfindungsgemässen Materials kommen vorzugsweise Cyankuppler der Formel 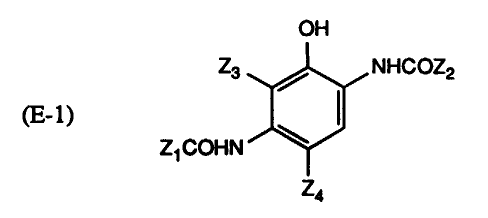 und/oder der Formel 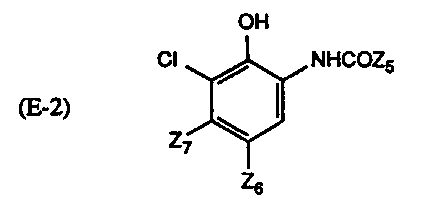 zum Einsatz, worin
Z₁ Alkyl, Aryl, Z₂ Alkyl, Cycloalkyl, Aryl, eine heterocyclische Gruppe, oder eine Ballastgruppe, Z₃ Wasserstoff oder Halogen ist, Z₁ und Z₃ zusammen einen Ring bilden können, und Z₄ Wasserstoff oder eine Abgangsgruppe ist, und Z₅ eine Ballastgruppe, Z₆ Wasserstoff oder eine Abgangsgruppe und Z₇ Alkyl ist.

Beispiele von gebräuchlichen Cyankupplern sind die folgenden:

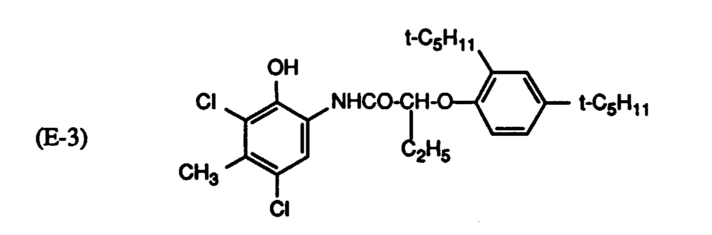

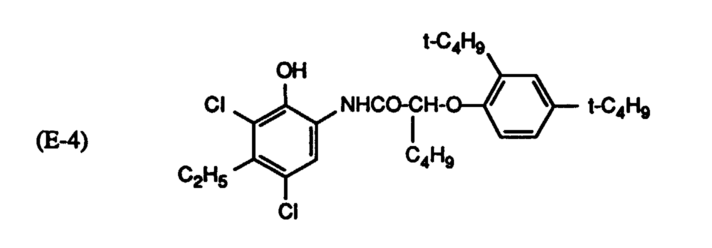

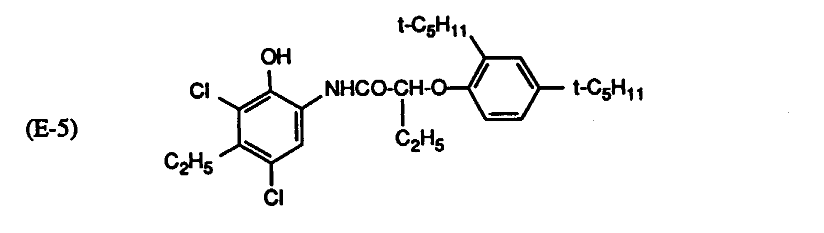

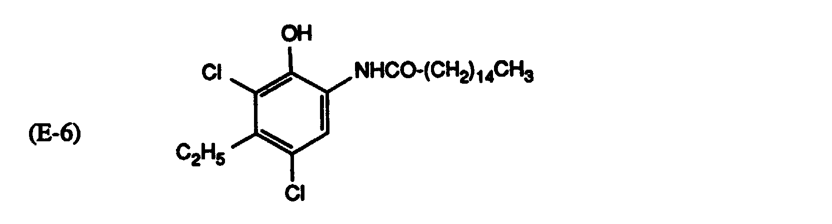

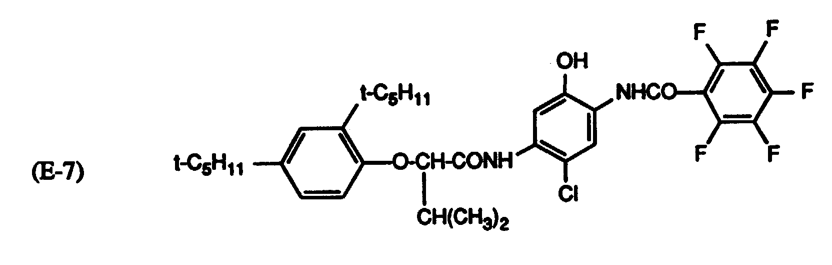

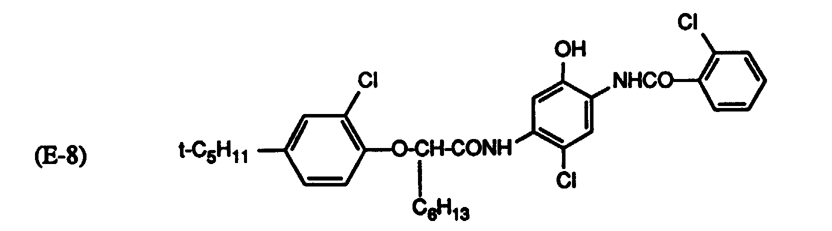

Die für farbfotographische Materialien üblicherweise verwendeten Farbentwickler sind p-Dialkylaminoaniline. Beispiele hierfür sind 4-Amino-N,N-diethylanilin, 3-Methyl-4-amino-N,N-diethylanilin, 4-Amino-N-ethyl-N-α-hydroxyethylanilin, 3-Methyl-4-amino-N-ethyl-N-α-hydroxyethylanilin, 3-Methyl-4-amino-N-ethyl-N-α-methansulphonamidoethylanilin 3-Methyl-4-amino-N-ethyl-N-α-hydroxyethylanilin, 3-Methyl-4-amino-N-ethyl-N-α-methoxyethyl-anilin, 3-α-Methansulphonamidoethyl-4-amino-N,N-diethylanilin, 3-Methoxy-4-amino-N-ethyl-N-α-hydroxyethylanilin, 3-Methoxy-4-amino-N-ethyl-N-α-methoxyethylanilin, 3-Acetamido-4-amino-N,N-diethylanilin, 4-Amino-N,N-dimethylanilin, N-Ethyl-N-α-[α'-(α"-methoxyethoxy)ethoxy]ethyl-3-methyl-4-aminoanilin, N-Ethyl-N-α-(α'-methoxyethoxy)ethyl-3-methyl-4-aminoanilin, sowie die Salze solcher Verbindungen, wie z.B. Sulfate, Hydrochloride oder Toluolsulfonate.

Die erfindungsgemässen Verbindungen der Formeln (I), (II), (III), (IV), die Magentakuppler sowie andere Farbkuppler können in bekannter Weise in das farbphotographische Material eingearbeitet werden, z.B. in Silberhalogenidemulsionen, die Gelatine und/oder andere Bindemittel enthalten. Sie finden z.B. Anwendung in Silberbromid-, Silberchlorid- oder Silberjodidemulsionen oder in Emulsionen, die Gemische von Silberhalogeniden enthalten, wie Silberbromid/jodid- oder Siblberchlorid/bromid-emulsionen.

Die erfindungsgemässen Verbindungen der Formeln (I), (II), (III) und (IV) können zusammen mit dem Magentakuppler und gegebenenfalls weiteren Zusätzen in das farbphotographische Material eingearbeitet werden, indem man sie in hochsiedenden organischen Lösungsmitteln vorlöst. Vorzugsweise verwendet man Lösungsmittel, die höher als 160°C sieden. Typische Beispiele solcher Lösungsmittel sind die Ester von Phthalsäure, Phosphorsäure, Zitronensäure, Benzoesäure oder von Fettsäuren, sowie Alkylamide und Phenole.

Meist verwendet man zusätzlich noch ein niedrig siedendes Lösungsmittel, um das Einarbeiten der Zusätze in das farbphotographische Material zu erleichtern. Beispiele für solche Lösungsmittel sind Ester wie z.B. Ethylacetat, Alkohole wie z.B. Butanol, Ketone wie z.B. Methyl-isobutyl-keton, Chlorkohlenwasserstoffe wie z.B. Methylenchlorid, oder Amide wie z.B. Dimethylformamid. Sind die Zusätze selbst flüssig, so kann man sie auch ohne Zuhilfenahme von Lösungsmitteln in das Photomaterial einarbeiten.

Die erfindungsgemässen Verbindungen können gegebenenfalls als Öl in der Gelatineschicht dispergiert werden.

Weitere Details über verwendbare hochsiedende Lösungsmittel sind in den folgenden Veröffentlichungen zu finden:

Phosphate: GB-A-791,219, BE-A-755,248, JP-A-76/76739, 78/27449, 78/218,252, 78/97573, 79/148,133, 82/216,177, 82/93323 und 83/216,177 und EP-A265,296.
Phthalate: GB-A-791,219, JP-A-77/98050, 82/93322, 82/216,176, 82/218,251, 83/24321, 83/45699, 84/79888.
Amide: GB-A-791,129, JP-A-76/105,043, 77/13600, 77/61089, 84/189,556, 87/239,149, EP-A-270,341, WO 88/00723.
Phenole: GB-A-820,329, FR-A-1,220,657, JP-A-69/69946, 70/3818, 75/123,026, 75/82078, 78/17914, 78/21166, 82/212,114 und 83/45699.

Andere sauerstoffhaltige Verbindungen: US-A-3,748,141, 3,779,765, JP-A-73/75126, 74/101,114, 74/10115, 75/101,625, 76/76740, 77/61089, EP-A-304,810 und BE-A-826,039.

Sonstige Verbindungen: W-A-72/115,369, 72/130,258, 73/127,521, 73/76592, 77/13193, 77/36294, 79/95233, 91/2,748, 83/105,147 und Research Disclosure 82/21918.

Die Menge an hochsiedendem Lösungsmittel liegt z.B. im Bereich von 50 mg bis 2 g pro m² Träger, vorzugsweise von 200 mg bis 1 g pro m².

Die photographischen Schichten können ferner Farbschleier-Inhibitoren enthalten. Diese verhindern das Entstehen von Farbschleiern, wie sie beispielsweise durch Reaktion des Kupplers mit unabsichtlich oxidiertem Entwickler oder mit Nebenprodukten des Farbbildungsprozesses entstehen. Solche Farbschleierinhibitoren sind meist Hydrochininderivate, können aber auch Derivate von Aminophenolen, von Gallussäure oder von Ascorbinsäure sein. Typische Beispiele hierfür sind in folgenden Veröffentlichungen zu finden:
US-A-2,360,290, 2,336,327, 2,403,721, 2,418,613, 2,675,314, 2,701,197, 2,704,713, 2,728,659, 2,732,300, 2,735,365; EP-A-124,877, EP-A-277,589, EP-A-338,785; JP-A-75/92988, 75/92989, 75/93928, 75/110,337, 84/5,247 und 77/146,235.

Die photographischen Schichten können auch sogenannte DIR-Kuppler (DIR bedeutet Development Inhibition Release) enthalten, die mit dem oxidierten Entwickler farblose Verbindungen ergeben. Sie werden zugesetzt zur Verbesserung der Schärfe und der Körnigkeit der Farbbilder.

Die photographischen Schichten im erfindungsgemässen Material können auch UV-Absorber enthalten. Diese filtern das UV-Licht aus und schützen damit die Farbstoffe, die Kuppler oder sonstigen Komponenten gegen Lichtabbau. Beispiele für solche UV-Absorber sind 2-(2-Hydroxyphenyl)-benztriazole, 2-Hydroxyphenyl-1,3,5-triazine, 2-Hydroxybenzophenone, Salicylsäureester, Acrylnitrilderivate oder Thiazoline. Solche UV-Absorber sind z.B. in folgenden Veröffenlichungen näher erläutert: US-A-3,314,794, 3,352,681, 3,705,805, 3,707,375, 4,045,229, 3,700,455, 3,533,794, 3,698,907, 3,705,805, 3,738,837 und JP-A-71/2784. Bevorzugte UV-Absorber sind die 2-(2-Hydroxyphenyl)-benztriazole (HBT) der Formel 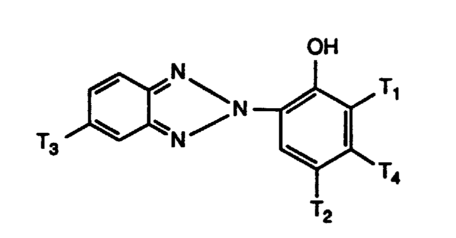 worin T₁, T₂ und T₃ unabhängig voneinander Wasserstoff, Halogen, Alkyl, Alkyl substituiert mit einer Carbonsäureestergruppe, Alkoxy, Aryloxy, Hydroxyl oder Acyloxy sind, und T₄ Wasserstoff, Alkoxy, Aryloxy oder Acyloxy ist.

Besonders bevorzugt sind solche HBT-Verbindungen, welche bei Zimmertemperatur flüssig sind.

Beispiele der bevorzugten HBT-Verbindungen sind:

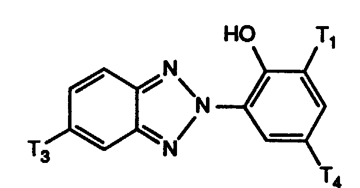

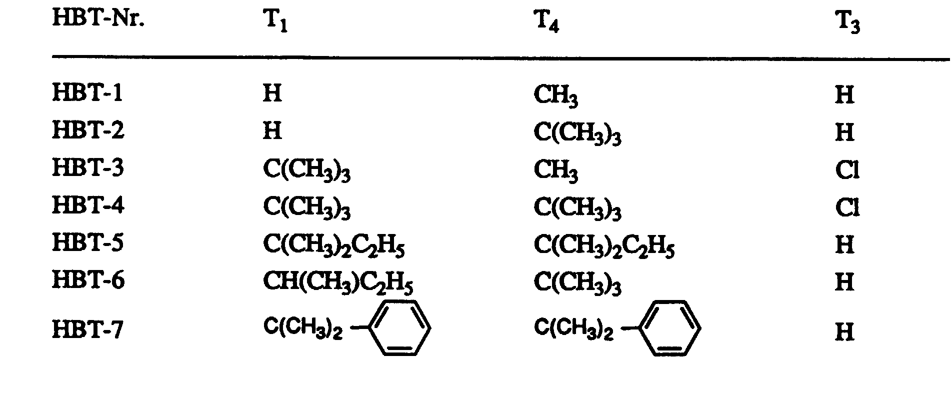

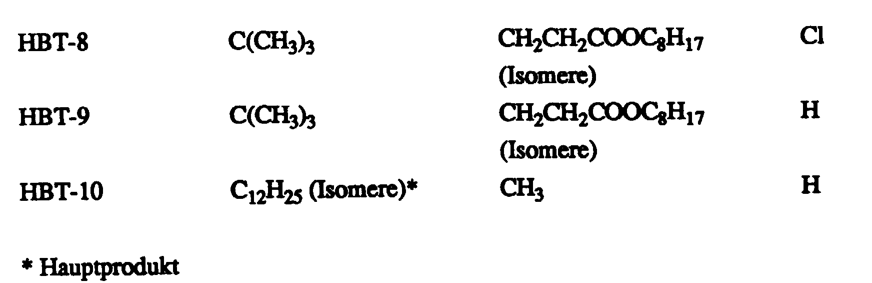

Andere bevorzugte UV-Absorber sind 2-Hydroxyphenyl-1,3,5-triazine der Formel 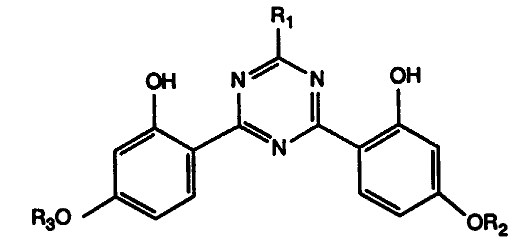 worin R₁ eine Gruppe der Formel 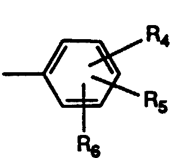 ist, wobei R₄, R₅ und R₆ unabhängig voneinander Wasserstoff, C₁-C₁₂-Alkyl oder Halogen ist oder R₁ eine Gruppe der Formel 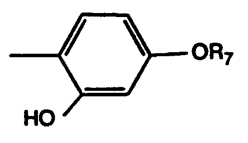 bedeutet und
R₂, R₃ und R₇ unabhängig voneinander einwertige organische Reste sind. Bevorzugt sind R₂, R₃ und R₇ unabhängig voneinander ein Rest CH₂CH(OR₈)CH₂OR₉, wobei R₈ Wasserstoff oder Acetyl und R₉ C₁-C₁₈-Alkyl ist.

Die photographischen Schichten können auch phenolische Verbindungen enthalten, die als Lichtschutzmittel für das Farbbild sowie als Mittel gegen Farbschleier wirken. Sie können in einer lichtempfindlichen Schicht (Farbschicht) oder in einer Zwischenschicht enthalten sein, allein oder zusammen mit anderen Additiven. Solche Verbindungen sind z.B. in den folgenden Veröffentlichungen näher beschrieben: US-A-3,700,455, 3,591,381, 3,573,052, 4,030,931, 4,174,220, 4,178,184, 4,228,235, 4,279,990, 4,346,165, 4,366,226, 4,447,523, 4,528,264, 4,581,326, 4,562,146, 4,559,297, GB-A-1,309,277, 1,547,302, 2,023,862, 2,135,788, 2,139,370, 2,156,091; DE-A-2,301,060, 2,347,708, 2,526,468, 2,621,203, 3,323,448; DD-A-200,691, 214,468; EP-A-106,799, 113,124, 125,522, 159,912, 161,577, 164,030, 167,762, 176,845, 246,766, 320,776; JP-A-74/134,326, 76/127,730, 76/30462, 77/3822, 77/154,632, 78/10842, 79/48535, 79/70830, 79/73032, 79/147,038, 79/154,325, 79/155,836, 82/142,638, 83/224,353, 84/5246, 84/72443, 84/87456, 84/192,246, 84/192,247, 84/204,039, 84/204,040, 84/212,837, 84/220,733, 84/222,836, 84/228,249, 86/2540, 86/8843, 86/18835, 86/18836, 87/11456, 87/42245, 87/62157, 86/6652, 89/137,258 sowie in Research Disclosure 79/17804.

Die photographischen Schichten können auch gewisse Phosphor-III-Verbindungen, insbesondere Phosphite und Phosponite, enthalten. Diese fungieren als Lichtschutzmittel für die Farbbilder sowie als Dunkellager-Stabilisator für Magentakuppler. Man setzt sie vorzugsweise den hochsiedenden Lösungsmitteln zu, zusammen mit dem Kuppler. Solche Phosphor-III-Verbindungen sind z.B. in den folgenden Veröffentlichungen näher beschrieben: US-A-4,407,935, US-A-4,436,811, US-A-4,956,406, EP-A-181,289, JP-A-73/32728, JP-A-76/1420 und JP-A-55/66741.

Die photographischen Schichten können auch metallorganische Komplexe enthalten, die Lichtschutzmittel für die Farbbilder sind, insbesondere für die Magenta-Farbstoffe. Solche Verbindungen und deren Kombination mit anderen Additiven sind z.B. in folgenden Veröffentlichungen näher beschrieben: US-A-4,050,938, 4,239,843, 4,241,154, 4,242,429, 4,241,155, 4,242,430, 4,273,854, 4,246,329, 4,271,253, 4,242,431, 4,248,949, 4,245,195, 4,268,605, 4,246,330, 4,269,926, 4,245,018, 4,301,223, 4,343,886, 4,346,165, 4,590,153; JP-A-81/167,138, 81/168,652, 82/30834, 82/161,744; EP-A-137,271, 161,577, 185,506; DE-A-2,853,865.

Die photographischen Schichten können auch Hydrochinonverbindungen enthalten. Diese wirken als Lichtschutzmittel für die Farbkuppler und für die Farbbilder sowie als Abfänger von oxidiertem Entwickler in Zwischenschichten. Sie werden vor allem in der Magentaschicht verwendet. Solche Hydrochinon-Verbindungen und deren Kombinationen mit anderen Additiven sind z.B. in folgenden Veröffentlichungen näher beschrieben: US-A-2,360,290, 2,336,327, 2,403,721, 2,418,613, 2,675,314, 2,701,197, 2,710,801, 2,732,300, 2,728,659, 2,735,765, 2,704,713, 2,937,086, 2,816,028, 3,582,333, 3,637,393, 3,700,453, 3,960,570, 3,935,016, 3,930,866,4,065,435, 3,982,944, 4,232,114, 4,121,939, 4,175,968, 4,179,293, 3,591,381, 3,573,052, 4,279,990, 4,429,031, 4,346,165, 4,360,589, 4,346,167, 4,385,111, 4,416,978,4,430,425, 4,277,558,4,489,155, 4,504,572,4,559,297, FR-A-885,982; GB-A-891,158, 1,156,167, 1,363,921, 2,022,274, 2,066,975, 2,071,348, 2,081,463, 2,117,526, 2,156,091; DE-A-2,408,168, 2,726,283, 2,639,930, 2,901,520, 3,308,766, 3,320,483, 3,323,699; DD-A-216,476, 214,468, 214,469, EP-A-84290, 110,214, 115,305, 124,915, 124,877, 144,288, 147,747, 178,165, 161,577; JP-A-75/33733, 75/21249, 77/128,130, 77/146,234, 79/70036, 79/133,131, 81/83742, 81/87040, 81/109,345, 83/134,628, 82/22237, 82/112,749, 83/17431, 83/21249, 84/75249, 84/149,348, 84/182,785, 84/180,557, 84/189,342, 84/228,249, 84/101,650, 79/24019, 79/25823, 86/48856, 86/48857, 86/27539, 86/6652, 86/72040, 87/11455, 87/62157, sowie in Research Disclosure 79/17901, 79/17905, 79/18813, 83/22827 und 84/24014.

Die photographischen Schichten können auch andere weitere Derivate von Hydrochinonethern enthalten. Diese Verbindungen wirken auch als Lichtschutzmittel und sind geeignet zur Stabilisierung von Magenta-Farbstoffen. Solche Verbindungen und deren Kombination mit anderen Additiven sind z.B. in folgenden Veröffentlichungen näher beschrieben:
US-A 3,285,937, 3,432,300, 3,519,429, 3,476,772, 3,591,381, 3,573,052, 3,574,627, 3,573,050, 3,698,909, 3,764,337, 3,930,866,4,113,488, 4,015,990,4,113,495, 4,120,723, 4,155,765, 4,159,910, 4,178,184, 4,138,259, 4,174,220, 4,148,656, 4,207,111, 4,254,216, 4,134,011, 4,273,864, 4,264,720, 4,279,990, 4,332,886, 4,436,165, 4,360,589, 4,416,978, 4,385,111, 4,459,015, 4,559,297, 4,631,252, 4,616,082; GB-A 1,347,556, 1,366,441, 1,547,392, 1,557,237, 2,135,788; DE-A 3,214,567, 40 08 785, 40 12 305; DD-214,469, EP-A 161,577, 167,762, 164,130, 176,845; JP-A 76/123,642, 77/35633, 77/147,433, 78/126, 78/10430, 78/53321, 79/24019, 79/25823, 79/48537, 79/44521, 79/56833, 79/70036, 79/70830, 79/73032, 79/95233, 79/145,530, 80/21004, 80/50244, 80/52057, 80/70840, 80/139,383, 81/30125, 81/151,936, 82/34552, 82/68833, 82/204,306 82/204,037, 83/134,634, 83/207,039, 84/60434, 84/101,650, 84/87450, 84/149,348, 84/182,785, 86/72040, 87/11455, 87/62157, 87/63149, 86/2151, 86/6652, 86/48855, 89/309,058 sowie in Research Disclosure 78/17051.

Bevorzugte Co-Stabilisatoren sind solche der nachfolgenden Formeln P, SA, SB, HQ, RE, KA und KB.

Verbindungen der Formel P 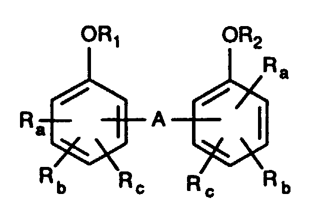 worin
R₁ und R₂ unabhängig voneinander Wasserstoff, Acyl oder Alkyl;
Rₐ, R_{b} und R_{c} unabhängig voneinander H, Alkyl, Cycloalkyl, Aryl, Halogen, Alkoxy, Aroxy, Acyloxy, Alkylthio, Arylthio, Acyl, Sulphonyl, Sulphamoyl, Acylamino, Sulphonylamino oder Nitro;
A eine Bindung, S 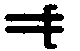 O] ₘ, Alkylen oder -NR_{d}-;
R_{d} Alkyl oder Acyl; und
m 0, 1 oder 2 bedeutet.

Beispiele für Verbindungen der Formel P:

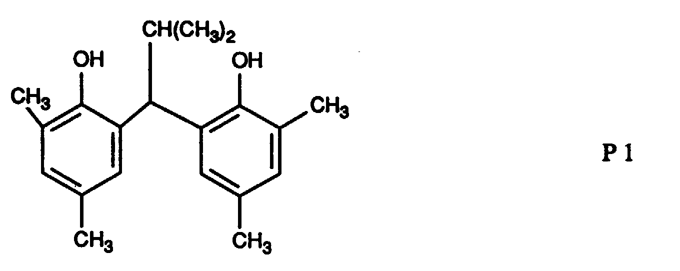

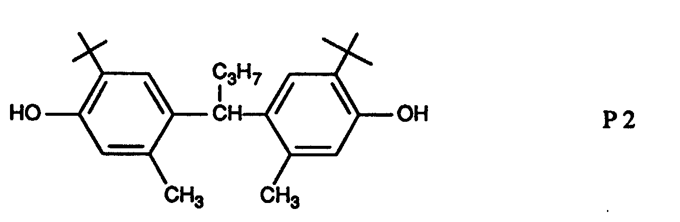

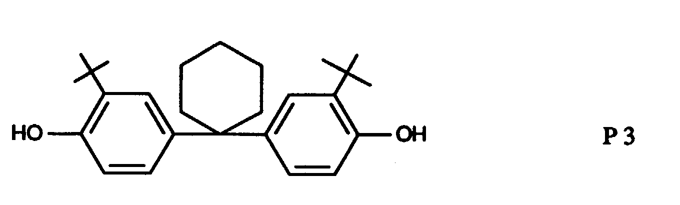

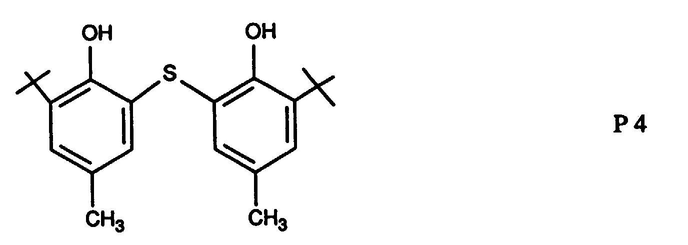

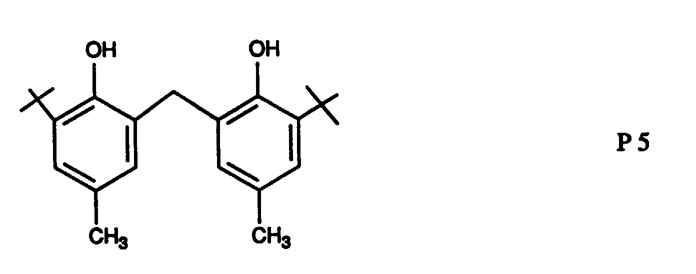

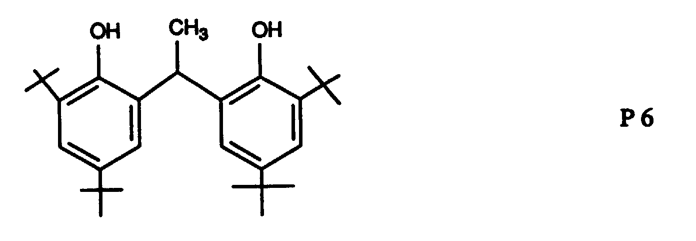

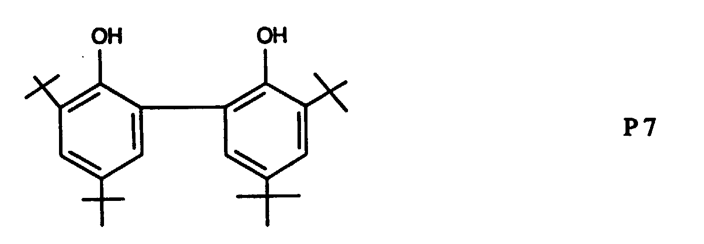

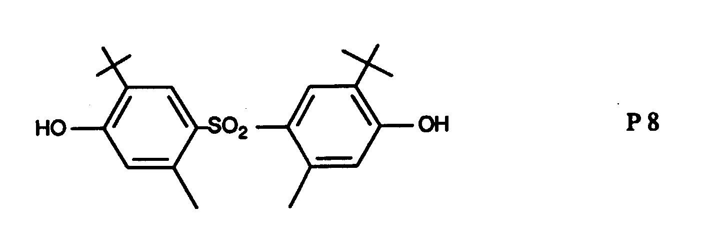

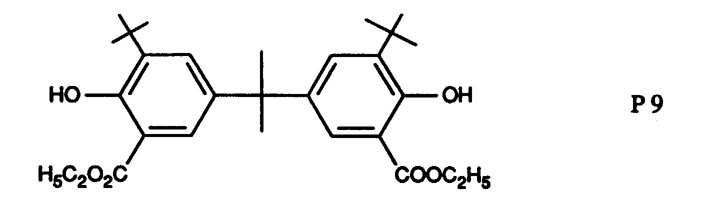

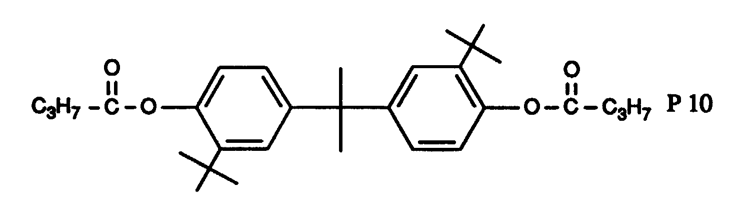

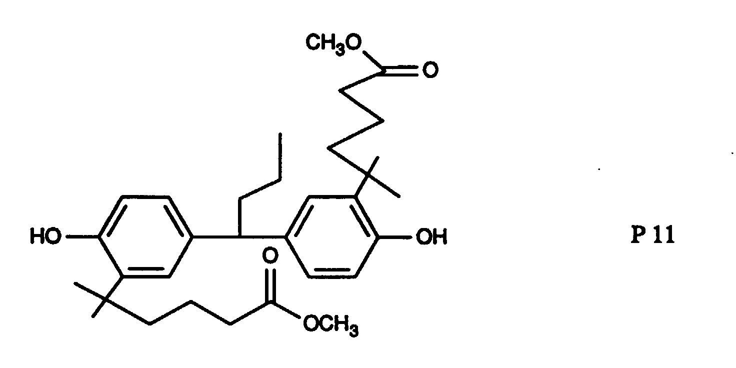

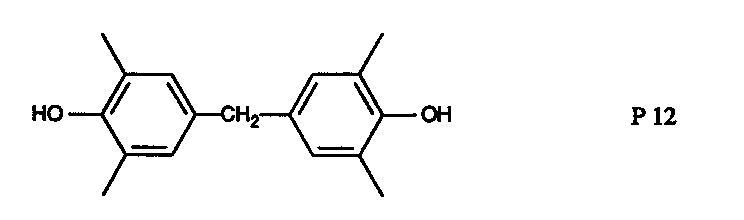

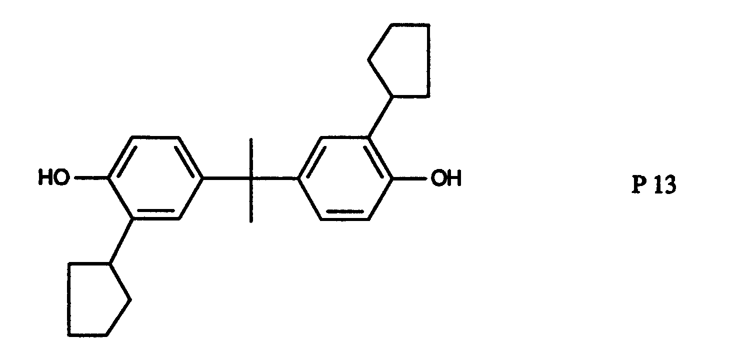

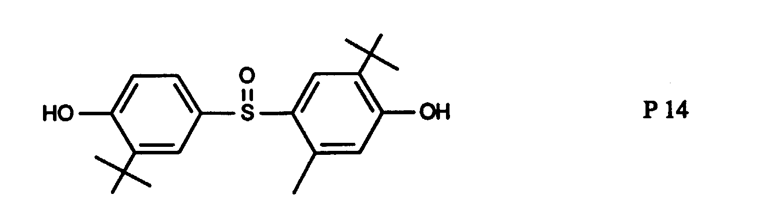

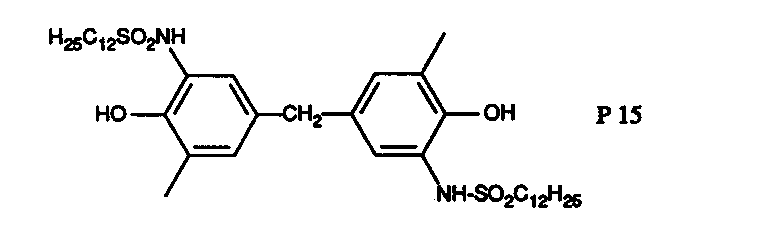

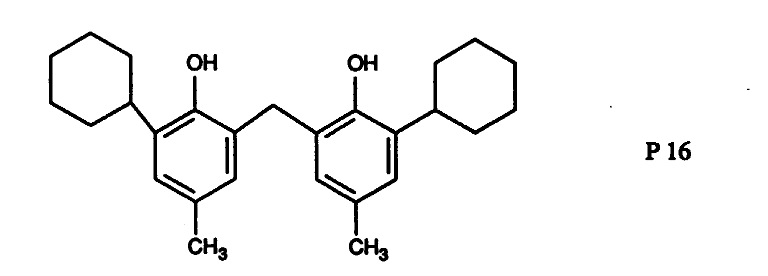

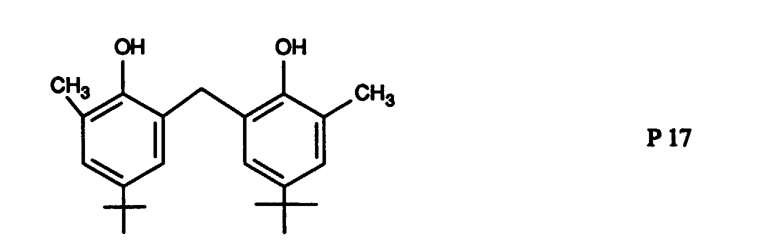

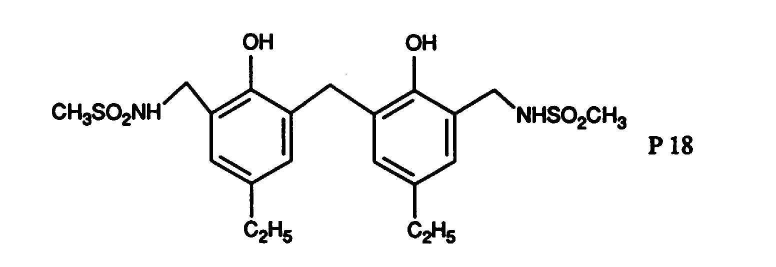

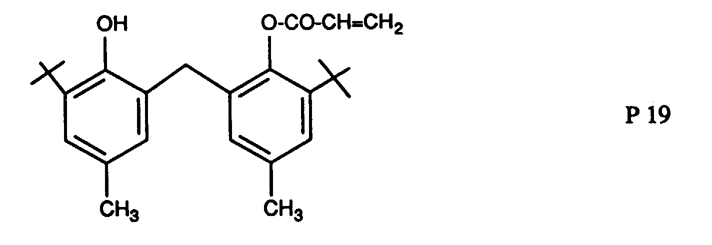

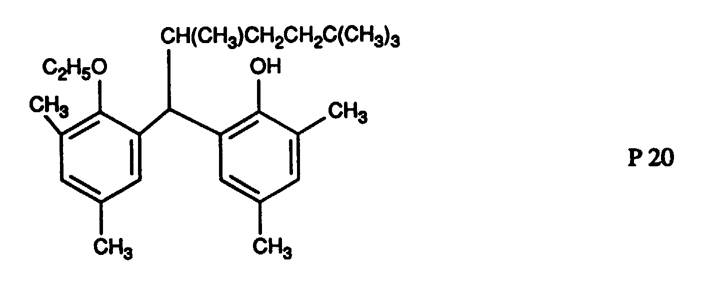

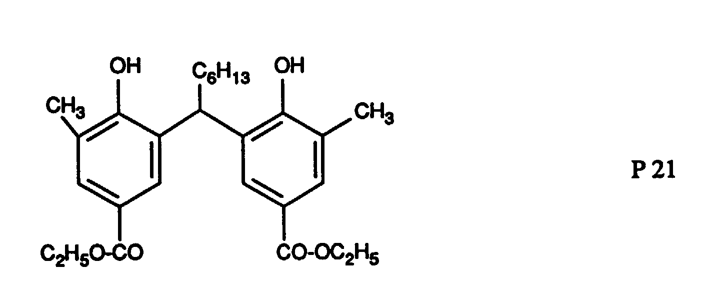

Verbindungen der Formel SA 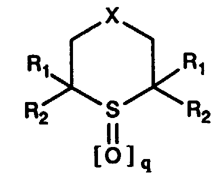 worin
R₁ Wasserstoff;
R₂ Phenyl oder
R₁ und R₂ Methyl;
q 0, 1 oder 2; und
X einen zweiwertigen Rest bedeutet, der den Ring der Formel SA zu einem Tetrahydrothiopyranring ergänzt

Beispiele für Verbindungen der Formel SA siehe US 4993271 und

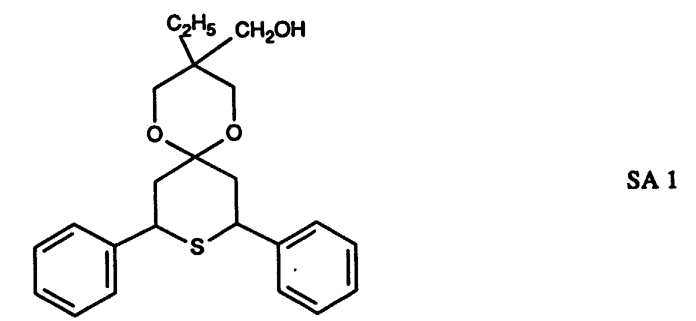

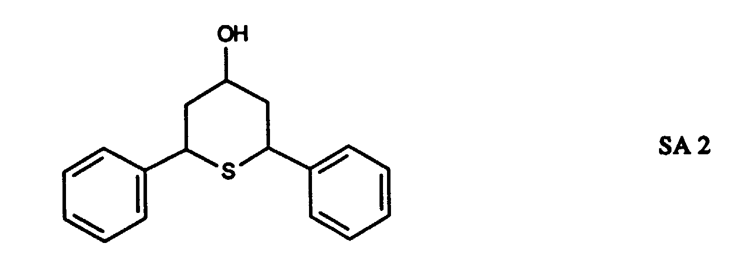

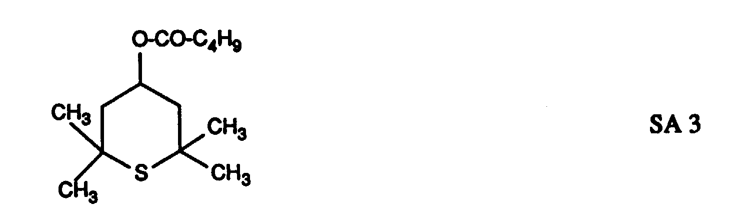

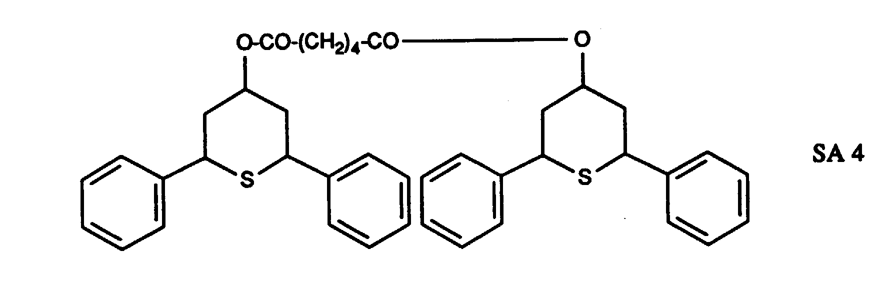

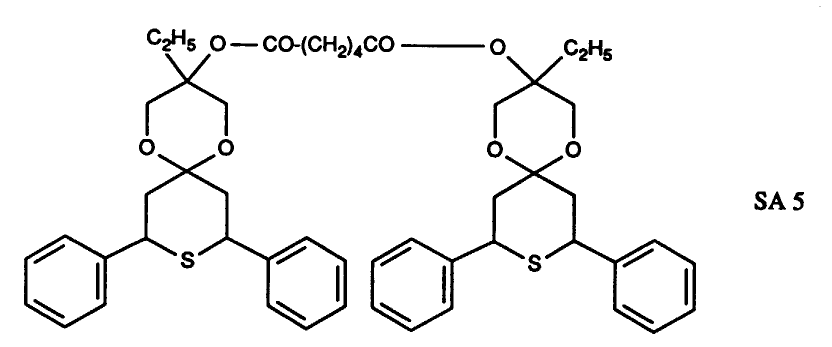

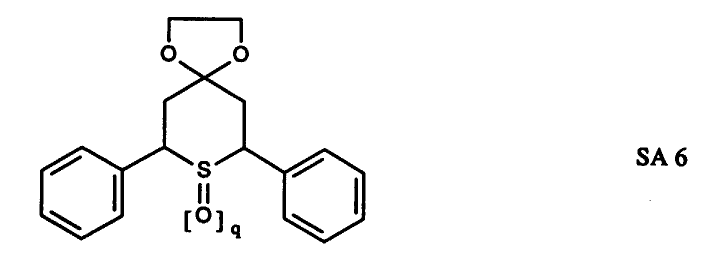

Verbindungen der Formel SB

R₃-S-(CₚH₂ₚ)-Z-R₄

worin
R₃ Alkyl, Aryl oder eine Gruppe (CₚH₂ₚ)-Z-R₄;
p 1-12;
Z -CO-O- oder -O-CO-;
R₄ eine ein-, zwei-, drei- oder vier-wertige Gruppe bedeutet.

Beispiele für Verbindungen der Formel SB

C₁₂H₂₅-S-CH₂CH₂CO-O-C₄H₉ SB 1

S(CH₂CH₂CO-OC₄H₉)₂ SB 2

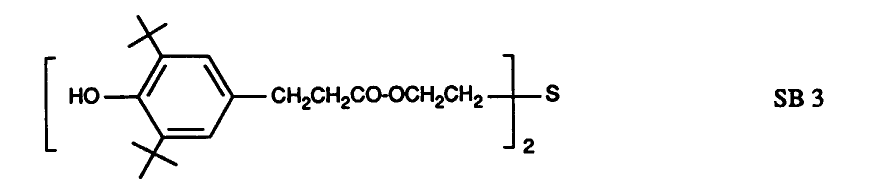

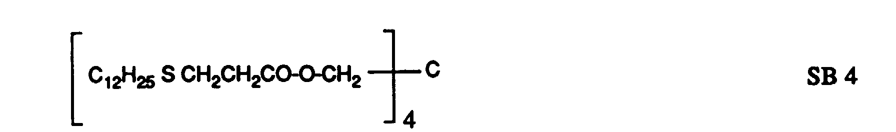

Verbindungen der Formel HQ 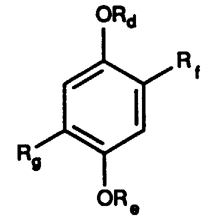 worin
Rₑ und R_{d} unabhängig voneinander Alkyl oder Cycloalkyl bedeutet; und
R_{f} und R_{g} unabhängig voneinander die Bedeutungen wie Rₐ, R_{b}, R_{c} haben.

Beispiele für Verbindungen der Formel HQ

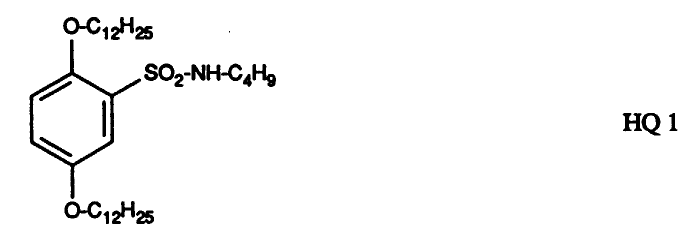

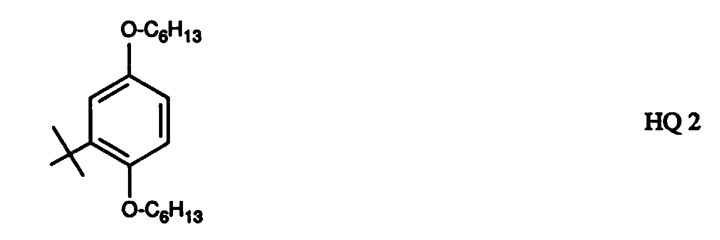

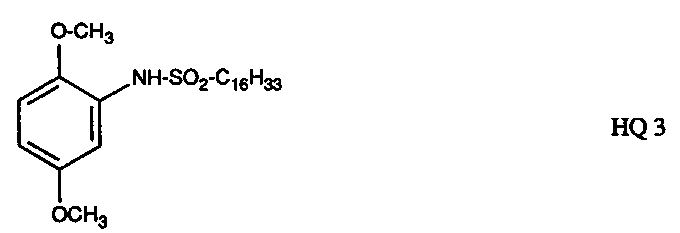

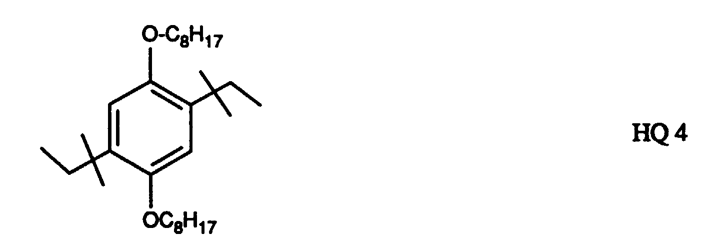

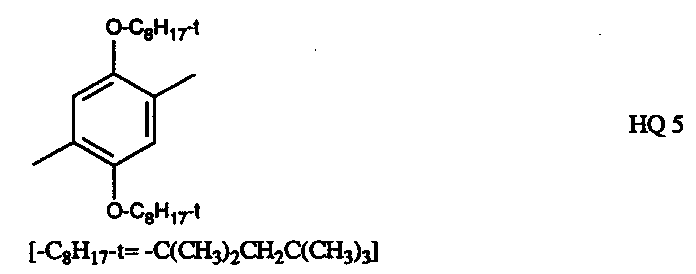

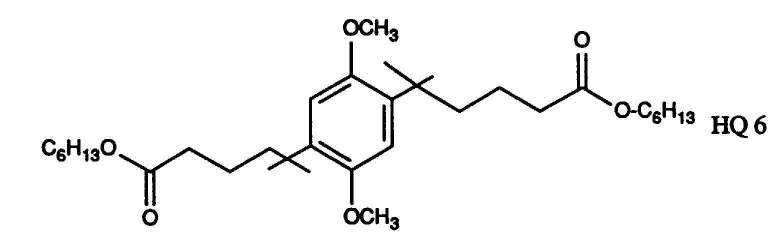

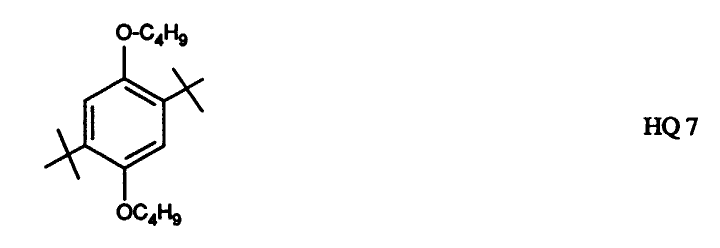

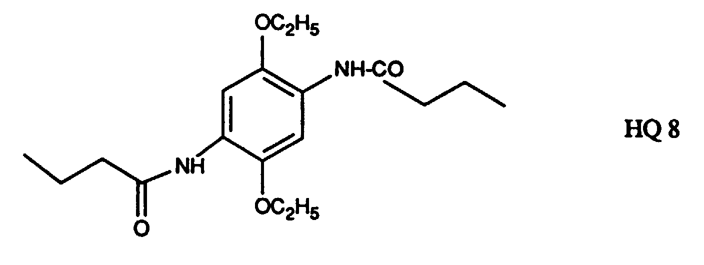

Verbindungen der Formel RE 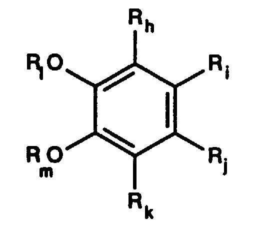 worin
Rₗ und Rₘ unabhängig voneinander H, Acyl oder Alkyl bedeutet; oder Rₗ und Rₘ zusammen an einen P-O-Aryl-Rest gebunden sind; und
Rₕ, Rᵢ, Rⱼ und Rₖ unabhängig voneinander die Bedeutung wie Rₐ, R_{b}, R_{c} haben, mit der Massgabe, dass mindestens einer der Reste Rᵢ, Rⱼ nicht Alkyl bedeutet.

Beispiele für Verbindungen der Formel RE

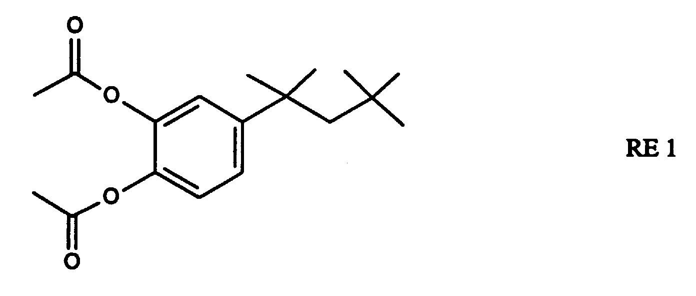

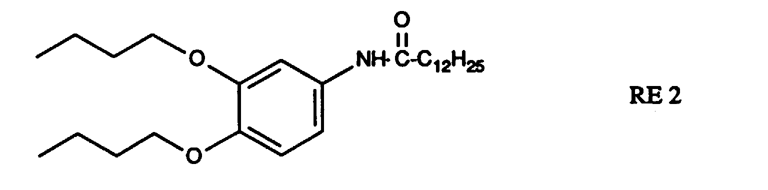

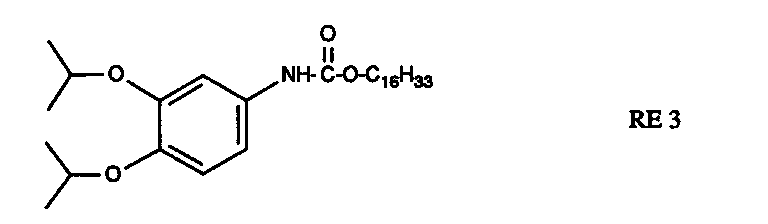

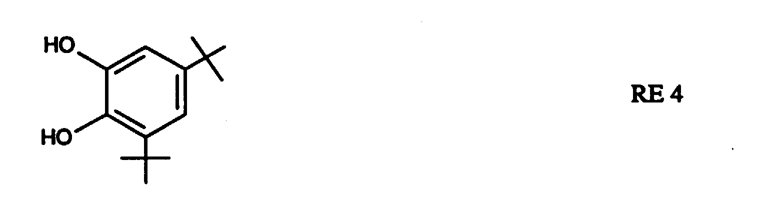

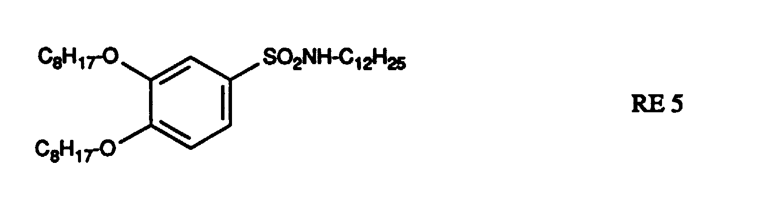

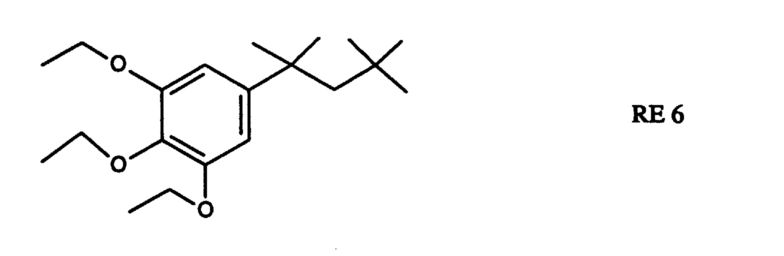

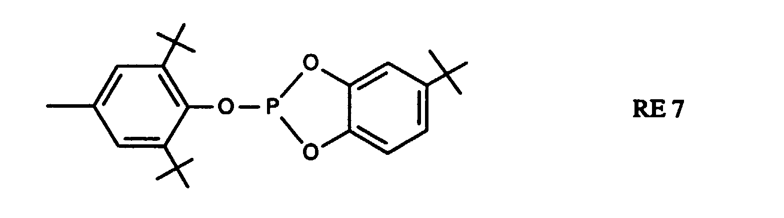

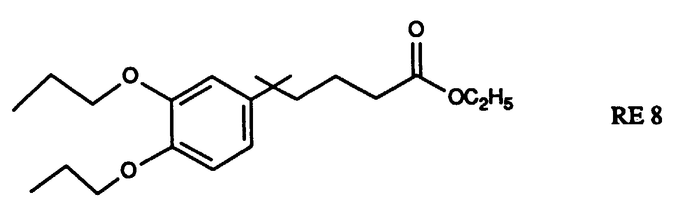

Verbindungen der Formel KA 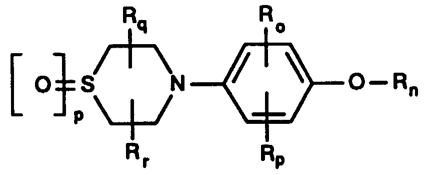 worin
Rₙ Alkyl, Cycloalkyl oder Aryl;
p 0, 1 oder 2 bedeuten; und
Rₒ, Rₚ, R_{q} und Rᵣ unabhängig voneinander die gleiche Bedeutung wie Rₐ, R_{b} und R_{c} haben.

Beispiele für Verbindungen der Formel KA

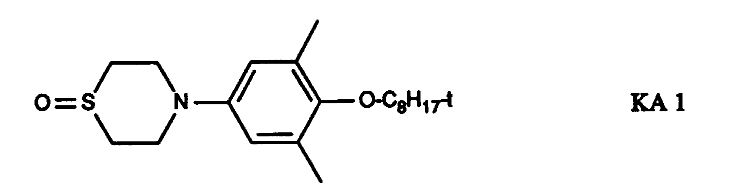

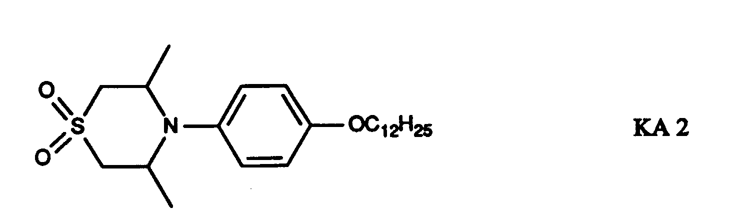

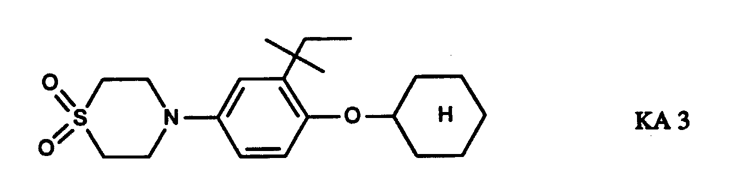

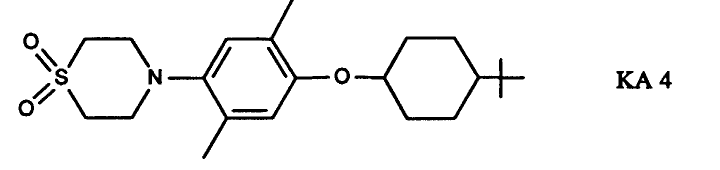

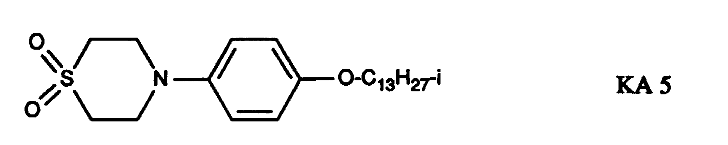

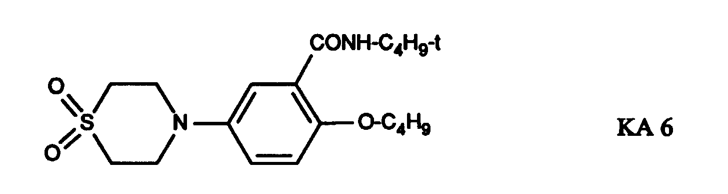

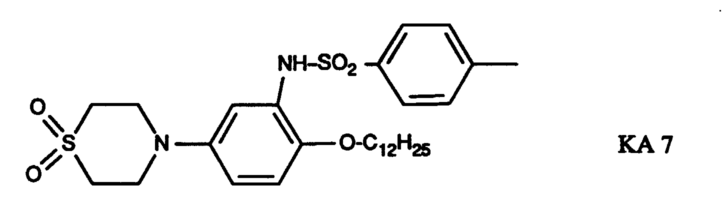

Verbindungen der Formel KB 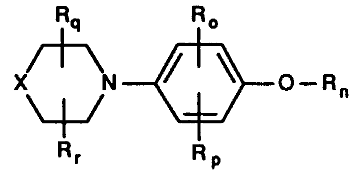 worin
X eine Bindung, 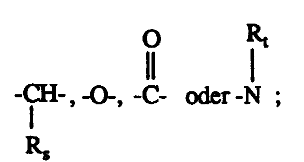
Rₜ Alkyl, Aryl, Acyl oder Sulphonyl bedeutet; und
Rₛ die Bedeutung wie Rₐ, R_{b} oder R_{c} hat.

Beispiele für Verbindungen der Formel KB

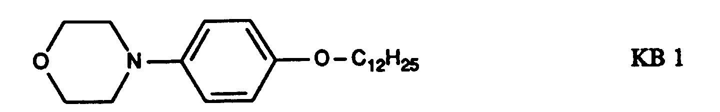

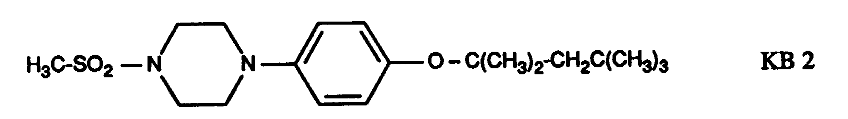

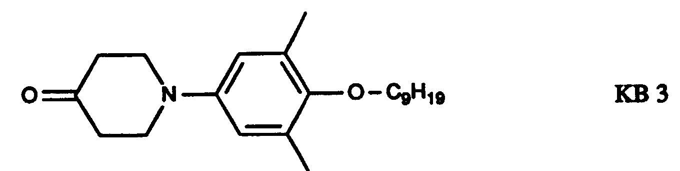

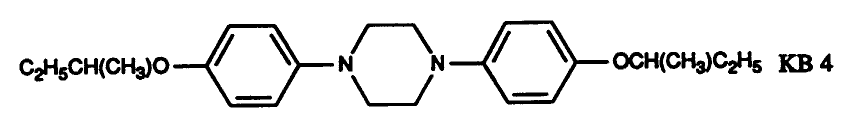

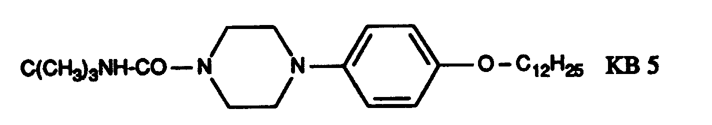

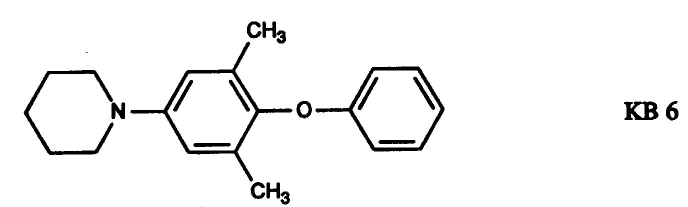

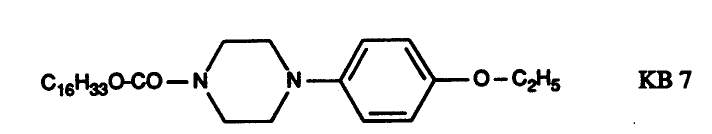

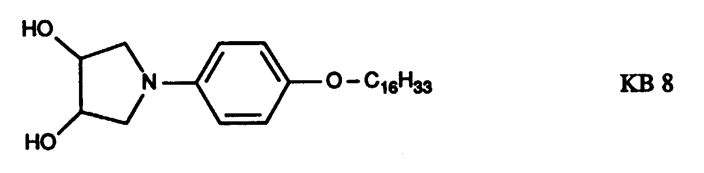

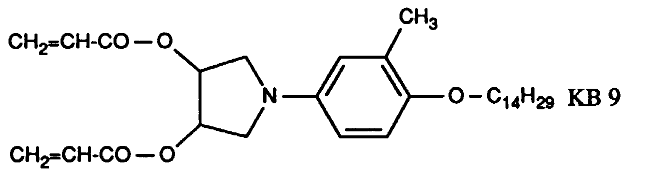

Als Silberhalogenidemulsionen können übliche Silberchlorid, -bromid oder -jodid-emulsionen verwendet werden oder Mischungen hiervon wie Silberchlorobromid- und Silberchloriodidemulsionen, worin die Silberhalogenide alle bekannten Kistallformen aufweisen können. Der Verwendung von Silberchloridemulsionen kommt im erfindungsgemässen Material besondere Bedeutung zu. Die Herstellung solcher Emulsionen sowie deren Sensibilisierung sind in RESEARCH DISCLOSURE, November 1989, Nr. 307,105 beschrieben. Diese Publikation erwähnt ferner eine Reihe von Bindemitteln für die genannten Emulsionen, welche auch in den erfindungsgemässen Materialien Anwendung finden können. Dasselbe gilt für die in der Publikation genannten Träger.

Die Silberhalogenidemulsion, welche zur Durchführung dieser Erfindung verwendbar ist, kann für alle gewünschten Wellenlängen sensibilisiert werden mit Hilfe von Sensibilisierungspigmenten. Es können hierfür Cyanin-Pigmente, Merocyanin-Pigmente, holopolare Pigmente, Hemicyanin-Pigmente, Styryl-Pigmente oder Hemioxanol-Pigmente verwendet werden.

Die photographischen Schichten können ferner übliche Weichmacher, wie Glycerin, enthalten. Die Emulsionen können auch mit den für Gelatine üblichen Härtern gehärtet werden. Schliesslich können die Emulsionen auch übliche Beschichtungshilfsmittel enthalten

Ein weiterer Gegenstand der vorliegenden Erfindung sind somit farbphotographische Aufzeichnungsmaterialien gemäss Anspruch 1, dadurch gekennzeichnet, dass diese weitere organische Stabilisatoren, UV-Absorber, optische Aufheller, Lichtschutzmittel, Farbschleier-Inhibitoren und/oder Weichmacher enthalten.

Gegenstand vorliegender Erfindung sind auch Verbindungen der Formel 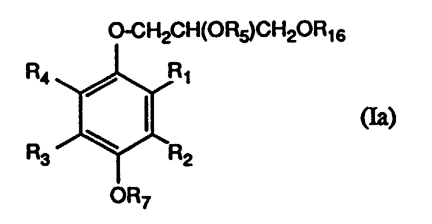 wobei
R₁ und R₃ unabhängig voneinander Wasserstoff, C₁-C₁₂-Alkyl, C₅-C₇-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 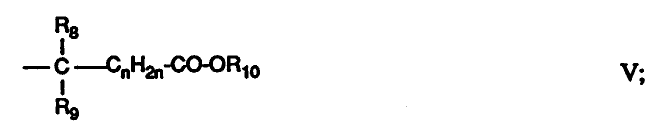
   worin R₈ und R₉ unabhängig voneinander C₁-C₈-Alkyl;
   n = 1-10
   R₁₀ Wasserstoff, C₁-C₂₄-Alkyl, das unsubstituiert oder durch ein oder mehrere -O- Atome unterbrochen und gegebenenfalls durch ein -OH substituiert ist, C₂-C₁₈-Alkenyl, C₅-C₁₂-Cycloalkyl das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
   worin R₁₁ C₁-C₁₈-Alkyl oder Phenyl;
   R₁₂ C₁-C₄-Alkyl;
   R₁₃, R₁₄ und R₁₅ unabhängig voneinander C₁-C₆-Alkyl oder Phenyl ist;
R₇ eine Gruppe der Formel VI 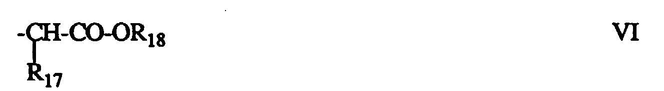 oder eine Gruppe der Formel VIIa

   -CH₂CH(OR₅)CH₂OR₁₆ VIIa;
worin R₅ und R₁₆ wie für Formel (Ia) angegeben;
R₁₇ Wasserstoff oder C₁-C₁₈-Alkyl; und
R₁₈ C₁-C₁₂-Alkyl, das unsubstituiert oder durch ein oder mehrere O-Atome unterbrochen ist, C₂-C₁₈-Alkenyl, Benzyl oder Phenyl, das gegebenenfalls mit 1-3 C₁-C₄-Alkyl substituiert ist; und
die Reste R₁₆ unabhängig voneinander Wasserstoff, C₁-C₁₈-Alkyl, C₂-C₁₈-Alkenyl, Phenyl-C₁-C₄-alkyl, durch ein oder mehrere O-Atome unterbrochenes C₃-C₂₄-Alkyl oder C₂-C₁₄-Hydroxyalkyl, Phenyl, Tolyl, C₅-C₆-Cycloalkyl, das gegebenenfalls mit 1 bis 3 C₁-C₈-Alkyl substituiert ist, oder -CO-R₁₁;
worin R₁₁ C₁-C₁₃-Alkyl oder Phenyl ist; bedeutet.

Bevorzugte Verbindungen sind solche der folgenden Formel 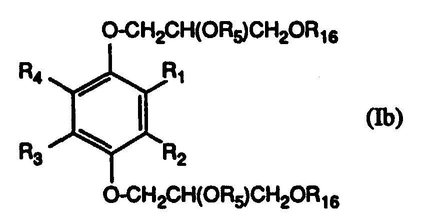 wobei
R₁ und R₃ unabhängig voneinander Wasserstoff, C₁-C₁₂-Alkyl, C₅-C₆-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 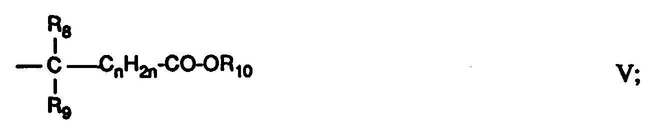
   worin R₈ und R₉ unabhängig voneinander C₁-C₈-Alkyl;
   n = 1-10
   R₁₀ Wasserstoff, C₁-C₂₄-Alkyl, das unsubstituiert oder durch ein oder mehrere -O- Atome unterbrochen und gegebenenfalls durch ein -OH substituiert ist, C₂-C₁₈-Alkenyl, C₅-C₁₂-Cycloalkyl, das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
   worin R₁₁ C₁-C₁₂-Alkyl;
   R₁₂ C₁-C₄-Alkyl;
   R₁₃, R₁₄ und R₁₅ unabhängig voneinander C₁-C₆-Alkyl oder Phenyl ist; die Reste R₁₆ unabhängig voneinander C₁-C₁₈-Alkyl, C₂-C₁₀-Alkenyl, Benzyl, durch ein oder mehrere O-Atome unterbrochenes C₃-C₁₈-Alkyl, Cyclohexyl oder -CO-R₁₁;
   worin R₁₁ C₁-C₁₂-Alkyl ist;
bedeutet.

Besonders bevorzugt sind Verbindungen der folgenden Formel 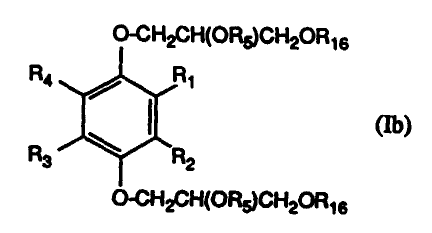 wobei
R₁ und R₃ unabhängig voneinander Wasserstoff, C₁-C₁₂-Alkyl, C₅-C₆-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 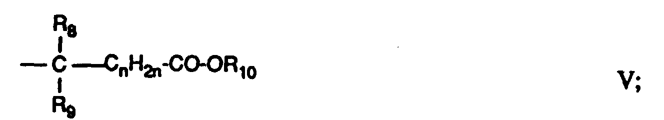
   worin R₈ und R₉ unabhängig voneinander C₁-C₈-Alkyl;
   n = 1-10
   R₁₀ Wasserstoff, C₁-C₂₄-Alkyl, das unsubstituiert oder durch ein oder mehrere -O- Atome unterbrochen und gegebenenfalls durch ein -OH substituiert ist, C₂-C₁₈-Alkenyl, C₅-C₁₂-Cycloalkyl, das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
   worin R₁₁ C₁-C₄-Alkyl;
   R₁₂ C₁-C₄-Alkyl;
   R₁₃, R₁₄ und R₁₅ unabhängig voneinander C₁-C₆-Alkyl oder Phenyl ist; die Reste R₁₆ unabhängig voneinander C₁-C₁₈-Alkyl, Allyl, Benzyl, durch ein oder mehrere O-Atome unterbrochenes C₃-C₁₂-Alkyl, Cyclohexyl oder -COR₁₁;
   worin R₁₁ C₁-C₄-Alkyl ist;
bedeutet.

Ganz besonders bevorzugt sind Verbindungen der Formel 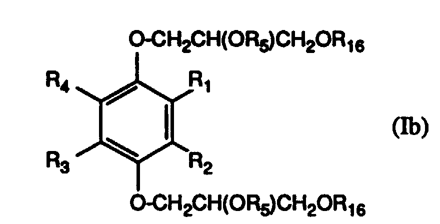
wobei R₁, R₃ und R₄ unabhängig voneinander Wasserstoff oder C₁-C₈-Alkyl;
R₂ Wasserstoff;
R₅ Wasserstoff oder -CO-CH₃; und
die Reste R₁₆ unabhängig voneinander C₁-C₁₂-Alkyl, Allyl oder durch 1-3 O-Atome unterbrochenes C₃-C₇-Alkyl;
bedeutet.

Ein weiterer Gegenstand der vorliegenden Erfindung sind Verbindungen der Formeln III 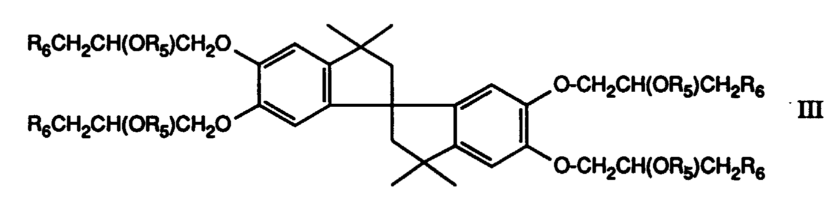 und IV 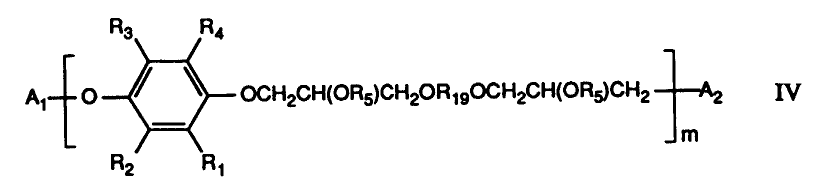 wobei
R₁, R₂, R₃, R₄, R₅, R₆, R₁₉ , A₁, A₂ und m die Bedeutungen wie in Anspruch 1 haben.

Weitere bevorzugte Verbindungen sind solche, wie sie bei der Beschreibung des fotografischen Materials erwähnt sind.

Die erfindungsgemässen Verbindungen können nach an sich bekannten Methoden hergestellt werden, zum Beispiel dadurch, dass man eine Verbindung einer der folgenden Formeln 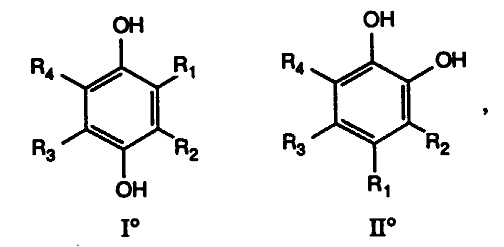 mit einem Glycidylether der Formel 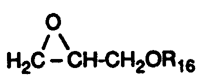 umsetzt, wobei die Reste R₁, R₂, R₃, R₄ und R₁₆ die in Anspruch 1 genannte Bedeutung haben.

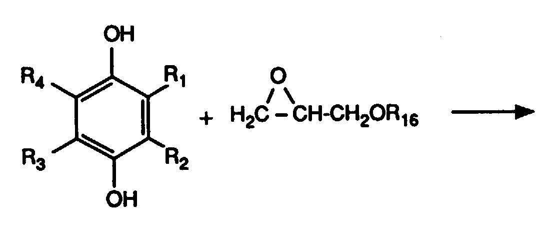

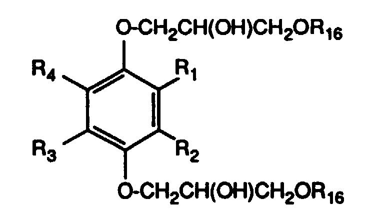

Erfindungsgemässe Verbindungen, in denen R₅ nicht Wasserstoff ist, sind durch weiteres Umsetzen mit einem Acylierungsmittel oder Silylierungsmittel herstellbar, z.B.

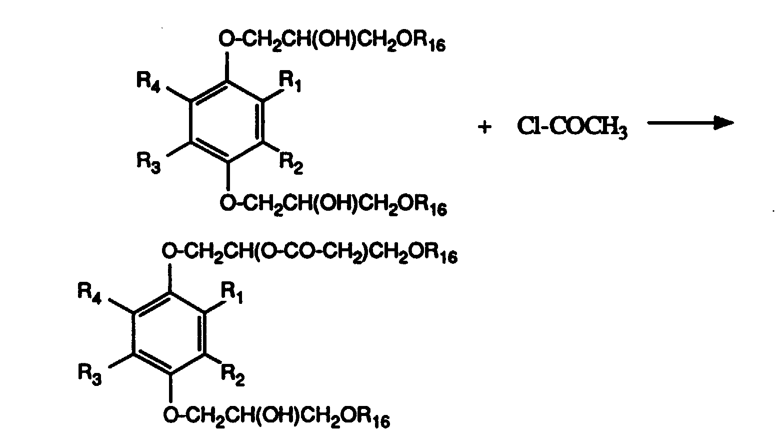

Die polymeren Verbindungen der Formel (IV) lassen sich duch Umsetzung einer Verbindung der Formel (I°) mit einem Bisglycidylether der Formel 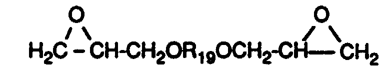 herstellen.

Die nachfolgenden Beispiele erläutern die Erfindung weiter.

### Beispiel 1:

Herstellung von 1,4-Di-(3'-butoxy-2'-hydroxypropoxy)-2,5-dimethylbenzol 20 g 2,5-Dimethylhydrochinon, 42,2 g Butylglycidylether und 5,3 g Ethyltriphenylphosphoniumbromid werden während 15 Stunden in 80 ml siedendem Xylol gerührt. Anschliessend lässt man auf Raumtemperatur abkühlen, wäscht die organische Phase mit Wasser, trocknet und engt das Lösungsmittel ein. Das Rohprodukt wird über Kieselgel chromatografiert und man erhält 27,5 g 1,4-Di-(3'-butoxy-2'-hydroxypropoxy)-2,5-dimethylbenzol als gelbliches Öl (Smp. <30°C).

| | |
|---|---|
| Elementaranalyse:C₂₂H₃₈O₆ | Berechnet: C 66,30; H 9,61 % |
| | Gefunden: C 66,00; H 9,70 % |

### Beispiel 2:

Verfährt man wie in Beispiel 1, verwendet jedoch anstelle des 2,5-Dimethylhydrochinons die entsprechende Menge 2,5-Di-tert.-butylhydrochinon, so erhält man 28,7 g 1,4-Di-(3'-butoxy-2'-hydroxypropoxy)-2,5-di-tert.-butylbenzol als weissen Feststoff (Smp. 59°C).

| | |
|---|---|
| Elementaranalyse:C₂₈H₅₀O₆ | Berechnet: C 69,67; H 10,44 % |
| | Gefunden: C 69,31;H 10,41 % |

### Beispiele 1a und 2a:

Verfährt man analog der Beispiele 1 und 2, so erhält man die weiter oben erwähnten Verbindungen 1-48, welche einen Schmelzpunkt von <20°C aufweisen, mit Ausnahme der Verbindungen 1 (59-60°C), 12 (40-41°C), 13 (58-60°C), 26 (131°C), 30 (116-123°C) und 37 (31-32°C).

### Beispiel 3:

Auf ein mit Polyethylen beschichtetes Trägermaterial wird eine Gelatineschicht aufgetragen, die Silberbromid, Magentakuppler (M-6) und einen Stabilisator enthält.

Die Gelatineschicht enthält folgende Komponenten (je m² Trägermaterial):

**Tabelle 1**

| Komponente | AgBr-Schicht |
|---|---|
| Gelatine | 5.15 g |
| Härtungsmittel | 300 mg |
| Netzmittel | 85 mg |
| Silberbromid | 260 mg |
| Magentakuppler | 325 mg |
| Trikresylphosphat | 162 mg |
| Stabilisator | 114 mg |

Als Härter wird 2,4-Dichlor-6-hydroxytriazin verwendet, als Netzmittel das Natriumsalz der Diisobutylnaphthalinsulfonsäure.

Auf die so erhaltenen Proben wird jeweils ein Stufenkeil mit einem Dichteunterschied von 0,15 logE pro Stufe aufbelichtet und anschliessend gemäss den Vorschriften des Herstellers im Verarbeitungsprozess EP2 der Firma Kodak für Negativ-Farbpapiere verarbeitet.

Nach Belichtung und Verarbeitung wird die Remissionsdichte im Grünbereich für die Magentastufe mit einer Dichte zwischen 0,9 und 1,1 des Keils gemessen. Dann wird der Keil hinter einem UV-Absorber-Filter in einem Atlas-Belichtungsgerät mit 15 kJ/cm² belichtet und erneut die Remissionsdichte gemessen. Der Magentafarbstoffdichteverlust (-ΔD) in % ist in Tabelle 2 angegeben.

**Tabelle 2**

| Probe Nr. | Stabilisator | -ΔD |
|---|---|---|
| 1 | kein | 87 |
| 2 | 3 | 53 |
| 3 | 14 | 54 |

### Beispiel 4:

Es wird wie in Beispiel 3 vorgegangen, jedoch der RA4 Prozess der Firma Kodak benützt. Der Magentafarbstoffdichteverlust (-ΔD) in % ist in Tabelle 3 angegeben.

**Tabelle 3**

| Probe Nr. | Stabilisator | -ΔD |
|---|---|---|
| 4 | kein | 81 |
| 5 | 14 | 44 |

In den Beispielen 3 und 4 sieht man, dass die Erfindungsverbindungen gute Lichtschutzmittel sind.

### Beispiel 5:

Es wird wie in Beispiel 3 vorgegangen, jedoch wird der Stabilisator als Öl benützt. Die Ölmengen sind in Tabelle 4 angegeben. Ein zweiter Stufenkeil wird im Blaubereich zur Bestimmung der Vergilbung gemessen. Dann wird der Keil in einem Klimaschrank 28 Tage lang bei 75°C und 60 % Relativfeuchtigkeit gelagert, erneut die Remissionsdichte (im Blau) gemessen und die Gelbfarbstoffdichtezunahme (-ΔD_{B}) berechnet.

Der Magentafarbstoffdichteverlust (-ΔD) in % und die Vergilbung (-ΔD_{B}) sind in Tabelle 4 angegeben. Der Farbstoffdichteverlust (-ΔD) ist auch für eine Magentafabstoffdichte von 2 in Tabelle 4 angegeben.

**Tabelle 4:**

| Probe Nr. | Öl | Menge (mg/m²) | -ΔD (%) | | -ΔD_{B} |
|---|---|---|---|---|---|
| | | | D = 1 | D = 2 | |
| 6 | Trikresylphosphat | 276 | 84 | 81 | 0,12 |
| 7 | Trikresylphosphat | 325 | 82 | 78 | 0,11 |
| 8 | Trikresylphosphat | 440 | 79 | 71 | 0,11 |
| 9 | 14 | 276 | 60 | 57 | 0,11 |
| 10 | 14 | 325 | 45 | 33 | 0,12 |
| 11 | 14 | 440 | 40 | 22 | 0,12 |

Man sieht, dass die Farbstoffdichteverluste bei D = 1 sowie D = 2 viel niedriger sind als bei dem üblichen Öl Trikresylphosphat. Die Klimavergilbung des Stabilisator(öl)s der Erfindung ist im Rahmen der experimentellen Genauigkeit gleich der bei dem üblichen Öl (Trikresylphosphat).

### Beispiel 6:

Es wird wie in Beispiel 5 vorgegangen, jedoch werden 306 mg des Magentakuppler (M-5) benützt. Es wird auch die Atlasvergilbung gemessen; hierzu wird die Remissionsdichte im Blaubereich zur Bestimmung der Vergilbung gemessen. Dann wird der Keil in einem Atlas-Belichtungsgerät mit 30 kJ/cm² belichtet, erneut die Remissionsdichte (im Blau) gemessen und die Gelbfarbstoffdichtezunahme (-ΔD_{B}) berechnet. Der Magentafarbstoffdichteverlust (-ΔD) nach 30 kJ/cm² Bestrahlung und die Vergilbung (-ΔD_{B}) sind in Tabelle 5 angegeben.

**Tabelle 5:**

| Probe Nr. | Öl | Menge (mg/m²) | -ΔD | -ΔD_{B} |
|---|---|---|---|---|
| 12 | Trikresylphosphat | 612 | 42 | 0,02 |
| 13 | 14 | 612 | 8 | 0,00 |
| 14 | 14 | 918 | 7 | 0,00 |

Man sieht, dass auch hier die Erfindungsverbindungen als photographisches Öl mit Lichtschutzeigenschaften für Magentakuppler/farbstoff sehr geeignet sind.

### Beispiel 7:

Es wird wie in Beispiel 3 vorgegangen, jedoch werden die Erfindungsverbindungen als Öl (325 mg/m²) für weitere Lichtschutzmittel benützt. Das verwendete weitere Lichtschutzmittel entspricht der Formel 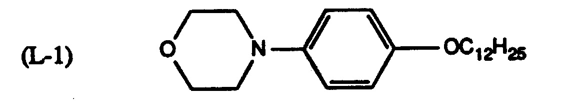 und wird in einer Menge von 114 mg/m² eingesetzt. Der Magentafarbstoffdichteverlust (-ΔD) und die Klimavergilbung (-ΔD_{B}) sind in Tabelle 6 angegeben.

**Tabelle 6:**

| Probe Nr. | Öl | Stabilisator | -ΔD | -ΔD_{B} |
|---|---|---|---|---|
| 15 | Trikresylphosphat | - | 82 | 0,11 |
| 16 | Trikresylphosphat | L-1 | 50 | 0,19 |
| 17 | 14 | - | 45 | 0,12 |
| 18 | 14 | L-1 | 38 | 0,18 |

Man sieht, dass die Erfindungsverbindungen nicht nur als Lichtschutzmittel wirken, sie sind auch geeignet als Kupplerlösungsmittel (Öle).

### Beispiel 8

Es wird wie in Beispiel 3 vorgegangen, jedoch wird der RA4 Prozess der Firma Kodak benützt, der Magentakuppler M-11 (204 mg) genommen, der Stabilisator in einer Menge von 102 mg eingesetzt und eine Belichtung von 30 kJ/cm²durchgeführt. Der Magentafarbstoffdichteverlust in % ist in Tabelle 7 angegeben.

**Tabelle 7**

| Probe Nr. | Stabilisator | -Δ D (%) |
|---|---|---|
| 19 | kein | 91 |
| 20 | 16 | 56 |
| 21 | 32/k | 59 |

### Beispiel 9

Es wird wie in Beispiel 8 vorgegangen, jedoch wird der Stabilisator als Oel (408 mg/m²) benützt und in den Proben 23-25 kein Trikresylphosphat eingesetzt. Der Magentafarbstoffdichteverlust in % ist in Tabelle 8 angegeben.

**Tabelle 8**

| Probe Nr. | Oel | -Δ D (%) |
|---|---|---|
| 22 | Trikresylphosphat | 92 |
| 23 | 16 | 36 |
| 24 | 14 | 34 |
| 25 | 32/j | 34 |

### Beispiel 10

Es wird wie in Beispiel 9 vorgegangen, jedoch beträgt die Oelmenge 204 mg/m² und in den Proben 27 und 28 wird kein Trikresylphosphat eingesetzt. Der Magentafarbstoffdichteverlust in % ist in Tabelle 9 angegeben.

**Tabelle 9**

| Probe Nr. | Oel | -Δ D (%) |
|---|---|---|
| 26 | Trikresylphosphat | 91 |
| 27 | 14 | 40 |
| 28 | 32/c | 32 |

### Beispiel 11

Es wird wie in Beispiel 10 vorgegangen, jedoch werden die Erfindungsverbindungen als Oel (204 mg/m²) für weitere Stabilisatoren benützt. Die verwendeten weiteren Stabilisatoren entsprechen der Formel 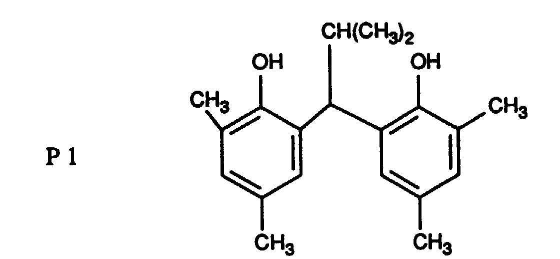 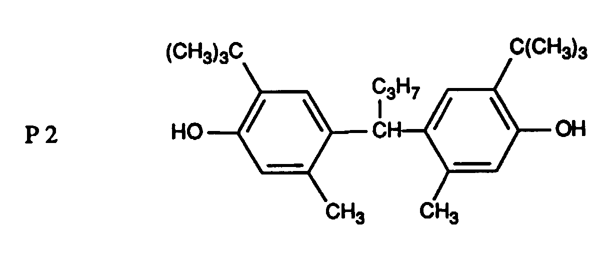 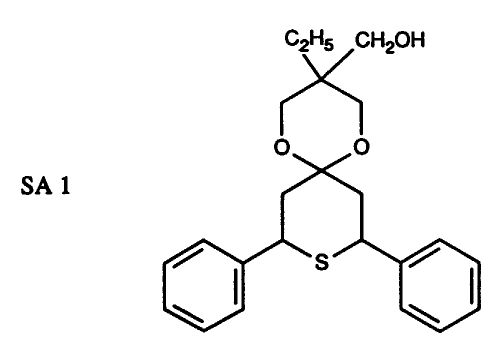 und werden in einer Menge von 102 mg/m² eingesetzt und in den Proben 33-39 wird kein Trikresylphosphat eingesetzt. Der Magentafarbstoffdichteverlust in % ist in Tabelle 10 angegeben.

**Tabelle 10**

| Probe Nr. | Oel | Stabilisator | -Δ D (%) |
|---|---|---|---|
| 29 | Trikresylphosphat | - | 91 |
| 30 | Trikresylphosphat | P1 | 55 |
| 31 | Trikresylphosphat | P2 | 70 |
| 32 | Trikresylphosphat | SA 1 | 88 |
| 33 | 14 | - | 40 |
| 34 | 14 | P1 | 24 |
| 35 | 14 | P2 | 30 |
| 36 | 14 | SA 1 | 28 |
| 37 | 32/c | - | 32 |
| 38 | 32/c | P1 | 22 |
| 39 | 32/c | SA 1 | 23 |

## Patentansprüche

1. Farbphotographisches Aufzeichnungsmaterial enthaltend einen Magentakuppler, dadurch gekennzeichnet, dass es als Stabilisator mindestens eine Verbindung der Formel 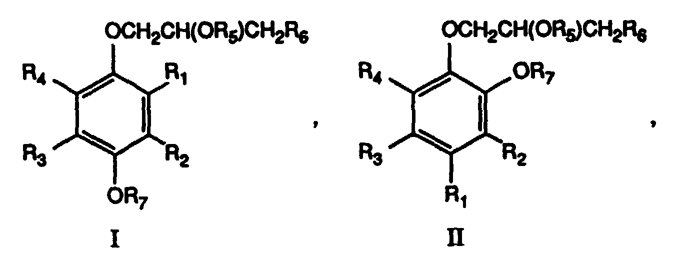 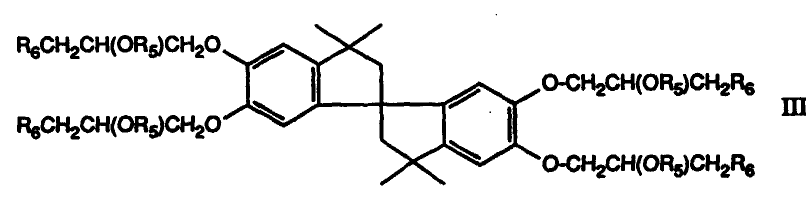 oder 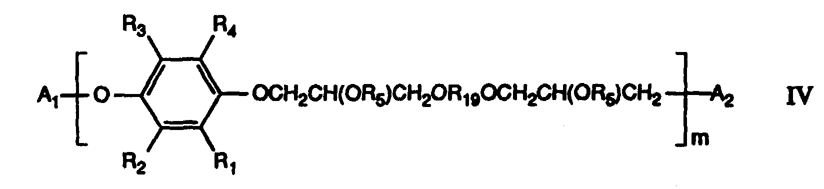 enthält, wobei
R₁ und R₃ unabhängig voneinander Wasserstoff, C₁-C₁₂-Alkyl, C₅-C₇-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 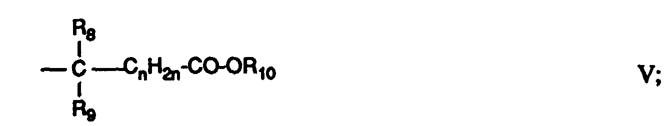
worin R₈ und R₉ unabhängig voneinander C₁-C₈-Alkyl;
n = 1-10
R₁₀ Wasserstoff, C₁-C₂₄-Alkyl, das unsubstituiert oder durch ein oder mehrere -O- Atome unterbrochen und gegebenenfalls durch ein -OH substituiert ist, C₂-C₁₈-Alkenyl, C₅-C₁₂-Cycloalkyl, das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
worin R₁₁ C₁-C₁₈-Alkyl, C₂-C₁₈-Alkenyl oder Phenyl;
R₁₂ C₁-C₄-Alkyl;
R₁₃, R₁₄ und R₁₅ unabhängig voneinander C₁-C₆-Alkyl oder Phenyl ist; die Reste R₆ unabhängig voneinander -OR₁₆ oder C₁-C₁₅-Alkyl;
worin R₁₆ Wasserstoff, C₁-C₁₈-Alkyl, C₂-C₁₈-Alkenyl, Phenyl-C₁-C₄-alkyl, durch ein oder mehrere O-Atome unterbrochenes C₃-C₂₄-Alkyl oder C₂-C₁₄-Hydroxyalkyl, Phenyl, Tolyl, C₅-C₆-Cycloalkyl, das gegebenenfalls mit 1 bis 3 C₁-C₈-Alkyl substituiert ist, oder -CO-R₁₁ ist;
worin R₁₁ C₁-C₁₈-Alkyl, C₂-C₁₈-Alkenyl oder Phenyl ist;
R₇ eine Gruppe der Formel VI 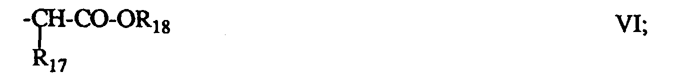 oder eine Gruppe der Formel VII
-CH₂CH(OR₅)CH₂R₆ VII;
worin R₅ und R₆ wie für Formel I angegeben;
R₁₇ Wasserstoff oder C₁-C₁₈-Alkyl; und
R₁₈ C₁-C₁₂-Alkyl, das unsubstituiert oder durch ein oder mehrere -O- Atome unterbrochen ist, C₂-C₁₈-Alkenyl, Benzyl oder Phenyl, das gegebenenfalls mit 1-3 C₁-C₄-Alkyl substituiert ist, ist;
R₁₉ C₂-C₁₀-Alkylen, Phenylen oder eine Gruppe -Phenylen-R₂₀-Phenylen-; worin R₂₀ -O-, -S-, -SO₂-, -CH₂- oder -C(CH₃)₂- ist;
m = 1-100;
A₁ Wasserstoff, -CH₂CH(OR₅)CH₂OR₁₉OCH(OR₅)CH₂OR₅ oder 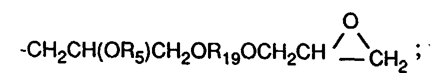 und
A₂ -OH oder 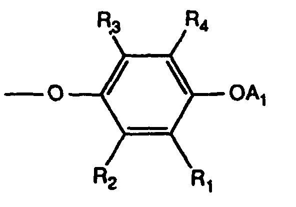
bedeutet;
oder in Formel I R₃ und R₇ zusammen mit den Atomen an die sie gebunden sind einen C₅-C₆ Ring, der gegebenenfalls mit 1 bis 3 C₁-C₈-Alkyl substituiert ist, bilden.

2. Farbphotographisches Aufzeichnungsmaterial gemäss Anspruch 1, dadurch gekennzeichnet, dass es als Stabilisator mindestens eine Verbindung der Formel 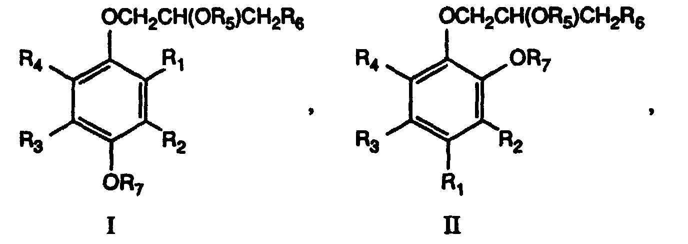 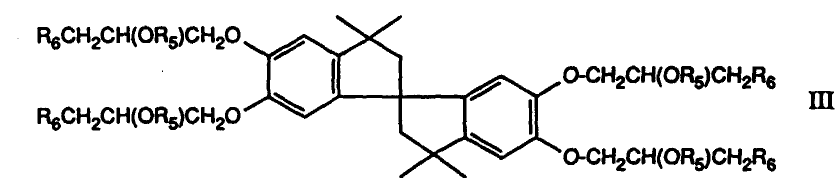 oder 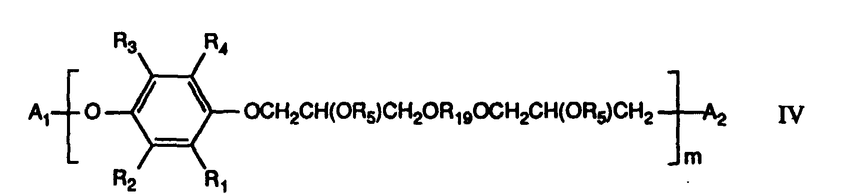
enthält, wobei R₁ und R₃ unabhängig voneinander Wasserstoff, C₁-C₈-Alkyl, Cyclohexyl, -C(CH₃)₂C₆H₅, C₁-C₄-Alkoxy oder eine Gruppe der Formel V 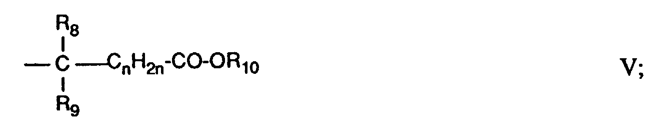
worin R₈ und R₉ Methyl;
n = 3 und
R₁₀ Wasserstoff, C₁-C₁₂-Alkyl, das unsubstituiert ist oder durch ein oder mehrere -O- Atome unterbrochen und gegebenenfalls durch ein -OH substituiert ist, C₂-C₁₈-Alkenyl oder Benzyl ist;
R₂ Wasserstoff;
R₄ Wasserstoff oder C₁-C₈-Alkyl;
R₅ Wasserstoff, -CO-R₁₁, -CO-OR₁₂ oder -Si(R₁₃)(R₁₄)(R₁₅);
worin R₁₁ C₁-C₁₈-Alkyl, C₂-C₁₈-Alkenyl oder Phenyl;
R₁₂ C₁-C₄-Alkyl;
R₁₃, R₁₄ und R₁₅ unabhängig voneinander C₁-C₆-Alkyl oder Phenyl ist;
die Reste R₆ unabhängig voneinander -OR₁₆;
worin R₁₆ Wasserstoff, C₁-C₁₈-Alkyl, C₂-C₁₈-Alkenyl, Phenyl-C₁-C₄-alkyl, durch ein oder mehrere O-Atome unterbrochenes C₃-C₂₄-Alkyl oder C₂-C₁₄-Hydroxyalkyl, Phenyl, Tolyl, C₅-C₆-Cycloalkyl, das gegebenenfalls mit 1 bis 3 C₁-C₈-Alkyl substituiert ist, oder -CO-R₁₁ ist;
worin R₁₁ C₁-C₁₈-Alkyl, C₂-C₁₈-Alkenyl oder Phenyl ist;
R₇ eine Gruppe der Formel VI 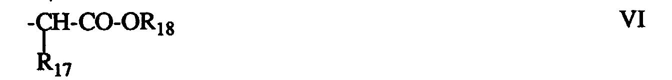
oder eine Gruppe der Formel VII
-CH₂CH(OR₅)CH₂R₆ VII;
worin R₅ und R₆ wie für Formel I angegeben;
R₁₇ Wasserstoff oder C₁-C₁₂-Alkyl und
R₁₈ C₁-C₈-Alkyl ist;
R₁₉ C₂-C₈-Alkylen, Phenylen oder eine Gruppe -Phenylen-R₂₀-Phenylen-;
worin R₂₀ -SO₂-, -CH₂- oder -C(CH₃)₂- ist;
m = 1-50;
A₁ Wasserstoff, -CH₂CH(OR₅)CH₂OR₁₉OCH(OR₅)CH₂OR₅ oder 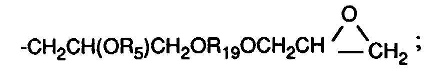 und
A₂ -OH oder 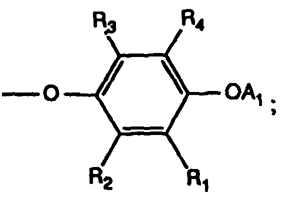
bedeuten.

3. Farbphotographisches Aufzeichnungsmaterial gemäss Anspruch 2, dadurch gekennzeichnet, dass es als Stabilisator mindestens eine Verbindung der Formel 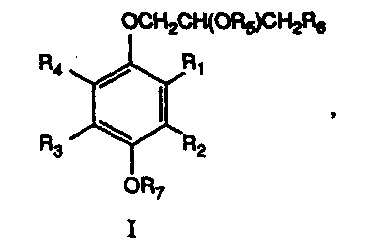 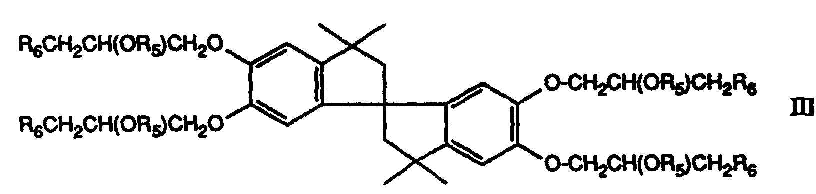 oder 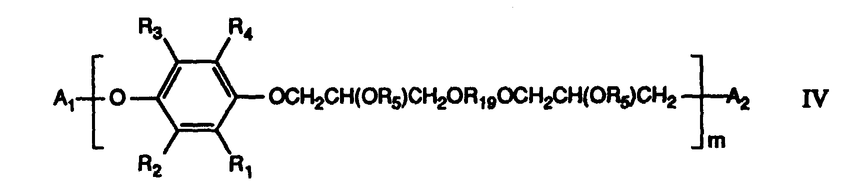
enthält, wobei R₁ und R₃ unabhängig voneinander Wasserstoff, C₁-C₈-Alkyl, C₁-C₄-Alkoxy oder eine Gruppe der Formel V 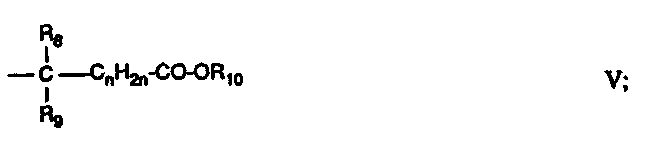
worin R₈ und R₉ Methyl;
n = 3; und
R₁₀ C₁-C₄-Alkyl ist;
R₂ Wasserstoff;
R₄ Wasserstoff oder C₁-C₈-Alkyl;
R₅ Wasserstoff, CO-R₁₁, COOR₁₂ oder Si(R₁₃)(R₁₄)(R₁₅);
worin R₁₁ C₁-C₄-Alkyl oder C₂-C₃-Alkenyl;
R₁₂ C₁-C₄-Alkyl;
R₁₃, R₁₄, R₁₅ unabhängig voneinander C₁-C₆-Alkyl ist;
die Reste R₆ unabhängig voneinander -OR₁₆;
worin R₁₆ C₁-C₁₈-Alkyl, Allyl, Benzyl, Phenyl, durch ein oder mehrere O-Atome unterbrochenes C₃-C₁₂-Alkyl, Cyclohexyl oder -CO-R₁₁ ist;
worin R₁₁ C₁-C₄-Alkyl oder C₂-C₃-Alkenyl ist;
R₇ eine Gruppe der Formel VII
-CH₂CH(OR₅)CH₂R₆ VII;
worin R₅ und R₆ wie für Formel I angegeben ist;
R₁₉ C₂-C₈-Alkylen oder eine Gruppe -Phenylen-R₂₀-Phenylen- worin R₂₀ -C(CH₃)₂- ist;
m = 1-25;
A₁ Wasserstoff, -CH₂CH(OR₅)CH₂OR₁₉OCH(OR₅)CH₂OR₅ oder 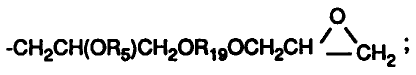 und
A₂ -OH oder 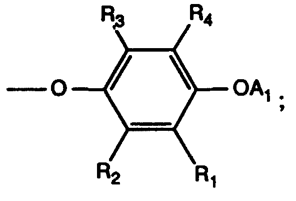
bedeutet.

4. Farbphotographisches Aufzeichnungsmaterial gemäss Anspruch 3, dadurch gekennzeichnet, dass es als Stabilisator mindestens eine Verbindung der Formel 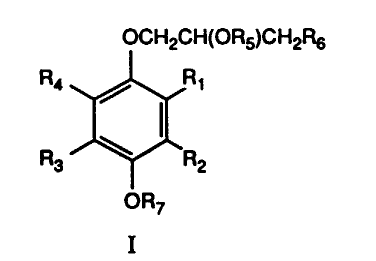 enthält, wobei
R₁, R₃ und R₄ unabhängig voneinander Wasserstoff oder C₁-C₈-Alkyl;
R₂ Wasserstoff;
R₅ Wasserstoff oder -CO-CH₃;
die Reste R₆ unabhängig voneinander -OR₁₆;
worin R₁₆ C₁-C₁₂-Alkyl, Allyl oder durch 1-3 O-Atome unterbrochenes C₃-C₇-Alkyl ist; und
R₇ eine Gruppe der Formel VII
-CH₂CH(OR₅)CH₂R₆ VII;
worin R₅ und R₆ wie für Formel I angegeben ist;
bedeutet.

5. Farbphotographisches Aufzeichnungsmaterial gemäss Anspruch 4, dadurch gekennzeichnet, dass es als Stabilisator mindestens eine Verbindung der Formel 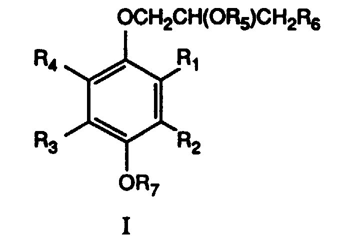 enthält, wobei
R₁, R₃ und R₄ unabhängig voneinander Wasserstoff oder C₁-C₈-Alkyl;
R₂ Wasserstoff;
R₅ Wasserstoff;
die Reste R₆ unabhängig voneinander -OR₁₆;
worin R₁₆ C₁-C₁₂-Alkyl ist; und
R₇ eine Gruppe der Formel VII
-CH₂CH(OR₅)CH₂R₆ VII;
worin R₅ und R₆ wie für Formel I angegeben ist;
bedeutet.

6. Farbphotographisches Aufzeichnungsmaterial gemäss Anspruch 1, dadurch gekennzeichnet, dass dieses weitere organische Stabilisatoren, UV-Absorber, optische Aufheller, Lichtschutzmittel, Farbschleier-Inhibitoren und/oder Weichmacher enthält.

7. Farbphotographisches Aufzeichnungsmaterial gemäss Anspruch 6, dadurch gekennzeichnet, dass dieses als weitere organische Stabilisatoren eine Verbindung der Formel P 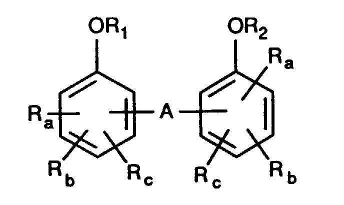 worin
R₁ und R₂ unabhängig voneinander Wasserstoff, Acyl oder Alkyl;
Rₐ, R_{b} und R_{c} unabhängig voneinander H, Alkyl, Cycloalkyl, Aryl, Halogen, Alkoxy, Aroxy, Acyloxy, Alkylthio, Arylthio, Acyl, Sulphonyl, Sulphamoyl, Acylamino, Sulphonylamino oder Nitro;
A eine Bindung, S 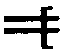 O]ₘ, Alkylen oder NR_{d};
R_{d} Alkyl oder Acyl; und
m = 0, 1, 2 bedeutet;
oder eine Verbindung der Formel SA 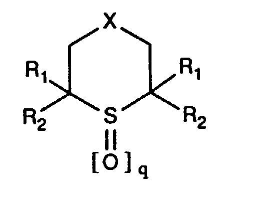 worin
R₁ Wasserstoff;
R₂ Phenyl oder
R₁ und R₂ Methyl;
q 0, 1 oder 2; und
X einen zweiwertigen Rest bedeutet, der den Ring der Formel SA zu einem Tetrahydrothiopyranring ergänzt;
oder eine Verbindung der Formel SB
R₃-S-(CₚH₂ₚ)-Z-R₄
worin
R₃ Alkyl, Aryl oder eine Gruppe (CₚH₂ₚ)-Z-R₄;
p 1-12;
Z -CO-O- oder -O-CO-;
R₄ eine ein-, zwei-, drei- oder vier-wertige Gruppe bedeutet;
oder eine Verbindung der Formel HQ 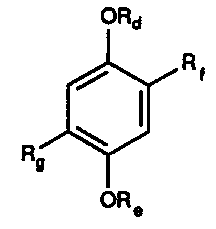 worin
Rₑ und R_{d} unabhängig voneinander Alkyl oder Cycloalkyl bedeutet; und
R_{f} und R_{g} unabhängig voneinander die Bedeutungen wie Rₐ, R_{b}, R_{c} haben;
oder eine Verbindung der Formel RE 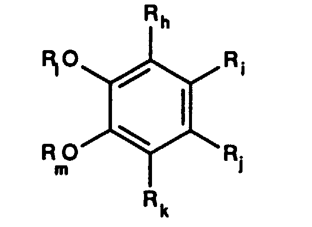 worin
Rₗ und Rₘ unabhängig voneinander H, Acyl oder Alkyl bedeutet; oder Rₗ und Rₘ zusammen an einen P-O-Aryl-Rest gebunden sind; und
Rₕ, Rᵢ, Rⱼ und Rₖ unabhängig voneinander die Bedeutung wie Rₐ, R_{b}, R_{c} haben, mit der Massgabe, dass mindestens einer der Reste Rᵢ, Rⱼ nicht Alkyl bedeutet;
oder eine Verbindung der Formel KA 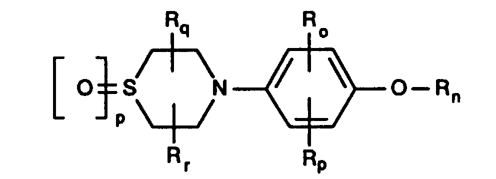 worin
Rₙ Alkyl, Cycloalkyl oder Aryl;
p 0, 1 oder 2 bedeuten; und
Rₒ, Rₚ, R_{q} und Rᵣ unabhängig voneinander die gleiche Bedeutung wie Rₐ, R_{b} und R_{c} haben;
oder eine Verbindung der Formel KB 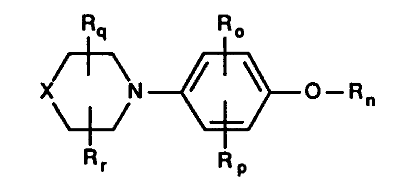 worin
X eine Bindung, 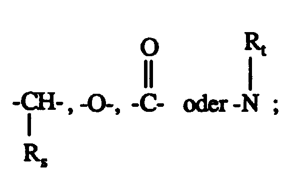
Rₜ Alkyl, Aryl, Acyl oder Sulphonyl bedeutet; und
Rₛ die Bedeutung wie Rₐ, R_{b} oder R_{c} hat;
enthalten.

8. Verbindungen der Formel 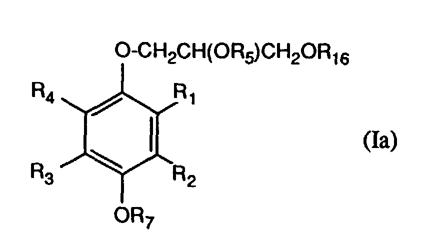 wobei
R₁ und R₃ unabhängig voneinander Wasserstoff, C₁-C₁₂-Alkyl, C₅-C₇-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 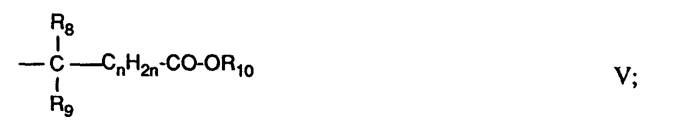
worin R₈ und R₉ unabhängig voneinander C₁-C₈-Alkyl;
n = 1-10
R₁₀ Wasserstoff, C₁-C₂₄-Alkyl, das unsubstituiert oder durch ein oder mehrere -O- Atome unterbrochen und gegebenenfalls durch ein -OH substituiert ist, C₂-C₁₈-Alkenyl,
C₅-C₁₂-Cycloalkyl, das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
worin R₁₁ C₁-C₁₈-Alkyl oder Phenyl;
R₁₂ C₁-C₄-Alkyl;
R₁₃, R₁₄ und R₁₅ unabhängig voneinander C₁-C₆-Alkyl oder Phenyl ist; R₇
eine Gruppe der Formel VI 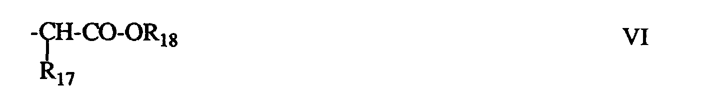 oder eine Gruppe der Formel VIIa
-CH₂CH(OR₅)CH₂OR₁₆ VIIa;
worin R₅ und R₁₆ wie für Formel (Ia) angegeben;
R₁₇ Wasserstoff oder C₁-C₁₈-Alkyl; und
R₁₈ C₁-C₁₂-Alkyl, das unsubstituiert oder durch ein oder mehrere O- Atome unterbrochen ist, C₂-C₁₈-Alkenyl, Benzyl oder Phenyl, das gegebenenfalls mit 1-3 C₁-C₄-Alkyl substituiert ist, ist;
die Reste R₁₆ unabhängig voneinander Wasserstoff, C₁-C₁₈-Alkyl, C₂-C₁₈-Alkenyl, Phenyl-C₁-C₄-alkyl, durch ein oder mehrere O-Atome unterbrochenes C₃-C₂₄-Alkyl oder C₂-C₁₄-Hydroxyalkyl, Phenyl, Tolyl, C₅-C₆-Cycloalkyl, das gegebenenfalls mit 1 bis 3 C₁-C₈-Alkyl substituiert ist, oder -CO-R₁₁;
worin R₁₁ C₁-C₁₈-Alkyl oder Phenyl ist; bedeutet.

9. Verbindungen gemäss Anspruch 8 der Formel 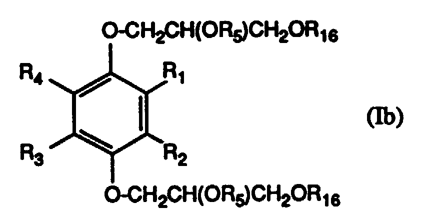 wobei
R₁ und R₃ unabhängig voneinander Wasserstoff, C₁-C₁₂-Alkyl, C₅-C₆-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 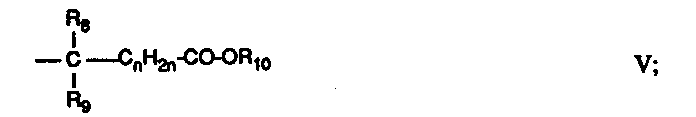
worin R₈ und R₉ unabhängig voneinander C₁-C₈-Alkyl;
n = 1-10
R₁₀ Wasserstoff, C₁-C₂₄-Alkyl, das unsubstituiert oder durch ein oder mehrere -O- Atome unterbrochen und gegebenenfalls durch ein -OH substituiert ist, C₂-C₁₈-Alkenyl,
C₅-C₁₂-Cycloalkyl, das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
worin R₁₁ C₁-C₁₂-Alkyl;
R₁₂ C₁-C₄-Alkyl;
R₁₃, R₁₄ und R₁₅ unabhängig voneinander C₁-C₆-Alkyl oder Phenyl ist;
die Reste R₁₆ unabhängig voneinander C₁-C₁₈-Alkyl, C₂-C₁₀-Alkenyl, Benzyl, durch ein oder mehrere O-Atome unterbrochenes C₃-C₁₈-Alkyl, Cyclohexyl oder -CO-R₁₁;
worin R₁₁ C₁-C₁₂-Alkyl ist;
bedeutet.

10. Verbindungen gemäss Anspruch 8 der Formel 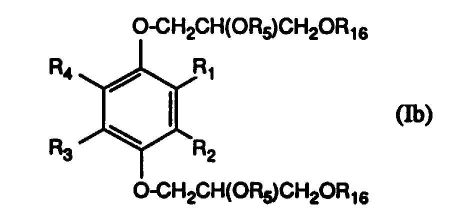 wobei
R₁ und R₃ unabhängig voneinander Wasserstoff, C₁-C₁₂-Alkyl, C₅-C₆-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 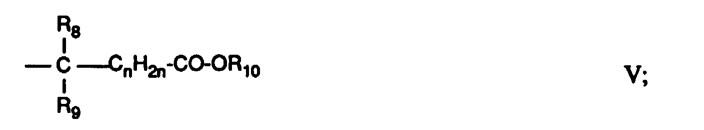
worin R₈ und R₉ unabhängig voneinander C₁-C₈-Alkyl;
n = 1-10
R₁₀ Wasserstoff, C₁-C₂₄-Alkyl, das unsubstituiert oder durch ein oder mehrere -O- Atome unterbrochen und gegebenenfalls durch ein -OH substituiert ist, C₂-C₁₈-Alkenyl, C₅-C₁₂-Cycloalkyl, das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
worin R₁₁ C₁-C₄-Alkyl;
R₁₂ C₁-C₄-Alkyl;
R₁₃, R₁₄ und R₁₅ unabhängig voneinander C₁-C₆-Alkyl oder Phenyl ist;
die Reste R₁₆ unabhängig voneinander C₁-C₁₈-Alkyl, Allyl, Benzyl, durch ein oder mehrere O-Atome unterbrochenes C₃-C₁₂-Alkyl, Cyclohexyl oder -COR₁₁;
worin R₁₁ C₁-C₄-Alkyl ist;
bedeutet.

11. Verbindungen gemäss Anspruch 8 der Formel 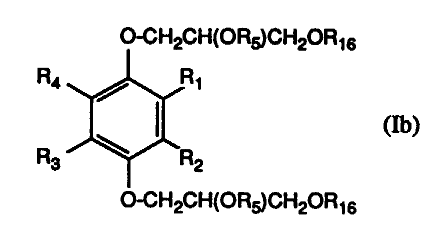
wobei R₁, R₃ und R₄ unabhängig voneinander Wasserstoff oder C₁-C₈-Alkyl;
R₂ Wasserstoff;
R₅ Wasserstoff oder -CO-CH₃; und
die Reste R₁₆ unabhängig voneinander C₁-C₁₂-Alkyl, Allyl oder durch 1-3 O-Atome unterbrochenes C₃-C₇-Alkyl;
bedeutet.

12. Verbindungen der Formeln III 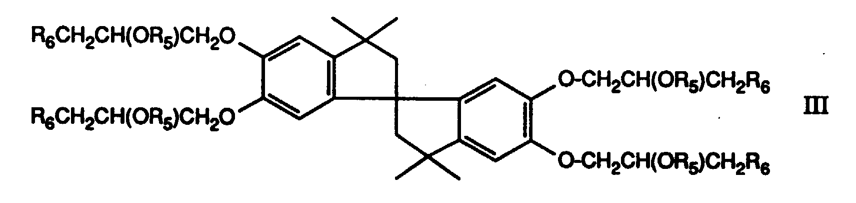 und IV 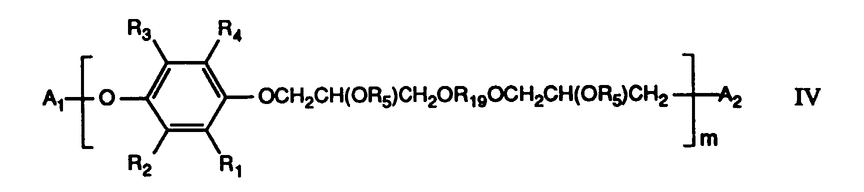 wobei
R₁ und R₃ unabhängig voneinander Wasserstoff, C₁-C₁₂-Alkyl, C₅-C₇-Cycloalkyl, das gegebenenfalls mit einer oder zwei C₁-C₄-Alkylgruppen substituiert ist, Phenyl-C₁-C₄-alkyl, Phenyl, C₁-C₈-Alkoxy oder eine Gruppe der Formel V 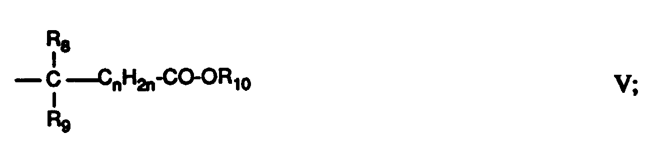
worin R₈ und R₉ unabhängig voneinander C₁-C₈-Alkyl;
n = 1-10
R₁₀ Wasserstoff, C₁-C₂₄-Alkyl, das unsubstituiert oder durch ein oder mehrere -O- Atome unterbrochen und gegebenenfalls durch ein -OH substituiert ist, C₂-C₁₈-Alkenyl, C₅-C₁₂-Cycloalkyl, das gegebenenfalls durch 1 bis 3 C₁-C₄-Alkyl substituiert ist, Phenyl, das gegebenenfalls durch ein oder zwei C₁-C₄-Alkyl substituiert ist, Phenyl-C₁-C₄-alkyl oder Furfuryl ist;
R₂ und R₄ unabhängig voneinander Wasserstoff oder C₁-C₁₂-Alkyl;
R₅ Wasserstoff, -CO-R₁₁, -CO-OR₁₂ oder -Si(R₁₃)(R₁₄)(R₁₅);
worin R₁₁ C₁-C₁₈-Alkyl, C₂-C₁₈-Alkenyl oder Phenyl;
R₁₂ C₁-C₄-Alkyl;
R₁₃, R₁₄ und R₁₅ unabhängig voneinander C₁-C₆-Alkyl oder Phenyl ist; die Reste R₆ unabhängig voneinander -OR₁₆ oder C₁-C₁₅-Alkyl;
worin R₁₆ Wasserstoff, C₁-C₁₈-Alkyl, C₂-C₁₈-Alkenyl, Phenyl-C₁-C₄-alkyl, durch -O- unterbrochenes C₃-C₂₄-Alkyl oder C₂-C₁₄ -Hydroxyalkyl, Phenyl, Tolyl, C₅-C₆-Cycloalkyl, das gegebenenfalls mit 1 bis 3 C₁-C₈-Alkyl substituiert ist, oder -CO-R₁₁ ist;
worin R₁₁ C₁-C₁₈-Alkyl, C₂-C₁₈-Alkenyl oder Phenyl ist;
R₁₉ C₂-C₁₀-Alkylen, Phenylen oder eine Gruppe -Phenylen-R₂₀-Phenylen-;
worin R₂₀ -O-, -S-, -SO₂-, -CH₂- oder -C(CH₃)₂- ist;
m = 1-100;
A₁ Wasserstoff, -CH₂CH(OR₅)CH₂OR₁₉OCH(OR₅)CH₂OR₅ oder 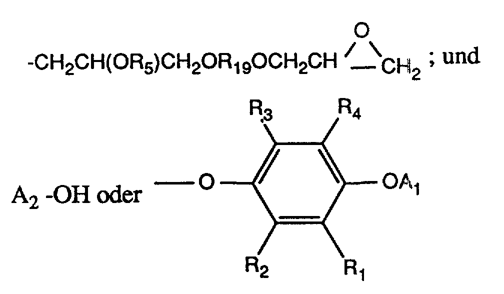 bedeutet.

13. Verfahren zum Stabilisieren von Magentakupplern und/oder Magentafarbstoffen in farbphotographischen Materialien, dadurch gekennzeichnet, dass man in das Material einen Stabilisator gemäss Anspruch 1 einarbeitet.

## Claims

1. A colour-photographic recording material which contains a magenta coupler and, as stabilizer, at least one compound of the formula 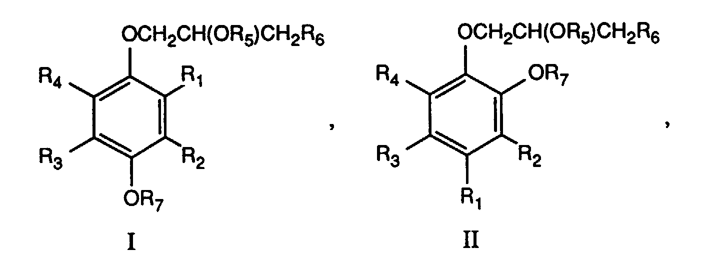 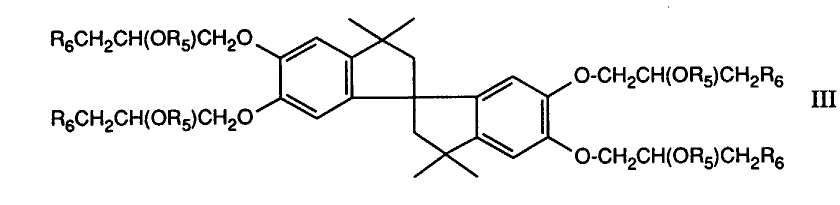 or 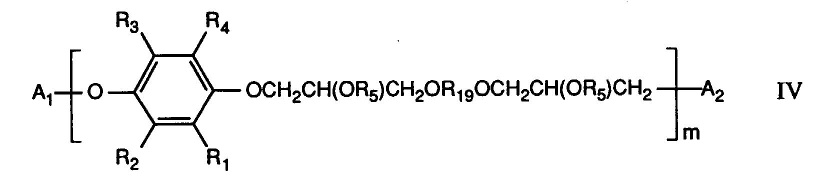 where
R₁ and R₃, independently of one another, are hydrogen, C₁-C₁₂alkyl, C₅-C₇cycloalkyl, which is unsubstituted or substituted by one or two C₁-C₄alkyl groups, phenyl-C₁-C₄alkyl, phenyl, C₁-C₈alkoxy or a group of the formula V 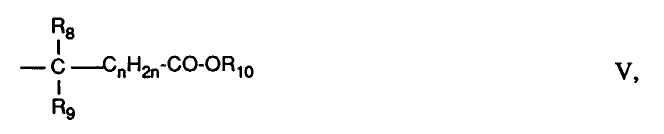
in which R₈ and R₉, independently of one another, are C₁-C₈alkyl,
n is 1-10, and
R₁₀ is hydrogen, C₁-C₂₄alkyl, which is unsubstituted or interrupted by one or more O atoms and is unsubstituted or substituted by one -OH, or is C₂-C₁₈alkenyl, C₅-C₁₂cycloalkyl, which is unsubstituted or substituted by 1 to 3 C₁-C₄alkyl, or is phenyl, which is unsubstituted or substituted by one or two C₁-C₄alkyl, or is phenyl-C₁-C₄alkyl or furfuryl;
R₂ and R₄, independently of one another, are hydrogen or C₁-C₁₂alkyl;
R₅ is hydrogen, -CO-R₁₁, -CO-OR₁₂ or -Si(R₁₃)(R₁₄)(R₁₅), in which
R₁₁ is C₁-C₁₈alkyl, C₂-C₁₈alkenyl or phenyl,
R₁₂ is C₁-C₄alkyl, and
R₁₃, R₁₄ and R₁₅, independently of one another, are C₁-C₆alkyl or phenyl;
the radicals R₆, independently of one another, are -OR₁₆ or C₁-C₁₅alkyl, in which
R₁₆ is hydrogen, C₁-C₁₈alkyl, C₂-C₁₈alkenyl, phenyl-C₁-C₄alkyl, C₃-C₂₄alkyl or C₂-C₁₄hydroxyalkyl, each of which is interrupted by one or more O atoms, or is phenyl, tolyl, C₅-C₆cycloalkyl, which is unsubstituted or substituted by 1 to 3 C₁-C₈alkyl, or is -CO-R₁₁, in which
R₁₁ is C₁-C₁₈alkyl, C₂-C₁₈alkenyl or phenyl;
R₇ is a group of the formula VI 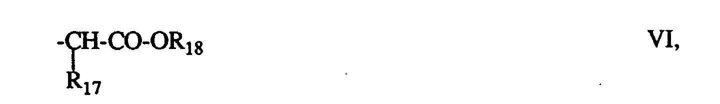 or a group of the formula VII
-CH₂CH(OR₅)CH₂R₆ VII,
in which R₅ and R₆ are as defined for formula I,
R₁₇ is hydrogen or C₁-C₁₈alkyl, and
R₁₈ is C₁-C₁₂alkyl, which is unsubstituted or interrupted by one or more O atoms, or is C₂-C₁₈alkenyl, benzyl or phenyl, which is unsubstituted or substituted by 1-3 C₁-C₄alkyl;
R₁₉ is C₂-C₁₀alkylene, phenylene or a -phenylene-R₂₀-phenylene- group, in which R₂₀ is -O-, -S-, -SO₂-, -CH₂- or -C(CH₃)₂-;
m is 1-100;
A₁ is hydrogen, -CH₂CH(OR₅)CH₂OR₁₉OCH(OR₅)CH₂OR₅ or 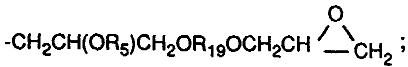 and
A₂ is -OH or 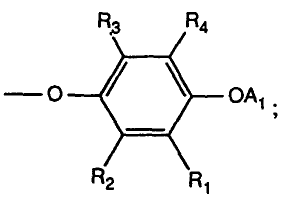 or R₃ and R₇ in the formula I, together with the atoms to which they are bonded, form a C₅-C₆ ring, which is unsubstituted or substituted by 1 to 3 C₁-C₈alkyl.

2. A colour-photographic recording material according to claim 1, which contains, as stabilizer, at least one compound of the formula 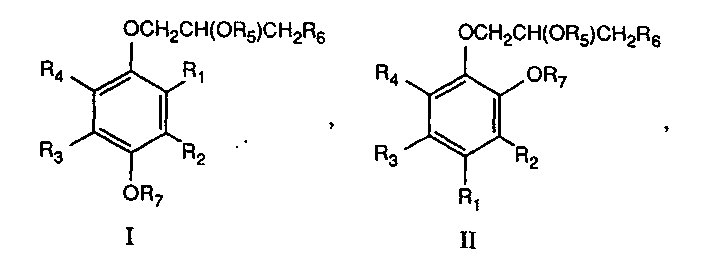 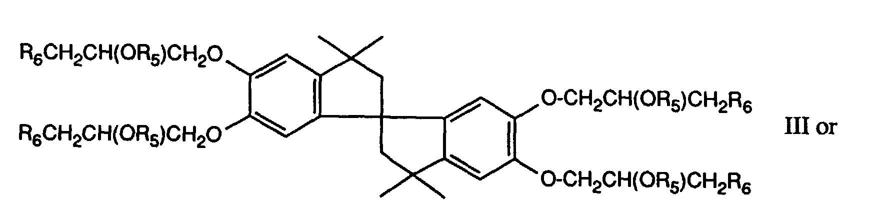 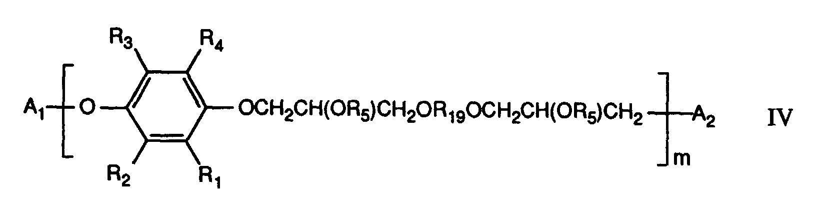
in which R₁ and R₃, independently of one another, are hydrogen, C₁-C₈alkyl, cyclohexyl, -C(CH₃)₂C₆H₅, C₁-C₄alkoxy or a group of the formula V 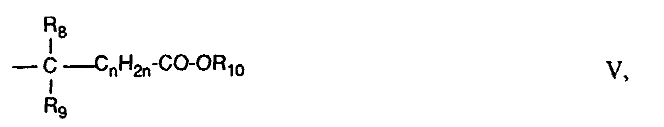
in which R₈ and R₉ are methyl,
n is 3, and
R₁₀ is hydrogen, C₁-C₁₂alkyl, which is unsubstituted or interrupted by one or more O atoms and is unsubstituted or substituted by one -OH, or is C₂-C₁₈alkenyl or benzyl;
R₂ is hydrogen;
R₄ is hydrogen or C₁-C₈alkyl;
R₅ is hydrogen, -CO-R₁₁, -CO-OR₁₂ or -Si(R₁₃)(R₁₄)(R₁₅), in which
R₁₁ is C₁-C₁₈alkyl, C₂-C₁₈alkenyl or phenyl,
R₁₂ is C₁-C₄alkyl, and
R₁₃, R₁₄ and R₁₅, independently of one another, are C₁-C₆alkyl or phenyl;
the radicals R₆, independently of one another, are -OR₁₆, in which
R₁₆ is hydrogen, C₁-C₁₈alkyl, C₂-C₁₈alkenyl, phenyl-C₁-C₄alkyl, C₃-C₂₄alkyl or C₂-C₁₄hydroxyalkyl, each of which is interrupted by one or more O atoms, or is phenyl, tolyl, C₅-C₆cycloalkyl, which is unsubstituted or substituted by 1 to 3 C₁-C₈alkyl, or is -CO-R₁₁, in which
R₁₁ is C₁-C₁₈alkyl, C₂-C₁₈alkenyl or phenyl;
R₇ is a group of the formula VI 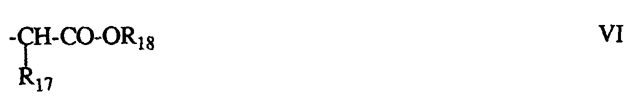 or a group of the formula VII
-CH₂CH(OR₅)CH₂R₆ VII,
in which R₅ and R₆ are as defined for formula I,
R₁₇ is hydrogen or C₁-C₁₂alkyl, and
R₁₈ is C₁-C₈alkyl;
R₁₉ is C₂-C₈ alkylene, phenylene or a -phenylene-R₂₀-phenylene- group, in which R₂₀ is -SO₂-, -CH₂- or -C(CH₃)₂-;
m is 1-50;
A₁ is hydrogen, -CH₂CH(OR₅)CH₂OR₁₉OCH(OR₅)CH₂OR₅ or 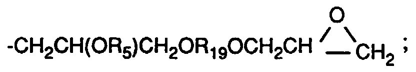 and
A₂ is -OH or

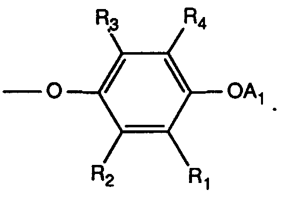

3. A colour-photographic recording material according to claim 2, which contains, as stabilizer, at least one compound of the formula 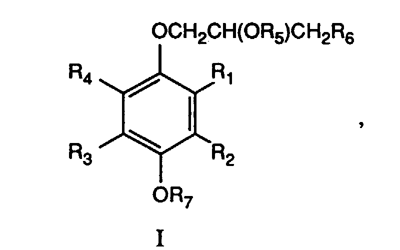 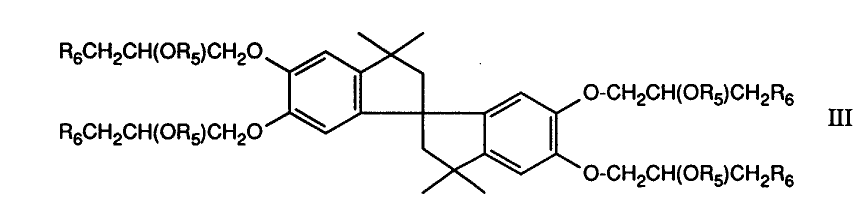 or 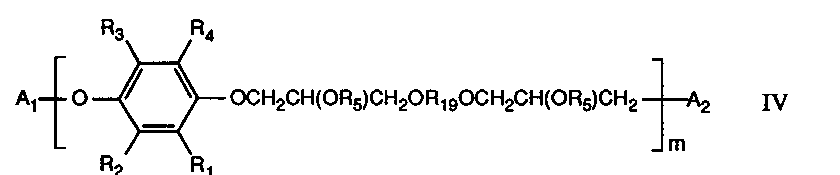
in which R₁ and R₃, independently of one another, are hydrogen, C₁-C₈alkyl, C₁-C₄alkoxy or a group of the formula V 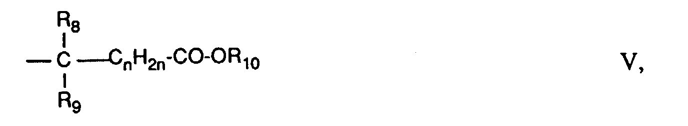
in which R₈ and R₉ are methyl,
n is 3, and
R₁₀ is C₁-C₄alkyl;
R₂ is hydrogen;
R₄ is hydrogen or C₁-C₈alkyl;
R₅ is hydrogen, CO-R₁₁, COOR₁₂ or Si(R₁₃)(R₁₄)(R₁₅), in which
R₁₁ is C₁-C₄alkyl or C₂-C₃alkenyl,
R₁₂ is C₁-C₄alkyl, and
R₁₃, R₁₄ and R₁₅, independently of one another, are C₁-C₆alkyl;
the radicals R₆, independently of one another, are -OR₁₆, in which
R₁₆ is C₁-C₁₈alkyl, allyl, benzyl, phenyl, C₃-C₁₂alkyl which is interrupted by one or more O atoms, or is cyclohexyl or -CO-R₁₁, in which
R₁₁ is C₁-C₄alkyl or C₂-C₃alkenyl;
R₇ is a group of the formula VII
-CH₂CH(OR₅)CH₂R₆ VII,
in which R₅ and R₆ are as defined for formula I;
R₁₉ is C₂-C₈alkylene or a phenylene-R₂₀-phenylene- group, in which
R₂₀ is -C(CH₃)₂-;
m is 1-25;
A₁ is hydrogen, -CH₂CH(OR₅)CH₂OR₁₉OCH(OR₅)CH₂OR₅ or 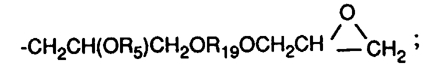 and
A₂ is -OH or

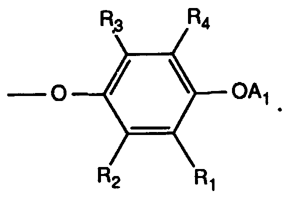

4. A colour-photographic recording material according to claim 3, which contains, as stabilizer, at least one compound of the formula 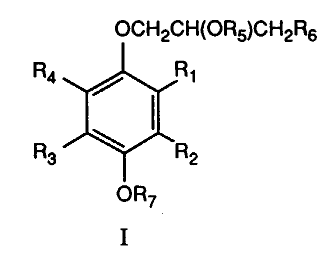 in which
R₁, R₃ and R₄, independently of one another, are hydrogen or C₁-C₈alkyl;
R₂ is hydrogen;
R₅ is hydrogen or -CO-CH₃;
the radicals R₆, independently of one another, are -OR₁₆, in which
R₁₆ is C₁-C₁₂alkyl, allyl or C₃-C₇alkyl which is interrupted by 1-3 O atoms; and
R₇ is a group of the formula VII
-CH₂CH(OR₅)CH₂R₆ VII,
in which R₅ and R₆ are as defined for formula I.

5. A colour-photographic recording material according to claim 4, which contains, as stabilizer, at least one compound of the formula 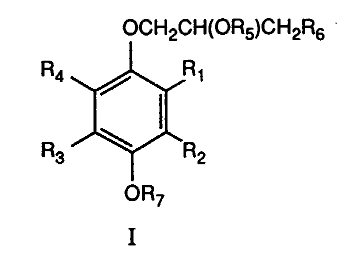 in which
R₁, R₃ and R₄, independently of one another, are hydrogen or C₁-C₈alkyl;
R₂ is hydrogen;
R₅ is hydrogen; the radicals R₆, independently of one another, are -OR₁₆, in which
R₁₆ is C₁-C₁₂alkyl and
R₇ is a group of the formula VII
-CH₂CH(OR₅)CH₂R₆ VII,
in which R₅ and R₆ are as defined for formula I.

6. A colour-photographic recording material according to claim 1, which contains further organic stabilizers, UV absorbers, optical brighteners, light stabilizers, colour cast inhibitors and/or plasticizers.

7. A colour-photographic recording material according to claim 6, which contains, as further organic stabilizers, a compound of the formula P 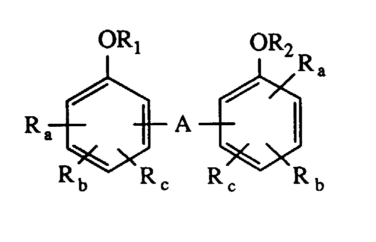 in which
R₁ and R₂, independently of one another, are hydrogen, acyl or alkyl;
Rₐ, R_{b} and R_{c}, independently of one another, are H, alkyl, cycloalkyl, aryl, halogen, alkoxy, aroxy, acyloxy, alkylthio, arylthio, acyl, sulfonyl, sulfamoyl, acylamino, sulfonylamino or nitro;
A is a bond S 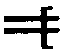 O] ₘ, alkylene or NR_{d};
R_{d} is alky or acyl; and
m is 0, 1 or 2;
or a compound of the formula SA 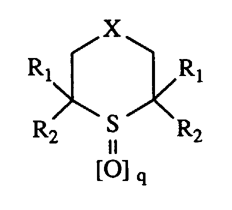 in which
R₁ is hydrogen
R₂ is phenyl or
R₁ and R₂ are methyl;
q is 0, 1 or 2; and
X is a divalent radical, which completes the ring in formula SA to give a tetrahydrothiopyran ring
or a compound of the formula SB
R₃-S-(CₚH₂ₚ)-Z-R₄
in which
R₃ is alkyl, aryl or a group (CₚH₂ₚ)-Z-R₄;
p is 1-12;
Z is -CO-O or -O-CO-;
R₄ is a mono-, di-, tri- or tetravalent group;
or a compound of the formula HQ 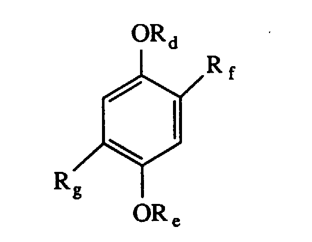 in which
Rₑ and R_{d}, independently of one another, are alkyl or cycloalkyl; and
R_{f} and R_{g}, independently of one another, are as Rₐ, R_{b} and R_{c};
or a compound of the formula RE 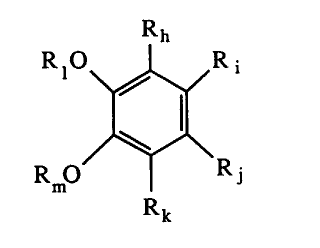 in which
Rₗ and Rₘ, independently of one another, are H, acyl or alkyl; or Rₗ and Rₘ are bound together to a P-O-aryl radical; and
Rₕ, Rᵢ, Rⱼ and Rₖ, independently of one another, are as Rₐ, R_{b} and R_{c}, provided that at least one of the radicals Rᵢ or Rⱼ is not alkyl;
or a compound of the formula KA 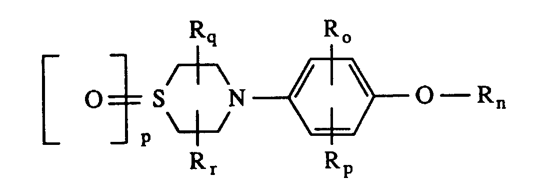 in which
Rₙ is alkyl, cycloalkyl or aryl;
p is 0, 1 or 2; and
Rₒ, Rₚ, R_{q} and Rᵣ, independently of one another, are Rₐ, R_{b} and R_{c};
or a compound of the formula KB 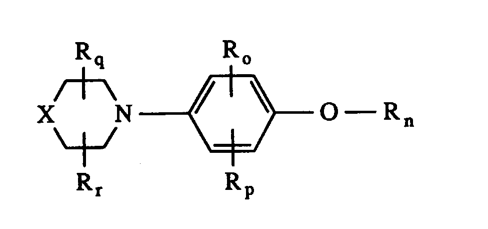 in which
X is a bond, 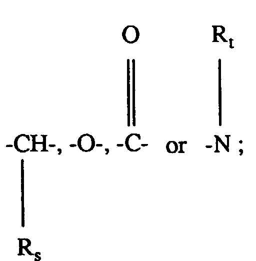
Rₜ is alkyl, aryl, acyl or sulfonyl; and
Rₛ is as Rₐ, R_{b} or R_{c}.

8. A compound of the formula 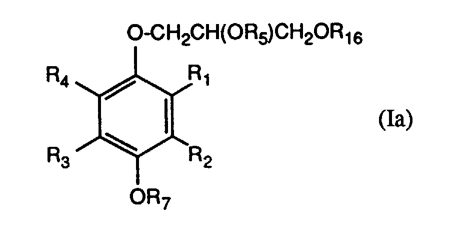 in which
R₁ and R₃, independently of one another, are hydrogen, C₁-C₁₂alkyl, C₅-C₇cycloalkyl, which is unsubstituted or substituted by one or two C₁-C₄alkyl groups, phenyl-C₁-C₄alkyl phenyl, C₁-C₈alkoxy or a group of the formula V 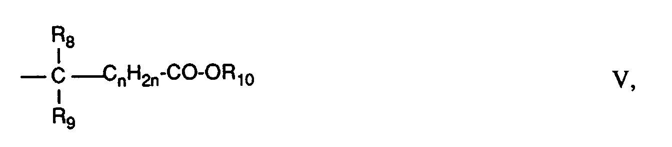
in which R₈ and R₉, independently of one another, are C₁-C₈alkyl,
nis 1-10, and
R₁₀ is hydrogen, C₁-C₂₄alkyl, which is unsubstituted or interrupted by one or more O atoms and is unsubstituted or substituted by one -OH, or is C₂-C₁₈alkenyl, C₅-C₁₂cycloalkyl, which is unsubstituted or substituted by 1 to 3 C₁-C₄alkyl, or is phenyl, which is unsubstituted or substituted by one or two C₁-C₄alkyl, or is phenyl-C₁-C₄alkyl or furfuryl;
R₂ and R₄, independently of one another, are hydrogen or C₁-C₁₂alkyl;
R₅ is hydrogen, -CO-R₁₁, -CO-OR₁₂ or -Si(R₁₃)(R₁₄)(R₁₅), in which
R₁₁ is C₁-C₁₈alkyl or phenyl,
R₁₂ is C₁-C₄alkyl, and
R₁₃, R₁₄ and R₁₅, independently of one another, are C₁-C₆alkyl or phenyl;
R₇ is a group of the formula VI 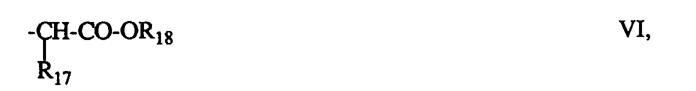 or a group of the formula VIIa
-CH₂CH(OR₅)CH₂OR₁₆ VIIa,
in which R₅ and R₁₆ are as defined for formula (Ia),
R₁₇ is hydrogen or C₁-C₁₈alkyl, and
R₁₈ is C₁-C₁₂alkyl, which is unsubstituted or interrupted by one or more O atoms, or is C₂-C₁₈alkenyl, benzyl or phenyl, which is unsubstituted or substituted by 1-3 C₁-C₄alkyl; and
the radicals R₁₆, independently of one another, are hydrogen, C₁-C₁₈alkyl, C₂-C₁₈alkenyl, phenyl-C₁-C₄alkyl, C₃-C₂₄alkyl or C₂-C₁₄hydroxyalkyl, each of which is interrupted by one or more O atoms, or are phenyl, tolyl, C₅-C₆cycloalkyl, which is unsubstituted or substituted by 1 to 3 C₁-C₈alkyl, or are -CO-R₁₁, in which
R₁₁ is C₁-C₁₈alkyl or phenyl.

9. A compound according to claim 8, of the formula 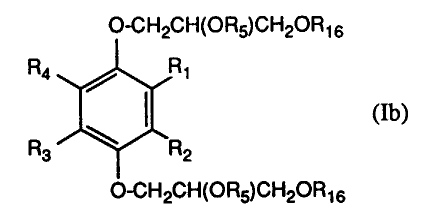 in which
R₁ and R₃, independently of one another, are hydrogen, C₁-C₁₂alkyl, C₅-C₆cycloalkyl, which is unsubstituted or substituted by one or two C₁-C₄alkyl groups, phenyl-C₁-C₄alkyl, phenyl, C₁-C₈alkoxy or a group of the formula V 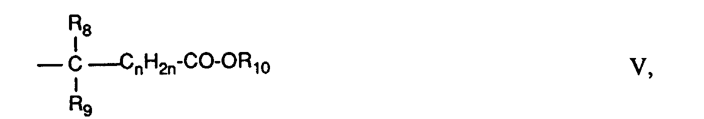
in which R₈ and R₉, independently of one another, are C₁-C₈alkyl,
n is 1-10, and
R₁₀ is hydrogen, C₁-C₂₄akyl, which is unsubstituted or interrupted by one or more O atoms and is unsubstituted or substituted by one -OH, or is C₂-C₁₈alkenyl, C₅-C₁₂cycloalkyl, which is unsubstituted or substituted by 1 to 3 C₁-C₄alkyl, or is phenyl, which is unsubstituted or substituted by one or two C₁-C₄alkyl, or is phenyl-C₁-C₄alkyl or furfuryl;
R₂ and R₄, independently of one another, are hydrogen or C₁-C₁₂alkyl;
R₅ is hydrogen, -CO-R₁₁, -CO-OR₁₂ or -Si(R₁₃)(R₁₄)(R₁₅), in which
R₁₁ is C₁-C₁₂alkyl,
R₁₂ is C₁-C₄alkyl, and
R₁₃, R₁₄ and R₁₅, independently of one another, are C₁-C₆alkyl or phenyl; and
the radicals R₁₆, independently of one another, are C₁-C₁₈alkyl, C₂-C₁₀alkenyl, benzyl, C₃-C₁₈alkyl which is interrupted by one or more O atoms, or are cyclohexyl or -CO-R₁₁, in which
R₁₁ is C₁-C₁₂alkyl.

10. A compound according to claim 8, of the formula 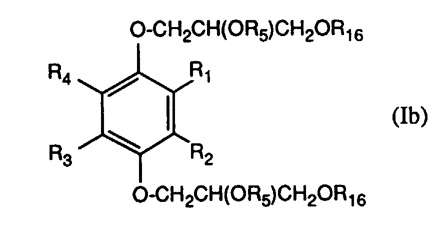 in which
R₁ and R₃, independently of one another, are hydrogen, C₁-C₁₂alkyl, C₅-C₆cycloalkyl, which is unsubstituted or substituted by one or two C₁-C₄alkyl groups, phenyl-C₁-C₄alkyl, phenyl, C₁-C₈alkoxy or a group of the formula V 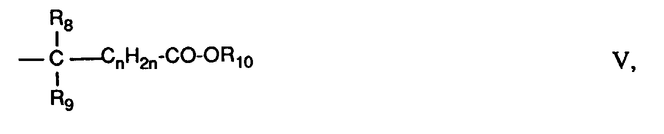
in which R₈ and R₉, independently of one another, are C₁-C₈alkyl,
n is 1-10, and
R₁₀ is hydrogen, C₁-C₂₄alkyl, which is unsubstituted or interrupted by one or more O atoms and is unsubstituted or substituted by one -OH, or is C₂-C₁₈alkenyl, C₅-C₁₂cycloalkyl, which is unsubstituted or substituted by 1 to 3 C₁-C₄alkyl, or is phenyl, which is unsubstituted or substituted by one or two C₁-C₄alkyl, or is phenyl-C₁-C₄alkyl or furfuryl;
R₂ and R₄, independently of one another, are hydrogen or C₁-C₁₂alkyl;
R₅ is hydrogen, -CO-R₁₁, -CO-OR₁₂ or -Si(R₁₃)(R₁₄)(R₁₅), in which
R₁₁ is C₁-C₄alkyl,
R₁₂ is C₁-C₄alkyl, and
R₁₃, R₁₄ and R₁₅, independently of one another, are C₁-C₆alkyl or phenyl; and
the radicals R₁₆, independently of one another, are C₁-C₁₈alkyl, allyl, benzyl, C₃-C₁₂alkyl which is interrupted by one or more O atoms, or are cyclohexyl or -COR₁₁, in which
R₁₁ is C₁-C₄alkyl.

11. A compound according to claim 8, of the formula 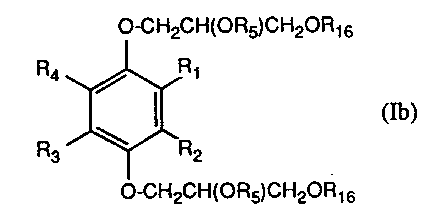
in which R₁, R₃ and R₄, independently of one another, are hydrogen or C₁-C₈alkyl;
R₂ is hydrogen;
R₅ is hydrogen or -CO-CH₃; and
the radicals R₁₆, independently of one another, are C₁-C₁₂alkyl, allyl or C₃-C₇alkyl which is interrupted by 1-3 O atoms.

12. A compound of the formula III 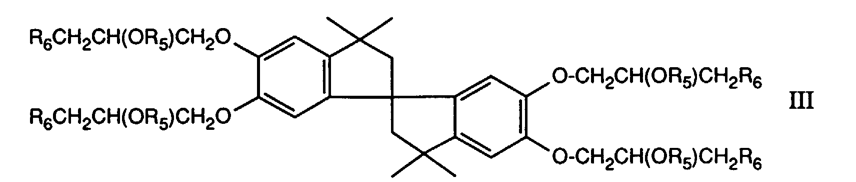 or IV 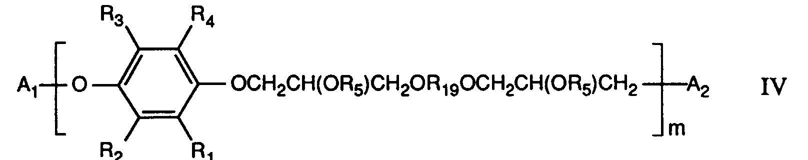 where
R₁ and R₃, independently of one another, are hydrogen, C₁-C₁₂alkyl, C₅-C₇cycloalkyl, which is unsubstituted or substituted by one or two C₁-C₄alkyl groups, phenyl-C₁-C₄alkyl, phenyl, C₁-C₈alkoxy or a group of the formula V 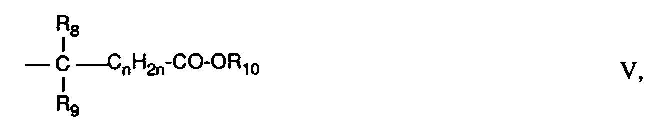
in which R₈ and R₉, independently of one another, are C₁-C₈alkyl,
n is 1-10, and
R₁₀ is hydrogen, C₁-C₂₄alkyl, which is unsubstituted or interrupted by one or more O atoms and is unsubstituted or substituted by one -OH, or is C₂-C₁₈alkenyl, C₅-C₁₂cycloalkyl, which is unsubstituted or substituted by 1 to 3 C₁-C₄alkyl, or is phenyl, which is unsubstituted or substituted by one or two C₁-C₄alkyl, or is phenyl-C₁-C₄alkyl or furfuryl;
R₂ and R₄, independently of one another, are hydrogen or C₁-C₁₂alkyl;
R₅ is hydrogen, -CO-R₁₁, -CO-OR₁₂ or -Si(R₁₃)(R₁₄)(R₁₅), in which
R₁₁ is C₁-C₁₈alkyl, C₂-C₁₈alkenyl or phenyl,
R₁₂ is C₁-C₄alkyl, and
R₁₃, R₁₄ and R₁₅, independently of one another, are C₁-C₆alkyl or phenyl;
the radicals R₆, independently of one another, are -OR₁₆ or C₁-C₁₅alkyl, in which
R₁₆ is hydrogen, C₁-C₁₈alkyl, C₂-C₁₈alkenyl, phenyl-C₁-C₄alkyl, C₃-C₂₄alkyl or C₂-C₁₄hydroxyalkyl, each of which is interrupted by -O-, or is phenyl, tolyl, C₅-C₆cycloalkyl, which is unsubstituted or substituted by 1 to 3 C₁-C₈alkyl, or is -CO-R₁₁, in which
R₁₁ is C₁-C₁₈alkyl, C₂-C₁₈alkenyl or phenyl;
R₁₉ is C₂-C₁₀alkylene, phenylene or a -phenylene-R₂₀-phenylene- group, in which
R₂₀ is -O-, -S-, -SO₂-, -CH₂- or -C(CH₃)₂-;
m is 1-100;
A₁ is hydrogen, -CH₂CH(OR₅)CH₂OR₁₉OCH(OR₅)CH₂OR₅ or 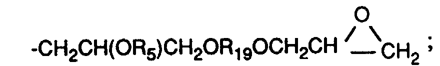 and
A₂ is -OH or

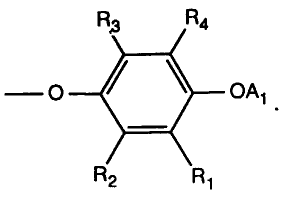

13. A process for stabilizing magenta couplers and/or magenta dyes in colour-photographic materials, in which a stabilizer according to claim 1 is incorporated into the material.

## Revendications

1. Matière d'enregistrement photographique couleur, contenant un coupleur magenta, caractérisée en ce qu'elle contient comme stabilisant au moins un composé de formule 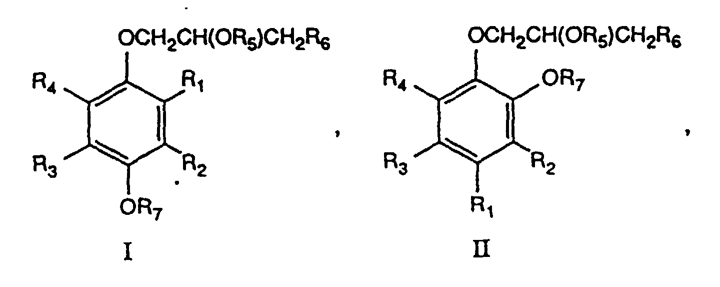 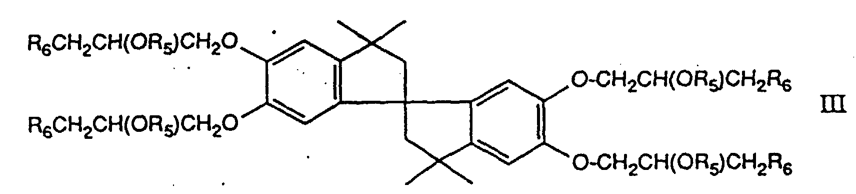 ou 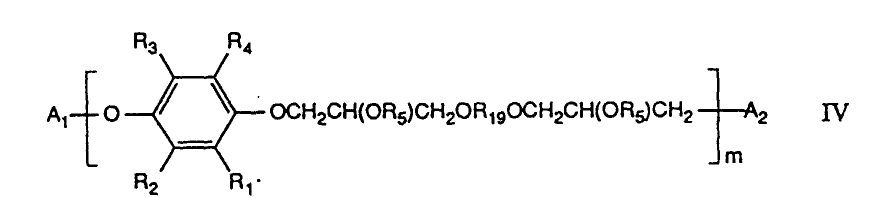
R₁ et R₃, indépendamment l'un de l'autre, représentent l'hydrogène, alkyle en C₁-C₁₂, cycloalkyle en C₅-C₇ qui est éventuellement substitué par un ou deux groupes alkyle en C₁-C₄, phénylalkyle en C₁-C₄, phényle, alcoxy en C₁-C₈ ou un groupe de formule V 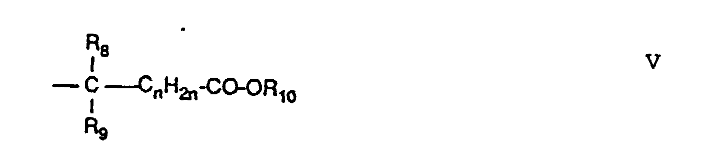
dans laquelle R₈ et R₉, indépendamment l'un de l'autre, sont alkyle en C₁-C₈ ;
n va de 1 à 10
R₁₀ représente un atome d'hydrogène, alkyle en C₁-C₂₄, lequel est non substitué ou interrompu par un ou plusieurs atomes -O- et éventuellement substitué par un groupe -OH-, alcényle en C₂-C₁₈, cycloalkyle en C₅-C₁₂, lequel est éventuellement substitué par 1 à 3 alkyles en C₁-C₄, phényle, lequel est éventuellement substitué par 1 ou 2 alkyles en C₁-C₄, phénylalkyle en C₁-C₄ ou furfuryle ;
R₂ et R₄, indépendamment l'un de l'autre, représentent l'hydrogène ou alkyle en C₁-C₁₂ ;
R₅ représente l'hydrogène, -CO-R₁₁, -CO-OR₁₂ ou -Si(R₁₃)(R₁₄)(R₁₅) ;
où R₁₁ représente alkyle en C₁-C₁₈, alcényle en C₂-C₁₈ ou phényle ;
R₁₂ représente alkyle en C₁-C₄ ;
R₁₃, R₁₄ et R₁₅, indépendamment les uns des autres, représentent alkyle en C₁-C₆ ou phényle ;
les radicaux R₆, indépendamment l'un de l'autre, représentent -OR₁₆ ou alkyle en C₁-C₁₅ ;
dans laquelle R₁₆ représente l'hydrogène, alkyle en C₁-C₁₈, alcényle en C₂-C₁₈, phénylalkyle en C₁-C₄, alkyle en C₃-C₂₄ ou hydroxyalkyle en C₂-C₁₄ interrompu par un ou plusieurs atomes -O-, phényle, tolyle, cycloalkyle en C₅-C₆, lequel est éventuellement substitué par 1 à 3 alkyles en C₁-C₈, ou représente -CO-R₁₁ ;
dans laquelle R₁₁ représente alkyle en C₁-C₁₈, alcényle en C₂-C₁₈ ou phényle ;
R₇ représente un groupe de formule VI 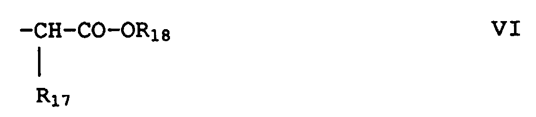 ou un groupe de formule VII
-CH₂CH(OR₅)CH₂R₆ VII
dans laquelle R₅ et R₆ ont les significations données dans la formule I ;
R₁₇ représente l'hydrogène ou alkyle en C₁-C₁₈ ; et R₁₈ représente alkyle en C₁-C₁₂, lequel est non substitué ou interrompu par un ou plusieurs atomes -O-, représente alcényle en C₂-C₁₈, benzyle ou phényle, lequel est éventuellement substitué par 1 à 3 atomes d'alkyle en C₁-C₄ ;
R₁₉ représente alkylène en C₂-C₁₀, phénylène ou un groupe -phénylène-R₂₀-phénylène- ;
dans lequel R₂₀ représente -O-, -S-, -SO₂-, -CH₂- ou -C(CH₃)₂- ;
m va de 1 à 100 ;
A₁ représente l'hydrogène, -CH₂CH(OR₅)CH₂OR₁₉OCH(OR₅)CH₂OR₅ ou 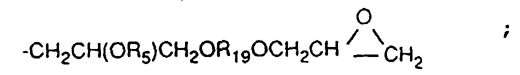 et
A₂ représente OH ou 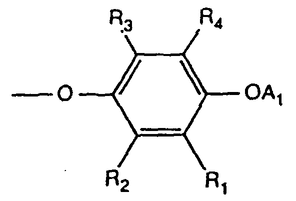 ou dans la formule I, R₃ et R₇, ensemble avec des atomes auxquels ils sont liés, forment un cycle en C₅-C₆, lequel est éventuellement substitué par 1 à 3 alkyles en C₁-C₈.

2. Matière d'enregistrement photographique couleur selon la revendication 1, caractérisée en ce qu'elle contient comme stabilisant au moins un composé de formules 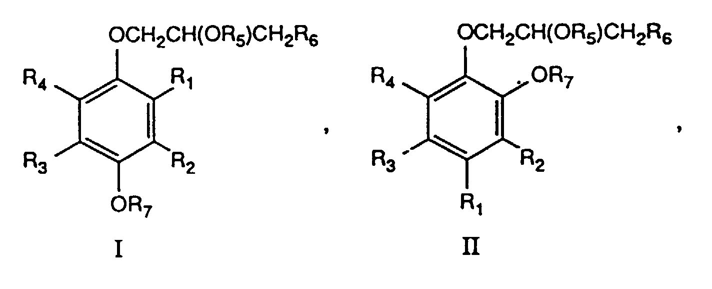 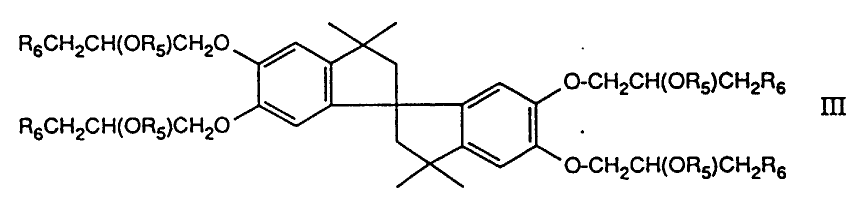 ou 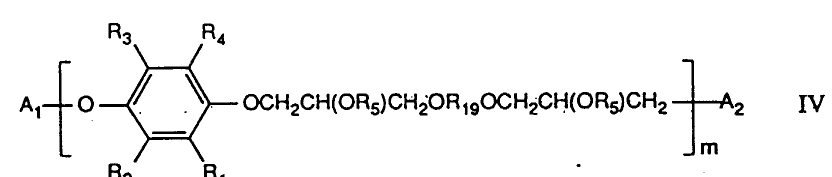
R₁ et R₃, indépendamment l'un de l'autre, représentent l'hydrogène, alkyle en C₁-C₈, cyclohexyle, -C(CH₃)₂C₆H₅, alcoxy en C₁-C₄ ou un groupe de formule V 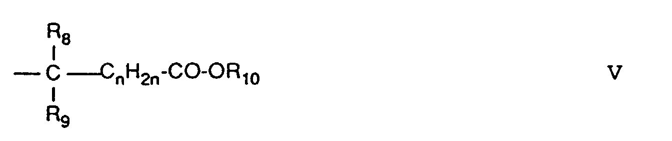
dans laquelle R₈ et R₉ représentent méthyle ;
n vaut 3 et
R₁₀ représente l'hydrogène, alkyle en C₁-C₁₂, qui est non substitué ou interrompu par un ou plusieurs atomes -O- et éventuellement substitué par un groupe -OH, alcényle en C₂-C₁₈ ou benzyle ;
R₂ représente l'hydrogène ;
R₄ représente l'hydrogène ou alkyle en C₁-C₈ ;
R₅ représente l'hydrogène, -CO-R₁₁, -CO-OR₁₂ ou -Si(R₁₃)(R₁₄)(R₁₅) ;
où R₁₁ représente alkyle en C₁-C₁₈, alcényle en C₂-C₁₈ ou phényle ;
R₁₂ représente alkyle en C₁-C₄ ;
R₁₃, R₁₄ et R₁₅, indépendamment les uns des autres, représentent alkyle en C₁-C₆ ou phényle ;
les radicaux R₆, indépendamment l'un de l'autre, représentent -OR₁₆ ;
dans laquelle R₁₆ représente l'hydrogène, alkyle en C₁-C₁₈, alcényle en C₂-C₁₈, phénylalkyle en C₁-C₄, alkyle en C₃-C₂₄ ou hydroxyalkyle en C₂-C₁₄ interrompu par un ou plusieurs atomes -O-, phényle, tolyle, cycloalkyle en C₅-C₆, qui est éventuellement substitué par 1 à 3 alkyles en C₁-C₈, ou représente -CO-R₁₁ ;
où R₁₁ représente alkyle en C₁-C₁₈, alcényle en C₂-C₁₈ ou phényle ;
R₇ représente un groupe de formule VI 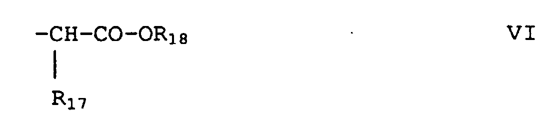 ou un groupe de formule VII
-CH₂CH(OR₅)CH₂R₆ VII
dans laquelle R₅ et R₆ ont des significations données dans la formule I ;
R₁₇ représente l'hydrogène ou alkyle en C₁-C₁₂ ; et
R₁₈ représente alkyle en C₁-C₈ ;
R₁₉ représente alkylène en C₂-C₈, phénylène ou un groupe -phényléne-R₂₀-phénylène- ;
dans lequel R₂₀ représente -SO₂-, -CH₂- ou -C(CH₃)₂- ;
m va de 1 à 50 ;
A₁ représente l'hydrogène, -CH₂CH(OR₅)CH₂OR₁₉OCH(OR₅)CH₂OR₅ ou 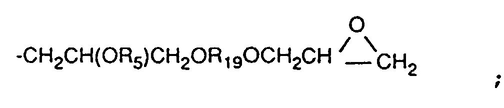 et
A₂ représente OH ou

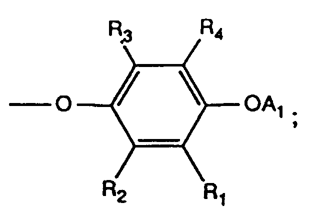

3. Matière d'enregistrement photographique couleur selon la revendication 2, caractérisée en ce qu'elle contient comme stabilisant au moins un composé de formules 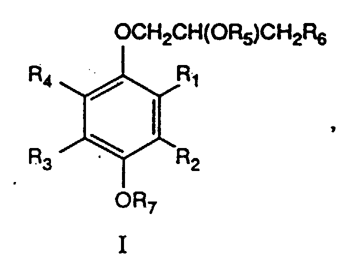 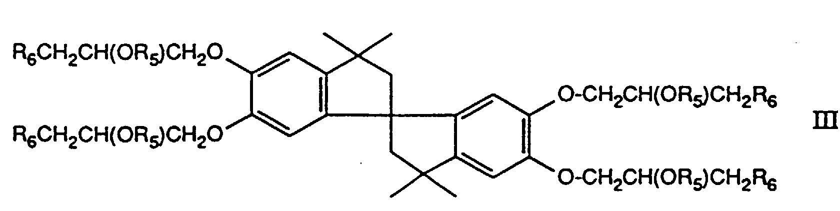 ou ou 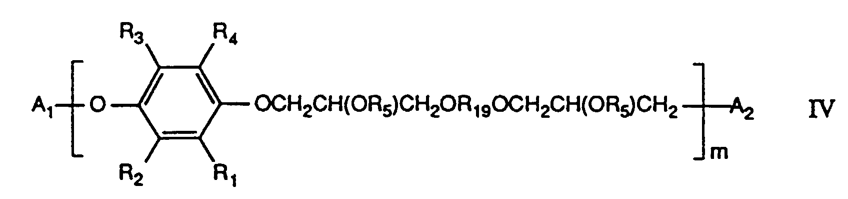
R₁ et R₃, indépendamment l'un de l'autre, représentent l'hydrogène, alkyle en C₁-C₈, alcoxy en C₁-C₄ ou un groupe de formule V 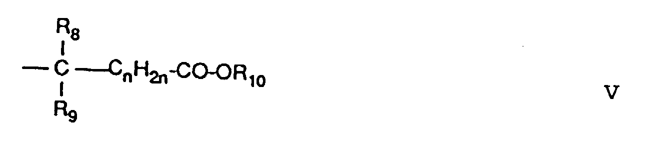
dans laquelle R₈ et R₉ représentent méthyle ;
n vaut 3 et
R₁₀ représente alkyle en C₁-C₄ ;
R₂ représente l'hydrogène ;
R₄ représente l'hydrogène ou alkyle en C₁-C₈ ;
R₅ représente l'hydrogène, -CO-R₁₁, -COOR₁₂ ou -Si(R₁₃)(R₁₄)(R₁₅) ;
où R₁₁ représente alkyle en C₁-C₄, alcényle en C₂-C₃ ; R₁₂ représente alkyle en C₁-C₄ ;
R₁₃, R₁₄ et R₁₅, indépendamment les uns des autres, représentent alkyle en C₁-C₆ ;
les radicaux R₆, indépendamment l'un de l'autre, représentent -OR₁₆ ;
où R₁₆ représente alkyle en C₁-C₁₈, allyle, benzyle, phényle, alkyle en C₃-C₁₂ interrompu par un ou plusieurs atomes -O-, cyclohexyle ou -CO-R₁₁- ;
où R₁₁ représente alkyle en C₁-C₄ ou alcényle en C₂-C₃ ;
R₇ représente un groupe de formule VII
-CH₂CH(OR₅)CH₂R₆ VII
dans laquelle R₅ et R₆ ont des significations données dans la formule I ;
R₁₉ représente alkylène en C₂-C₈ ou un groupe -phénylène-R₂₀-phénylène-;
dans lequel R₂₀ représente -C(CH₃)₂- ;
m va de 1 à 25 ;
A₁ représente l'hydrogène, -CH₂CH(OR₅)CH₂OR₁₉OCH(OR₅)CH₂OR₅ ou 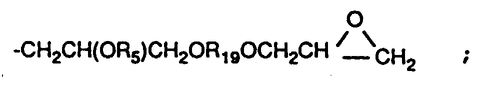 et
A₂ représente OH ou

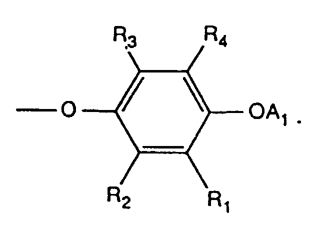

4. Matière d'enregistrement photographique couleur selon la revendication 3, caractérisée en ce qu'elle contient comme stabilisant au moins un composé de formule 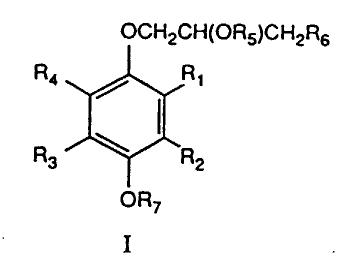
R₁, R₃ et R₄, indépendamment les uns des autres, représentent l'hydrogène ou alkyle en C₁-C₈ ;
R₂ représente l'hydrogène ;
R₅ représente l'hydrogène ou -CO-CH₃ ;
les radicaux R₆, indépendamment l'un de l'autre, représentent -OR₁₆ ;
où R₁₆ représente alkyle en C₁-C₁₂, allyle ou alkyle en C₃-C₇ interrompu par 1 à 3 atomes de -O- ; et
R₇ représente un groupe de formule VII
-CH₂CH(OR₅)CH₂R₆ VII
dans laquelle R₅ et R₆ ont les significations données dans la formule I.

5. Matière d'enregistrement photographique couleur selon la revendication 4, caractérisée en ce qu'elle contient comme stabilisant au moins un composé de formule 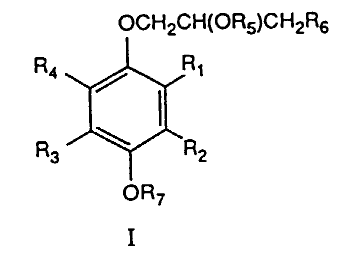
R₁, R₃ et R₄, indépendamment les uns des autres, représentent l'hydrogène ou alkyle en C₁-C₈ ;
R₂ représente l'hydrogène ;
R₅ représente l'hydrogène ;
les radicaux R₆, indépendamment l'un de l'autre, représentent -OR₁₆ ;
où R₁₆ représente alkyle en C₁-C₁₂ ; et
R₇ représente un groupe de formule VII
-CH₂CH(OR₅)CH₂R₆ VII
dans laquelle R₅ et R₆ ont les significations données dans la formule I.

6. Matière d'enregistrement photographique couleur selon la revendication 1, caractérisée en ce qu'elle contient d'autres stabilisants organiques, des absorbeurs d'UV, des azurants optiques, des agents photoprotecteurs, des inhibiteurs de voile coloré et/ou assouplissants.

7. Matière d'enregistrement photographique couleur selon la revendication 6, caractérisée en ce qu'elle contient comme autre stabilisant organique un composé de formule P 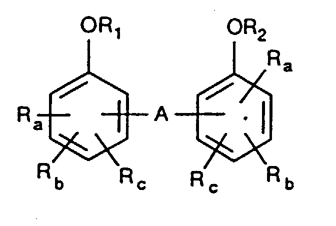 dans laquelle
R₁ et R₂, indépendamment l'un de l'autre, représentent l'hydrogène, acyle ou alkyle ;
Rₐ, R_{b} et R_{c}, indépendamment les uns des autres, représentent H, alkyle, cycloalkyle, aryle, halogène, alcoxy, aroxy, acyloxy, alkylthio, arylthio, acyle, sulfonyle, sulfamoyle, acylamino, sulfonylamino ou nitro ;
A représente un composé S 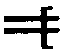 O]ₘ, alkylène ou -NR_{d} ;
R_{d} représente alkyle ou acyle ; et
m vaut 0, 1 ou 2 ;
ou un composé de formule SA 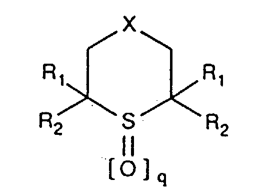 dans laquelle
R₁ représente l'hydrogène ;
R₂ représente phényle ou
R₁ et R₂ représentent méthyle ;
q vaut 0, 1 ou 2 ; et
X représente un radical bivalent, qui complète le cycle de formule SA en un cycle tétrahydrothiopyranne ;
ou un composé de formule SB
R₃-S-(CₚH₂ₚ)-Z-R₄
dans laquelle
R₃ représente alkyle, aryle ou un groupe (CₚH₂ₚ)-Z-R₄ ;
p va de 1 à 12 ;
Z -CO-O- ou -O-CO- ;
R₄ représente un groupe mono-, bi-, tri- ou tétravalent ;
ou un composé de formule HQ 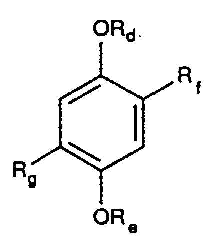 dans laquelle
Rₑ et R_{d}, indépendamment l'un de l'autre, représentent alkyle ou cycloalkyle ; et
R_{f} et R_{g}, indépendamment l'un de l'autre, ont les significations de Rₐ, R_{b}, R_{c} ;
ou un composé de formule RE 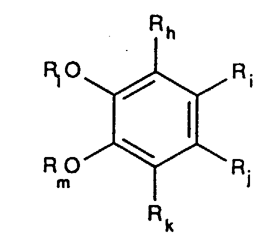 dans laquelle
Rₗ et Rₘ, indépendamment l'un de l'autre, représentent H, acyle ou alkyle ; ou Rₗ et Rₘ, ensemble, sont liés à ur radical P-O-aryle ; et
Rₕ, Rᵢ, Rⱼ et Rₖ, indépendamment les uns des autres, ont les significations de Rₐ, R_{b}, R_{c}, à la condition qu'au moins l'un des radicaux Rᵢ, Rⱼ ne soit pas alkyle ;
ou un composé de formule KA 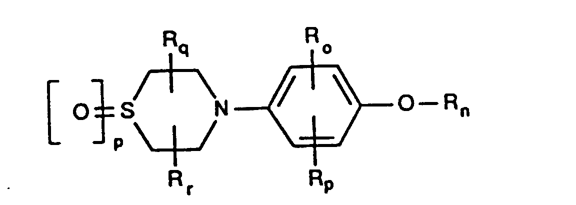 dans laquelle
Rₙ représente alkyle, cycloalkyle ou aryle ;
p vaut 0, 1 ou 2 ; et
Rₒ, Rₚ, R_{q} et Rᵣ, indépendamment les uns des autres, ont les mêmes significations de Rₐ, R_{b} et R_{c}.
ou un composé de formule KB 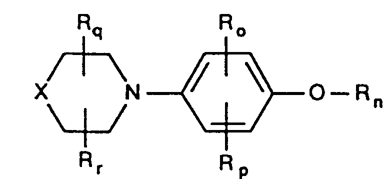 dans laquelle
X représente une liaison, 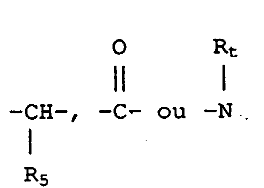
Rₜ représente alkyle, aryle, acyle ou sulfonyle ; et Rₛ a la signification de Rₐ, de R_{b} ou de R_{c}.

8. Composés de formule 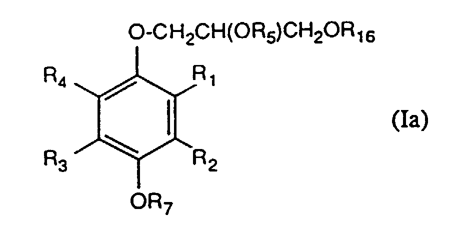 dans laquelle
R₁ et R₃, indépendamment l'un de l'autre, représentent l'hydrogène, alkyle en C₁-C₁₂, cycloalkyle en C₅-C₇, qui est éventuellement substitué par un ou deux groupes alkyle en C₁-C₄, phénylalkyle en C₁-C₄, phényle, alcoxy en C₁-C₈ ou un groupe de formule V 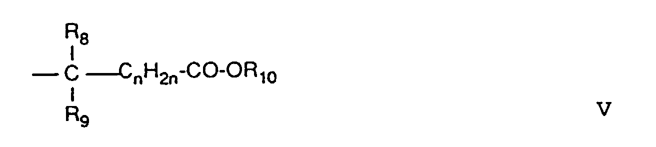
dans laquelle R₈ et R₉, indépendamment l'un de l'autre, représentent alkyle en C₁-C₈ ;
n va de 1 à 10 ;
R₁₀ représente l'hydrogène, alkyle en C₁-C₂₄, qui est non substitué ou interrompu par un ou plusieurs atomes de -O- et éventuellement substitué par un groupe -OH, alcényle en C₂-C₁₈, cycloalkyle en C₅-C₁₂, qui est éventuellement substitué par 1 à 3 alkyles en C₁-C₄, phényle, qui est éventuellement substitué par 1 ou 2 alkyles en C₁-C₄, phénylalkyle en C₁-C₄ ou furfuryle ;
R₂ et R₄, indépendamment l'un de l'autre, sont l'hydrogène ou alkyle en C₁-C₁₂ ;
R₅ représente l'hydrogène, -CO-R₁₁, -CO-OR₁₂ ou -Si(R₁₃)(R₁₄)(R15) ;
où R₁₁ représente alkyle en C₁-C₁₈ ou phényle ;
R₁₂ représente alkyle en C₁-C₄ ;
R₁₃, R₁₄ et R₁₅, indépendamment les uns des autres, représentent alkyle en C₁-C₆ ou phényle ;
R₇ représente un groupe de formule VI 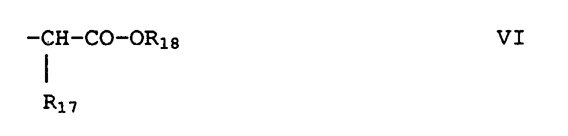 ou un groupe de formule VIIa
-CH₂CH(OR₅)CH₂OR₁₆ VIIa
dans laquelle R₅ et R₁₆ ont des significations données dans la formule Ia ;
R₁₇ représente l'hydrogène ou alkyle en C₁-C₁₈ ; et
R₁₈ représente alkyle en C₁-C₁₂, qui est non substitué ou interrompu par un ou plusieurs atomes de O, alcényle en C₂-C₁₈, benzyle ou phényle qui est éventuellement substitué par 1 à 3 alkyles en C₁-C₄ ;
les radicaux R₁₆, indépendamment l'un de l'autre, représente l'hydrogène, alkyle en C₁-C₁₈, alcényle en C₂-C₁₈, phénylalkyle en C₁-C₄, alkyle en C₃-C₂₄ ou hydroxyalkyle en C₂-C₁₄ interrompu par un ou plusieurs atomes de O, phényle, tolyle, cycloalkyle en C₅-C₆, qui est éventuellement substitué par 1 à 3 alkyles en C₁-C₈, ou -CO-R₁₁ ;
où R₁₁ représente alkyle en C₁-C₁₈ ou phényle.

9. Composés selon la revendication 8 de formule : 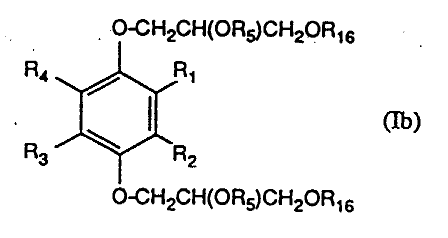 dans laquelle
R₁ et R₃, indépendamment l'un de l'autre, étant l'hydrogène, alkyle en C₁-C₁₂, cycloalkyle en C₅-C₆, qui est éventuellement substitué par un ou deux groupes alkyle en C₁-C₄, phénylalkyle en C₁-C₄, phényle, alcoxy en C₁-C₈ ou un groupe de formule V 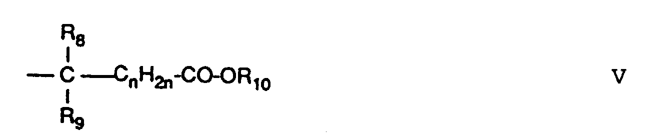
dans laquelle R₈ et R₉, indépendamment l'un de l'autre, représentent alkyle en C₁-C₈ ;
n va de 1 à 10 ;
R₁₀ représente l'hydrogène, alkyle en C₁-C₂₄, qui est non substitué ou interrompu par un ou plusieurs atomes de -O- et éventuellement substitué par un groupe -OH, alcényle en C₂-C₁₈, cycloalkyle en C₅-C₁₂, qui est éventuellement substitué par 1 à 3 alkyles en C₁-C₄, phényle, qui est éventuellement substitué par 1 ou 2 alkyles en C₁-C₄, phénylalkyle en C₁-C₄ ou furfuryle ;
R₂ et R₄, indépendamment l'un de l'autre, représentent l'hydrogène ou alkyle en C₁-C₁₂ ;
R₅ représente l'hydrogène, -CO-R₁₁, -CO-OR₁₂ ou -Si(R₁₃)(R₁₄)(R15) ;
où R₁₁ représente alkyle en C₁-C₁₂ ;
R₁₂ représente alkyle en C₁-C₄ ;
R₁₃, R₁₄ et R₁₅, indépendamment les uns des autres, représentent alkyle en C₁-C₆ ou phényle ;
les radicaux R₁₆, indépendamment l'un de l'autre, représentent alkyle en C₁-C₁₈, alcényle en C₂-C₁₀, benzyle ou alkyle en C₃-C₁₈ interrompu par un ou plusieurs atomes de O, cyclohexyle ou -CO-R₁₁ ;
où R₁₁ représente alkyle en C₁-C₁₂.

10. Composés selon la revendication 8 de formule : 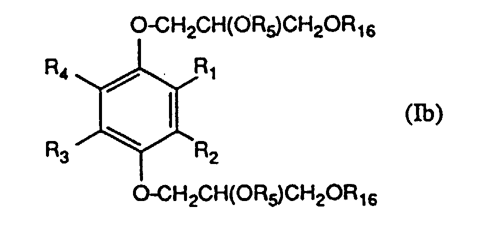 dans laquelle
R₁ et R₃, indépendamment l'un de l'autre, représentent l'hydrogène, alkyle en C₁-C₁₂, cycloalkyle en C₅-C₆, qui est éventuellement substitué par un ou deux groupes alkyle en C₁-C₄, phénylalkyle en C₁-C₄, phényle, alcoxy en C₁-C₈ ou un groupe de formule V 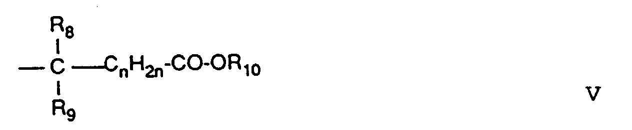
dans laquelle R₈ et R₉, indépendamment l'un de l'autre, représentent alkyle en C₁-C₈ ;
n va de 1 à 10 ;
R₁₀ représente l'hydrogène, alkyle en C₁-C₂₄, qui est non substitué ou interrompu par un ou plusieurs atomes de -O- et éventuellement substitué par un groupe -OH, alcényle en C₂-C₁₈, cycloalkyle en C₅-C₁₂, qui est éventuellement substitué par 1 à 3 alkyles en C₁-C₄, phényle, qui est éventuellement substitué par 1 ou 2 alkyles en C₁-C₄, phénylalkyle en C₁-C₄ ou furfuryle ;
R₂ et R₄, indépendamment l'un de l'autre, représentent l'hydrogène ou alkyle en C₁-C₁₂ ;
R₅ représente l'hydrogène, -CO-R₁₁, -CO-OR₁₂ ou -Si(R₁₃)(R₁₄)(R₁₅) ;
où R₁₁ représente alkyle en C₁-C₄ ;
R₁₂ représente alkyle en C₁-C₄ ;
R₁₃, R₁₄ et R₁₅, indépendamment les uns des autres, représentent alkyle en C₁-C₆ ou phényle ;
les radicaux R₁₆, indépendamment l'un de l'autre, représentent alkyle en C₁-C₁₈, allyle, benzyle, alkyle en C₃-C₁₂ interrompu par un ou plusieurs atomes de O, cyclohexyle ou -CO-R₁₁ ;
où R₁₁ représente alkyle en C₁-C₄.

11. Composés selon la revendication 8 de formule 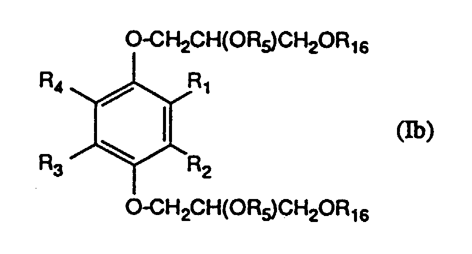 dans laquelle
R₁, R₃ et R₄, indépendamment les uns des autres, représentent l'hydrogène ou alkyle en C₁-C₈ ;
R₂ représente l'hydrogène ;
R₅ représente l'hydrogène ou -CO-CH₃ ; et
les radicaux R₁₆, indépendamment l'un de l'autre, représentent alkyle en C₁-C₁₂, allyle ou alkyle en C₃-C₇ interrompu par 1 à 3 atomes de O.

12. Composés de formule III 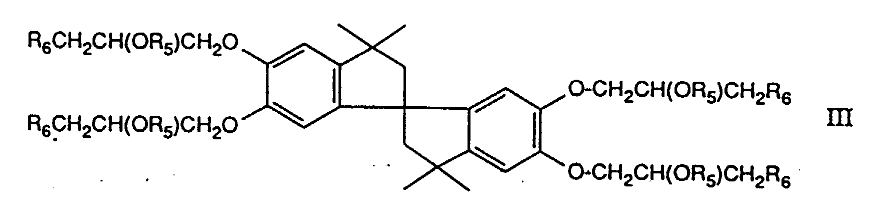 et IV 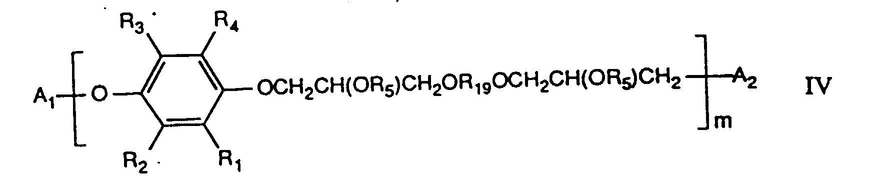 dans lesquelles
R₁ et R₃, indépendamment l'un de l'autre, représentent l'hydrogène, alkyle en C₁-C₁₂, cycloalkyle en C₅-C₇ qui est éventuellement substitué par un ou deux groupes alkyle en C₁-C₄, phénylalkyle en C₁-C₄, phényle, alcoxy en C₁-C₈ ou un groupe de formule V 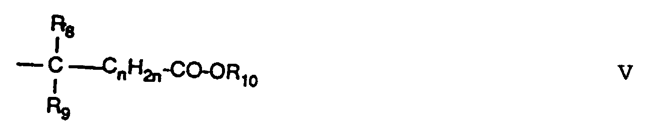
dans laquelle R₈ et R₉, indépendamment l'un de l'autre, sont alkyle en C₁-C₈ ;
n va de 1 à 10
R₁₀ représente un atome d'hydrogène, alkyle en C₁-C₂₄, lequel est non substitué ou interrompu par un ou plusieurs atomes -O- et éventuellement substitué par un groupe -OH-, alcényle en C₂-C₁₈, cycloalkyle en C₅-C₁₂, lequel est éventuellement substitué par 1 à 3 alkyles en C₁-C₄, phényle, lequel est éventuellement substitué par 1 ou 2 alkyles en C₁-C₄, phénylalkyle en C₁-C₄ ou furfuryle ;
R₂ et R₄, indépendamment l'un de l'autre, représentent l'hydrogène ou un alkyle en C₁-C₁₂ ;
R₅ représente l'hydrogène, -CO-R₁₁, -CO-OR₁₂ ou -Si(R₁₃)(R₁₄)(R₁₅) ;
où R₁₁ représente alkyle en C₁-C₁₈, alcényle en C₂-C₁₈ ou phényle ;
R₁₂ représente alkyle en C₁-C₄ ;
R₁₃, R₁₄ et R₁₅, indépendamment les uns des autres, représentent alkyle en C₁-C₆ ou phényle ;
les radicaux R₆, indépendamment l'un de l'autre, représentent -OR₁₆ ou alkyle en C₁-C₁₅ ;
où R₁₆ représente l'hydrogène, alkyle en C₁-C₁₈, alcényle en C₂-C₁₈, phénylalkyle en C₁-C₄, alkyle en C₃-C₂₄ ou hydroxyalkyle en C₂-C₁₄ interrompu par un ou plusieurs atomes -O-, phényle, tolyle, cycloalkyle en C₅-C₆, lequel est éventuellement substitué par 1 à 3 alkyles en C₁-C₈, ou représente -CO-R₁₁ ;
dans laquelle R₁₁ représente alkyle en C₁-C₁₈, alcényle en C₂-C₁₈ ou phényle ;
R₁₉ représente alkylène en C₂-C₁₀, phénylène ou un groupe -phénylène-R₂₀-phénylène- ;
dans lequel R₂₀ représente -O-, -S-, -SO₂-, -CH₂- ou -C(CH₃)₂- ;
m va de 1 à 100 ;
A₁ représente l'hydrogène, -CH₂CH(OR₅)CH₂OR₁₉OCH(OR₅)CH₂OR₅ ou 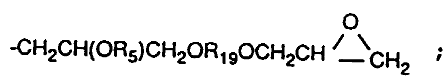 et
A₂ représente OH ou

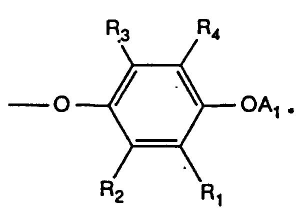

13. Procédé pour la stabilisation de coupleurs magenta et/ou de colorants magenta dans des matières photographiques couleur caractérisé en ce que l'on incorpore à la matière un stabilisant selon la revendication 1.
